(19) Europäisches Patentamt
European Patent Office
Office européen des brevets

(11) Numéro de publication : **0 346 238 B1**

# (12) FASCICULE DE BREVET EUROPEEN

(45) Date de publication du fascicule du brevet : **09.06.93 Bulletin 93/23**

(51) Int. Cl.$^5$ : **C07D 279/28**, A61K 31/54

(21) Numéro de dépôt : **89401601.3**

(22) Date de dépôt : **09.06.89**

(54) **Dérivés de la phénothiazine analgésiques.**

(30) Priorité : **10.06.88 FR 8807770**

(43) Date de publication de la demande : **13.12.89 Bulletin 89/50**

(45) Mention de la délivrance du brevet : **09.06.93 Bulletin 93/23**

(84) Etats contractants désignés : **AT BE CH DE ES FR GB GR IT LI LU NL SE**

(56) Documents cités :
**US-A- 2 957 870**
**US-A- 3 112 310**
**TETRAHEDRON LETTERS, vol. 27, no. 33, 1986, pages 3911-3912, Pergamon Press, LTD, GB; N. NARASIMHAMURTHY et al.: "Thiocarbonyl to carbonyl group transformation using CuCl and NaOH"**
**TETRAHEDRON LETTERS, vol. 39, no. 10, 1983, pages 1729-1734, Pergamon Press, LTD, GB; M.T.M. EL-WASSIMY et al.: "The reaction of t-butyl hypochlorite with thiocarbonyl compound - a convenient method for the C=S - C=O transformation"**

(73) Titulaire : **RHONE-POULENC SANTE**
**20, avenue Raymond Aron**
**F-92165 Antony Cédex (FR)**

(72) Inventeur : **Garret, Claude**
**129 rue Jean Jaurès**
**F-94120 Fontenay sous Bois (FR)**
Inventeur : **Guyon, Claude**
**17 bis avenue Henri Martin**
**F-94100 Saint Maur des Fosses (FR)**
Inventeur : **Plau, Bernard**
**1 Impasse des Rouges-Gorges**
**F-94260 Fresnes (FR)**
Inventeur : **Taurand, Gérard**
**2/124 allée Jean de la Bruyère**
**F-94000 Creteil (FR)**

(74) Mandataire : **Savina, Jacques et al**
**RHONE-POULENC SANTE, Service Brevets,**
**20 Avenue Raymond Aron**
**F-92165 Antony Cédex (FR)**

EP 0 346 238 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

Dans le domaine de l'analgésie, les récents progrès de la réceptorologie ont permis de mettre en évidence plusieurs types de récepteurs opiacés.

Les composés de type morphinique classiques agissent au niveau des récepteurs Mu, mais présentent l'inconvénient de provoquer des effets secondaires génants (phénomènes de dépendance physique et psychique, dépression respiratoire...) en conséquence desquels il est risqué d'utiliser de tels produits chez certains sujets.

Les produits plus spécifiques des récepteurs Kappa présentent une activité analgésique puissante sans provoquer les effets secondaires des composés de type morphinique classique.

Des amides dérivés de la phénothiazine de formule générale:

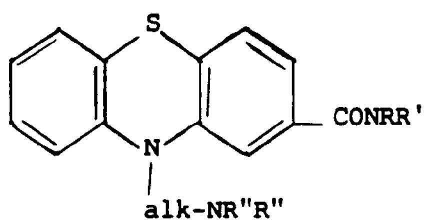

dans laquelle R est notamment un atome d'hydrogène avaient été décrits dans le brevet US 3 112 310 pour leur activité sur le système nerveux central.

Il a maintenant été trouvé qu'une classe particulière d'amides dérivés de la phénothiazine non étudiée jusqu'à présent et définie par la formule générale ;

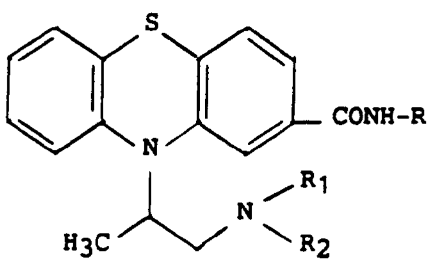

(I)

ainsi que leurs sels d'addition avec les acides présente une activité analgésique puissante liée à une affinité préférentielle pour les récepteurs Kappa, qui n'est pas observée pour les amides dérivés de la phénothiazine déjà connus.

Dans la formule générale (I), le symbole R est un radical alcoyle contenant 1 à 6 atomes de carbone en chaîne droite ou ramifiée, et les symboles $R_1$ et $R_2$ identiques ou différents sont des radicaux alcoyle droits ou ramifiés contenant 1 à 4 atomes de carbone ou forment ensemble avec l'atome d'azote auquel ils sont rattachés, un hétérocycle contenant 4 à 7 chaînons.

Il est entendu que les produits de formule générale (I) existent sous des formes isomères et que ces formes isomères ainsi que leurs mélanges entrent dans le cadre de la présente invention.

Les dérivés de la phénothiazine de formule générale (I) peuvent être obtenus par transthioamidification d'un thioamide primaire de formule générale :

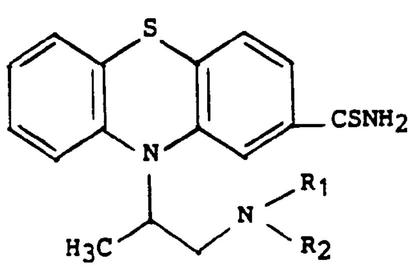

(II)

dans laquelle $R_1$ et $R_2$ sont définis comme précédemment, par action d'une amine de formule générale ;

R - $NH_2$ (III)

dans laquelle R est défini comme précédemment, suivie de l'oxydation du thioamide substitué obtenu, de formule générale :

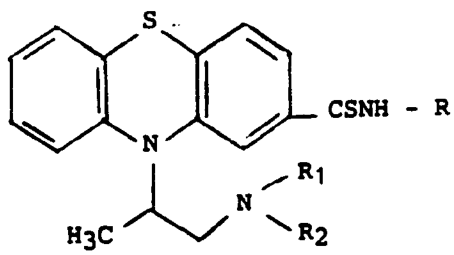

(IV)

dans laquelle R, $R_1$ et $R_2$ sont définis comme précédemment.

Dans la pratique, il n'est pas indispensable d'isoler le thioamide de formule générale (IV) pour préparer l'amide de formule générale (I).

La réaction s'effectue avantageusement dans un solvant organique tel qu'un alcool (par exemple l'éthanol, le méthanol, l'isopropanol) ou sans solvant, à une température comprise entre 100 et 250°C.

Lorsque l'on veut isoler l'amide de formule générale (I), sans isoler préalablement le thioamide de formule générale (IV), on opère directement par chromatographie ou cristallisation.

Lorsque l'on veut isoler le thioamide substitué de formule générale (IV), on opère de préférence en présence d'acide sulfhydrique, puis on oxyde le thioamide secondaire obtenu ou son sel, par toute méthode connue pour obtenir un amide à partir du thioamide correspondant sans toucher au reste de la molécule.

L'oxydation s'effectue avantageusement au moyen d'un sel mercurique (par exemple l'acétate mercurique) ou d'un sel cuivreux, dans un solvant organique tel qu'une cétone (acétone par exemple), un alcool, un ester ou un acide carboxylique tel que par exemple l'acide acétique, à une température comprise entre 0 et 100°C.

Il est également possible d'effectuer l'oxydation par analogie avec les méthodes décrites par :

- H.J. Kim et coll., Synthesis, 11, 970 (1986),
- M.T.M. El-Wassimy, Tetrahedron 39 (10), 1729 (1983),
- K.A. Jorgensen et coll., Tetrahedron 38 (9), 1163 (1982),
- A.G. Samuelson et coll., Tetrahedron Letters, 27 (33), 3911 (1986).

Les produits de formule générale (I) peuvent également être obtenus à partir d'un nitrile de formule générale :

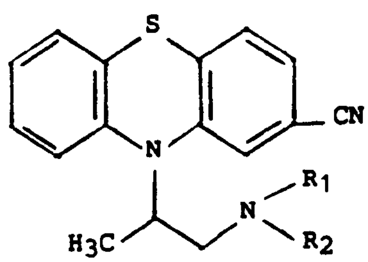

(V)

dans laquelle $R_1$ et $R_2$ sont définis comme précédemment, par toute méthode connue pour obtenir un amide substitué à partir d'un nitrile sans toucher au reste de la molécule.

Notamment on prépare in situ un imidate intermédiaire sur lequel on fait agir un dérivé halogéné de formule générale :

R - Hal (VI)

dans laquelle R est défini comme précédemment et Hal représente un atome d'iode ou de brome.

De préférence la réaction s'effectue dans un mélange alcool-alcoolate ou alcool-potasse par exemple tertiobutanol - tertiobutylate de potassium, tertiobutanol-potasse ou isobutanol - isobutylate de potassium, à une température comprise entre 50 et 150°C.

Il est également possible d'opérer en présence d'un fort excès d'une amine de formule générale (III), avec ou sans solvant, à une température comprise entre 150 et 250°C.

Le cas échéant les solvants sont avantageusement choisis parmi les alcools (éthanol, méthanol par exem-

ple), les éthers à haut point d'ébullition, les polyéthers, les hydrocarbures aromatiques (par exemple le toluène, le xylène, le chlorobenzène).

Il est également possible d'opérer selon la méthode décrite par S. LINKE, Synthesis, 4, 303 (1978) ou selon la méthode décrite par G.W. CANNON et coll., J. Org. Chem., 18, 516 (1953).

Les amides de formule générale (I) peuvent aussi être obtenus à partir d'un acide de formule générale :

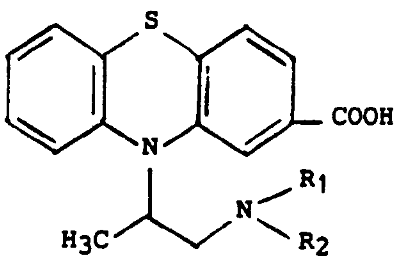

(VII)

dans laquelle $R_1$ et $R_2$ sont définis comme précédemment, par toute méthode connue pour obtenir un amide substitué à partir d'un acide sans toucher au reste de la molécule.

On opère notamment par action d'une amine de formule générale (III) sur un dérivé réactif de l'acide, par exemple le chlorure d'acide, un ester activé ou un anhydride mixte, dans un solvant organique tel qu'un éther, un solvant chloré (chlorure de méthylène, chloroforme, dichloréthane, par exemple) ou dans un amide (diméthylformamide) en présence d'un accepteur d'acide tel qu'une base organique azotée comme par exemple une trialcoylamine (triéthylamine notamment) à une température comprise entre -40 et +40°C.

Il est également possible de faire réagir l'amine de formule générale (III) directement sur l'acide en opérant en présence d'un agent de condensation tel qu'un carbodiimide (dicyclohexylcarbodiimide), le NN'-carbonyldiimidazole ou le N-hydroxybenzotriazole dans un solvant organique tel que cité ci-dessus, et à une température telle que définie ci-dessus.

Le thioamide de formule générale (II) peut être obtenu à partir d'un nitrile de formule générale (V) par toute méthode connue pour obtenir un thioamide à partir d'un nitrile, sans toucher au reste de la molécule.

On opère généralement en milieu basique anhydre, en présence d'acide sulfhydrique à une température comprise entre 0 et 100°C. La réaction s'effectue avantageusement en présence d'une base organique azotée comme la triéthylamine, dans un solvant organique tel que la pyridine.

L'acide de formule générale (VII) peut être obtenu à partir d'un nitrile de formule générale (V) par toute méthode connue pour obtenir un acide à partir d'un nitrile sans toucher au reste de la molécule. On opère notamment par hydrolyse en milieu acide ou basique dans un solvant organique à une température comprise entre 50°C et la température de reflux du mélange réactionnel. La réaction s'effectue avantageusement dans le glycol en présence de potasse.

Le nitrile de formule générale (V) peut être obtenu selon le schéma suivant :

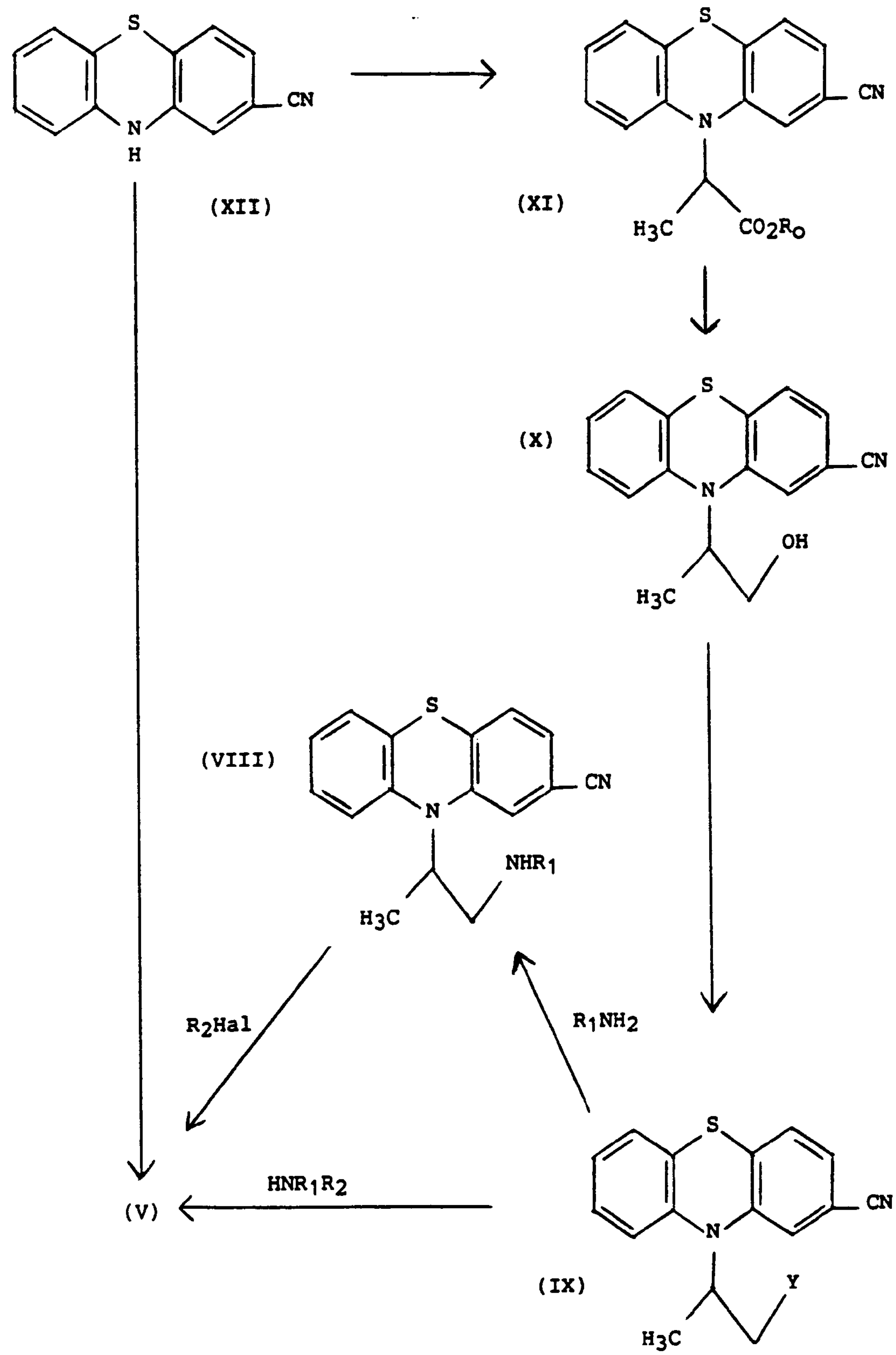


(XII) (XI) (X) (VIII) (IX) (V)

dans lequel $R_1$ et $R_2$ sont définis comme précédemment, Hal est un atome d'halogène,Y est un reste p.toluènesulfonyloxy, méthylsulfonyloxy ou diaryloxyphosphoryloxy et $R_o$ est un radical alcoyle (éthyle par exemple) et dont les conditions opératoires sont définies plus en détail ci-après dans les exemples 1, 5, 6, 8, 21, 22, 26 à 28 et 32.

Le nitrile de formule générale (XII) peut être obtenu comme décrit dans le brevet américain 2 877 224.

Les isomères des produits de formule générale (I) peuvent être obtenus selon les méthodes connues.

On opère notamment par préparation de l'isomère du dérivé de la phénothiazine de formule générale (X) qui est transformé en amide dérivé de la phénothiazine de formule générale (I) par les méthodes décrites précédemment.

Le dérivé optiquement actif du produit de formule générale (X) est notamment obtenu par préparation de l'ester d'un diacide, formation d'un sel optiquement actif, séparation des isomères par cristallisation et saponification de l'isomère obtenu.

Plus particulièrement l'ester est obtenu au moyen de l'anhydride d'un diacide comme par exemple l'anhydride phtalique, l'anhydride maléique ou succinique. Le sel est formé par addition d'une amine optiquement active, par exemple la (+) phényl-1 éthylamine, ou la (-) phényl-1 éthylamine.

Dans les exemples qui suivent, on appelle série D les dérivés de la phénothiazine préparés à partir de l'alcool de formule générale (X) pour lequel le pouvoir rotatoire en solution dans le chloroforme est positif; on appelle série L les dérivés de la phénothiazine préparés à partir de l'alcool de formule générale (X) pour lequel le pouvoir rotatoire en solution dans le chloroforme est négatif.

Les produits de formule générale (I) peuvent être purifiés par chromatographie ou cristallisation.

Les dérivés de la phénothiazine de formule générale (I) peuvent être transformés en sels d'addition avec les acides, par action d'un acide dans un solvant organique tel qu'un alcool, une cétone, un ester, un éther ou un solvant chloré. Le sel précipite, éventuellement après concentration de sa solution, il est séparé par filtration ou décantation.

Comme sels pharmaceutiquement acceptables, on peut citer les sels d'addition avec les acides minéraux tels que chlorhydrates bromhydrates, sulfates, nitrates, phosphates, ou organiques tels que acétates, propionates, succinates, maléates, fumarates, méthanesulfonates, p.toluènesulfonates, iséthionates ou des dérivés de substitution de ces composés.

Les dérivés de la phénothiazine de formule générale (I) présentent une activité analgésique et diurétique particulièrement intéressante du fait de leur affinité préférentielle pour les récepteurs Kappa et de leur faible toxicité.

Ils se sont en effet montrés actifs à des concentrations comprises entre 1 et 100 nM dans la méthode de binding à l'éthylkétocyclazocine tritiée dans des homogénats de cervelet de cobaye, inspirée de la technique de L.E. Robson et coll., Opioid binding sites of the Kappa type in ginea pig cerebellum , Neuroscience, 12, 621 (1984).

Ils se sont également montrés actifs dans la technique de l'inhibition des contractions induites par stimulation électrique sur iléon isolé de cobaye (inspirée de W.D.M. Paton, Brit. J. Pharmacol., 11, 119 (1957) à des concentrations comprises entre 1 et 100 nm.

Les produits ayant une affinité pour les récepteurs Kappa manifestent un effet diurétique [J.D. Leander, The Journal of Pharmacology and Experimental Therapeutics, 224 (1), 89 (1983) et G.R. Slizgi et coll., The Journal of Pharmacology and Experimental Therapeutics, 230 (3), 641 (1984)]. Il a été également mis en évidence par l'étude de plusieurs produits, que les produits de formule générale (I) manifestent un effet diurétique significatif chez le rat, à des doses comprises entre 1 et 20 mg/kg par voie sous-cutanée, dans la technique décrite par J.D. Leander (référence ci-dessus).

Par ailleurs la toxicité aigue ($DL_{50}$) des produits de formule (I) chez la souris est comprise entre 30 et 100 mg/kg p.o. et des doses nettement supérieures à 100 mg/kg p.o.

D'un intérêt particulier sont les produits de formule générale (I) dans laquelle le symbole R est un radical alcoyle contenant 2 à 6 atomes de carbone en chaine droite ou ramifiée, et les symboles $R_1$ et $R_2$ identiques ou différents sont les radicaux alcoyle droits ou ramifiés contenant 1 à 3 atomes de carbone ou forment ensemble avec l'atome d'azote auquel ils sont rattachés un un hétérocycle contenant 5 à 7 chainons.

Parmi ces produits plus spécialement actifs sont les produits de formule générale (I) dans laquelle le symbole R est un radical alcoyle contenant 3 à 6 atomes de carbone en chaine droite ou ramifiée et les symboles $R_1$ et $R_2$ identiques ou différents sont des radicaux alcoyle droits ou ramifiés contenant 2 ou 3 atomes de carbone ou forment ensemble avec l'atome d'azote auquel ils sont rattachés, un hétérocycle contenant 5 à 7 chainons, sous forme d'un mélange d'isomères ou sous forme des isomères de série L, et notamment les produits suivants :

- le N-propyl [(pyrrolidinyl-1)-1 propyl-2]-10 phénothiazinecarboxamide-2 sous ses formes isomères ou leurs mélanges,
- le N-(méthyl-3 butyl) [(pyrrolidinyl-1)-1 propyl-2]-10 phénothiazinecarboxamide-2 sous ses formes isomères ou leurs mélanges,
- le N-(méthyl-2 propyl) [(pyrrolidinyl-1)-1 propyl-2]-10 phénothiazinecarboxamide-2 sous ses formes isomères ou leurs mélanges,
- le N-butyl [(pyrrolidinyl-1)-1 propyl-2]-10 phénothiazinecarboxamide-2 sous ses formes isomères ou

leurs mélanges,

- le N-(méthyl-3 butyl) [pipéridino-1 propyl-2]-10 phénothiazinecarboxamide-2 sous ses formes isomères ou leurs mélanges.

Les exemples suivants, donnés à titre non limitatif, illustrent la présente invention.

## EXEMPLE 1

A une solution de 4,38 g de [diéthylamino-1 propyl-2-(2RS)]-10 phénothiazinecarbothioamide-2 dans 60 cm3 d'éthanol absolu, on ajoute 4,85 cm3 de propylamine. Le mélange est porté à 150°C pendant 16,5 heures. Le mélange réactionnel est dilué par 200 cm3 d'acétate d'éthyle puis lavé par 3 fois 100 cm3 d'eau distillée. La phase organique est séchée sur sulfate de magnésium, filtrée puis concentrée à sec sous pression réduite (30 mm de Hg; 4 kPa) à 40°C. Le résidu est purifié par chromatographie sur une colonne (hauteur: 24,0 cm; diamètre : 4 cm) de gel de silice (0,04-0,06 mm) avec une légère surpression d'azote (40 kPa) en éluant avec des mélanges de cyclohexane et d'acétate d'éthyle 90-10 (1,5 litre) 75-25 (3 litres) (en volumes) puis par de l'acétate d'éthyle pur (1,5 litre) et par un mélange d'acétate d'éthyle et de méthanol 95-5 (3 litres) (en volumes) en recueillant des fractions de 100 cm3. Les fractions 20 à 35 sont réunies et concentrées sous pression réduite (30 mm de Hg; 4 kPa) pour donner 3,95 g d'une meringue orangée. 0,13 g de ce produit sont dissous dans 1 cm3 de propanol-2 bouillant et additionnés de 0,039 g d'acide fumarique dissous dans 1 cm3 de propanol-2. La cristallisation est amorcée par grattage. Le mélange est laissé sous agitation pendant 24 heures à 5°C puis les cristaux sont filtrés, séchés à 50°C sous pression réduite (5 mm de Hg; 0,7 kPa) pour donner 0,044 g de fumarate de [diéthylamino-1 propyl-2-(2RS)]-10 N-propyl phénothiazinecarboxamide-2 fondant à 110°C.

RMN du proton (250 MHz, DMSO, δ en ppm, J en Hz) :

0,87 (Mt, 9H, $-CH_3$ du propyle et $-N(CH_2C\underline{H}_3)_2$); 1,52 (Mt, 2H, $-CH_2-C\underline{H}_2-CH_3$); 1,62 (D, J = 7, $-CH_3$); 2,53 (Mt, masqué par la bande du solvant, $-N(C\underline{H}_2CH_3)_2$); 2,72 (DD, J = 13 et 6, 1H, 1H du $>N-CH_2-$); 3,05 (DD, J = 13 et 7,5, 1H, 1H du $>N-CH_2-$); 3,20 (Mt, J = 5,5 et 7, 2H, $-CONH-C\underline{H}_2-$); 4,18 (Mt, J = 7,5 -7 et 6, 1H, $>N-CH<$); 6,6 (S,2H, -CH = CH- du fumarate); 6,9 à 7,25 (Mt, 5H, aromatiques); 7,39 (DD, J = 8 et 1, 1H, - H en 3); 7,52 (D, J = 1, 1H, - H en 1); 8,4 (T, J = 5,5, 1H, -CONH-).

Spectre infra-rouge (KBr), bandes caractéristiques en $cm^{-1}$ :

3280, 3070, 2960, 2940, 2880, 2600, 2500, 2270, 1695, 1630, 1590, 1550, 1470, 1400, 1350, 1320, 1235, 990, 980, 870, 840, 750, 635.

Le [diéthylamino-1 propyl-2-(2RS)]-10 phénothiazinecarbothioamide-2 peut être préparé de la manière suivante :

Un mélange de 8,44 g de [diéthylamino-1 propyl-2-(2RS)]-10 phénothiazinecarbonitrile-2 et de 3,5 cm3 de triéthylamine dans 60 cm3 de pyridine anhydre est saturé par bullage d'acide sulfhydri que pendant 5 heures à 25°C. La solution limpide obtenue est maintenue sous agitation pendant 12 heures à 25°C, puis le mélange est dégazé par bullage d'azote pendant 90 minutes. Le mélange réactionnel est dilué par 500 cm3 d'acétate d'éthyle et lavé par 8 fois 200 cm3 d'eau distillée. La phase organique est séchée sur sulfate de magnésium, filtrée et concentrée à sec sous pression réduite (30 mm de Hg; 4 kPa). Le résidu est purifié par chromatographie sur colonne d'alumine basique (0,05-0,16 mm) (hauteur ; 31 cm; diamètre ; 2,6 cm) en éluant par des mélanges de cyclohexane et d'acétate d'éthyle 80-20 (5 litres) et 50-50 (5 litres) (en volumes) et en recueillant des fractions de 100 cm3. Les fractions 4 à 90 sont réunies et concentrées sous pression réduite (30 mm de Hg; 4 kPa) pour donner 8,54 g de [diéthylamino-1 propyl-2-(2RS)]-10 phénothiazinecarbothioamide-2 sous la forme d'un miel jaune orangé.

Spectre RMN du proton (250 MHz, DMSO, δ en ppm, J en Hz):

0,84 (T, J = 7, 6H, $-N(CH_2C\underline{H}_3)_2$); 1,62 (D, J = 7, 3H, $-CH_3$); 2,45 (Mt, environ 4H, $-N(C\underline{H}_2CH_3)_2$); 2,69 (DD, J = 13,5 et 6, 1H, 1H du $>N-CH_2-$); 2,99 (DD, J = 13,5 et 6,5, 1H, 1H du $>N-CH_2-$); 4,13 (Mt, J = 7 -6,5 et 6, 1H, $>N-CH<$); 6,9 à 7,2 (Mt, 5H, aromatiques); 7,39 (DD, J = 8 et 1, 1H, -H en 3); 7,74 (D, J = 1, 1H, -H en 1); 9,47 et 9,83 (2S, 1H chacun, $-CSNH_2$).

Le [diéthylamino-1 propyl-2-(2RS)]-10 phénothiazinecarbonitrile-2 peut être préparé de la manière suivante :

A une solution de 114,4 g de p-toluènesulfochlorure dans 600 cm3 de N,N-diméthylformamide, on ajoute goutte à goutte une solution de 78,7 g de diéthylamino-1 propanol-2 dans 60 cm3 de N,N-diméthylformamide en 55 minutes. Le mélange est agité à 25°C pendant 12 heures.

A une solution de 44,86 g de phénothiazinecarbonitrile-2 dans 600 cm3 de N,N-diméthylformamide, on ajoute 19,2 g d'hydrure de sodium à 50% en dispersion dans la vaseline en 20 minutes. Le mélange obtenu est ensuite chauffé à 110°C puis on ajoute en 35 minutes la solution de chlorhydrate de diéthylamino-1 p-toluènesulfonate de propyle-2 précédemment préparée. Le mélange réactionnel est agité à 110°C pendant 7 heures puis dilué, après refroidissement, par 2 litres d'acétate d'éthyle. Le mélange est lavé par 5 fois 1 litre d'eau

distillée. La phase organique est extraite par 240 cm3 d'acide chlorhydrique 4N et la phase aqueuse acide est lavée par 750 cm3 d'acétate d'éthyle, puis elle est alcalinisée par 250 cm3 de lessive de soude 5N. Le milieu alcalin est alors extrait par 1250 cm3 d'acétate d'éthyle. La phase organique est lavée par 3 fois 300 cm3 d'eau distillée, séchée sur sulfate de magnésium, filtrée et concentrée à sec sous pression réduite (30 mm de Hg; 4 kPa) à 40°C. Le résidu est agité en présence de 100 cm3 d'éther éthylique; il y a formation d'un précipité qui est filtré et le filtrat est purifié par chromatographie sur colonne de gel de silice (0,2 - 0,063 mm) (hauteur : 82 cm; diamètre : 4,5 cm) en éluant par un mélange de cyclohexane et d'acétate d'éthyle 80-20 (7 litres) (en volumes) et en recueillant des fractions de 100 cm3. Après concentration à sec sous pression réduite (30 mm de Hg; 4 kPa) à 50°C, on obtient à partir des fractions 4 à 9, 6,76 g de [diéthylamino-1 propyl-2-(2RS)]-10 phénothiazinecarbonitrile-2, et à partir des fractions 10 à 22, 8,5 g d'un mélange de [diéthylamino-1 propyl-2-(2RS)]-10 phénothiazinecarbonitrile-2 et de [diéthylamino-2 propyl-1-(1RS)]-10 phénothiazinecarbonitrile-2. Le mélange est purifié par chromatographie sur colonne de gel de silice (0,2 - 0,063 mm) (hauteur : 67 cm; diamètre : 3,0 cm) en éluant par un mélange de cyclohexane et d'acétate d'éthyle 85-15 (3 litres) (en volumes) et en recueillant des fractions de 50 cm3. Les fractions 22 à 29 sont réunies et concentrées à sec à 50°C sous pression réduite (30 mm de Hg; 4 kPa) pour donner 2,76 g de [diéthylamino-1 propyl-2-(2RS)]-10 phénothiazinecarbonitrile-2.

Spectre RMN du proton (250 MHz, DHSO, δ en ppm, J en Hz):

0,85 (T, J = 7,5, 6H, $-N(CH_2C\underline{H}_3)_2$); 1,57 (D, J = 7, 3H, $-CH_3$); 2,25 à 2,57 (Mt, environ 4H, $-N(C\underline{H}_2CH_3)_2$); 2,63 (DD, J = 13,5 et 6, 1H, 1H du $>NCH_2-$); 2,98 (DD, J = 13,5 et 6,5, 1H, 1H du $>NCH_2-$); 4,08 (Mt, J = 7- 6,5 et 6, 1H $>N-CH<$); 6,9 à 7,25 (Mt, 5H, aromatiques); 7,31 (DD, J = 8 et 1, 1H, -H en 3), 7,59 (D, J = 1, 1H, -H en 1).

EXEMPLE 2

A une solution de 1,86 g de [diéthylamino-1 propyl-2-(2RS)]-10 phénothiazinecarbothioamide-2 dans 25 cm3 d'éthanol absolu, on ajoute 2,9 cm3 de méthyl-3 butylamine. Le mélange est porté à 150°C pendant 16 heures puis dilué par 100 cm3 d'acétate d'éthyle et lavé par 3 fois 50 cm3 d'eau distillée. La phase organique est séchée sur sulfate de magnésium, filtrée puis concentrée à sec sous pression réduite (30 mm de Hg; 4 kPa), à 40°C. Le résidu est purifié parchromatographie sur une colonne (hauteur : 18,5 cm; diamètre : 2,6 cm) de gel de silice (0,04-0,06 mm) avec une légère surpression d'azote (40 kPa) en éluant avec un mélange de cyclohexane et d'acétate d'éthyle 50-50 (1 litre) (en volumes) puis par de l'acétate d'éthyle pur (500 cm3) en recueillant des fractions de 60 cm3. Les fractions 8 à 18 sont réunies et concentrées à sec sous pression réduite (30 mm de Hg; 4 kPa) à 40°C pour donner 0,36 g d'une meringue orangée. Cette dernière est purifiée par chromatographie sur colonne d'alumine basique (0,05-0,16 mm) (hauteur ; 8,5 cm; diamètre : 1,2 cm3 en éluant par un mélange de cyclohexane et d'acétate d'éthyle 90-10 (150 cm3) (en volumes) et en recueillant des fractions de 7 cm3. Les fractions 6 à 20 sont réunies et concentrées sous pression réduite (30 mm de Hg; 4 kPa) à 40°C pour donner 0,22 g d'une laque jaunâtre. Ce produit est dissous dans 2 cm3 de propanol-2 bouillant et additionné de 0,06 g d'acide fumarique dissous dans 1 cm3 de propanol-2. La cristallisation est amorcée par grattage. Le mélange est laissé sous agitation pendant 24 heures puis les cristaux sont filtrés, lavés par 3 fois 1 cm3 d'oxyde de diéthyle, séchés à 50°C sous pression réduite (5 mm de Hg; 0,7 kPa) pour donner 0,13 g de fumarate de [diéthylamino-1 propyl-2-(2RS)]-10 N-(méthyl-3 butyl) phénothiazinecarboxamide-2 fondant à 138°C.

Spectre RMN du proton (250 MHz, DMSO, δ en ppm, J en Hz):

0,87 (T, J = 7, 6H, $-N(CH_2C\underline{H}_3)_2$); 0,92 (D, J = 7, 6H, $-CH(C\underline{H}_3)_2$; 1,42 (Q, J = 7, 2H $>NCH_2C\underline{H}_2-$); 1,62 (Mt, 1H, $>CH-$ du méthyl-3 butyle); 1,64 (D, J = 7, 3H, $-CH_3$); 2,52 (Mt, masqué par la bande du DMSO, $>N-C\underline{H}_2-CH_2-$); 2,73 (DD, J = 13 et 6, 1H, 1H du $>N-CH_2-$) ; 3,05 (DD, J = 13 et 7,5, 1H, 1H du $>N-CH_2-$); 3,26 (Mt, 2H, $-CONH-C\underline{H}_2-$); 4,17 (Mt, J = 7,5 - 7 et 6, 1H, $>N-CH<$); 6,62 (S,2H, -CH=CH- du fumarate); 6,9 à 7,25 (Mt, 5H, aromatiques); 7,39 (DD, J = 8 et 1, 1H, -H en 3); 7,52 (D, J = 1, 1H, -H en 1); 8,33 (T, J = 6, 1H, -CONH-).

Spectre infra-rouge (KBr), bandes caractéristiques en $cm^{-1}$ :

3300, 3060, 2960, 2940, 2870, 2640, 2500, 1900, 1705, 1630, 1590, 1555, 1460, 1415, 1385, 1310, 1230, 985, 880, 830, 755, 635.

EXEMPLE 3

A une solution de 0,87 g de [diéthylamino-1 propyl-2-(2RS)]-10 N-éthyl phénothiazinecarbothioamide-2 dans 11 cm3 d'acide acétique glacial, on ajoute goutte à goutte une solution de 0,70 g d'acétate mercurique dans 11 cm3 d'acide acétique pendant une période de 10 minutes. Le mélange réactionnel est agité 90 minutes à 25°C puis filtré sur verre fritté garni de supercel. La célite est lavée par 2 fois 2 cm3 d'acide acétique et les filtrats réunis sont concentrés à sec sous pression réduite (30 mm de Hg; 4 kPa) à 40°C pour donner un résidu

qui est dilué dans 50 cm3 d'acétate d'éthyle. La phase organique est lavée par 20 cm3 de soude 1N, 3 fois 20 cm3 d'eau distillée puis par une fois 20 cm3 de saumure, séchée sur sulfate de magnésium, filtrée et concentrée à sec sous pression réduite (30 mm de Hg; 4 kPa) à 40°C pour donner un résidu qui est purifié par chromatographie sur une colonne (hauteur ; 15,5 cm; diamètre ; 1,6 cm) d'alumine (0,05-0,16 mm) en éluant avec des mélanges de cyclohexane et d'acétate d'éthyle 80-20 (375 cm3) 75-25 (3 litres) (en volumes) en recueillant des fractions de 25 cm3. Les trois premiers litres sont éliminés et les fractions 3 à 12 sont réunies et concentrées à sec sous pression réduite (30 mm de Hg; 4 kPa à 40°C) pour donner 0,54 g de [diéthylamino-1 propyl-2-(2RS)]-10 N-éthyl phénothiazinecarboxamide-2.

A une solution de 0,44 g de [diéthylamino-1 propyl-2-(2RS)]-10 N-éthyl phénothiazinecarboxamide-2 dans 5 cm3 de propanol-2 bouillant, on ajoute 0,13 g d'acide fumarique dissous dans 5 cm3 de propanol-2 au reflux. La cristallisation est amorcée par grattage et le mélange est agité pendant 5 heures à 25°C. Les cristaux sont filtrés sur verre fritté, lavés par 2 fois 3 cm3 de propanol-2 glacé et séchés à l'air libre pour donner 0,51 g de fumarate de [diéthylamino-1 propyl-2-(2RS)]-10 N-éthyl phénothiazinecarboxamide-2 fondant à 135°C.

RMN du proton (400 MHz, DMSO, δ en ppm, J en Hz).

0,90 et 1,27 (2T, J = 7, respectivement 6H et 3H, $-N(CH_2C\underline{H_3})2$ et $>NHCH_2C\underline{H_3}$); 1,67 (D, J = 7, 3H, $-CH_3$); 2,61 (Mt, 4H, $-N(C\underline{H_2}CH_3)_2$); 2,84 (DD, J = 14 et 6, 1H, 1H du $>N-CH_2-$); 3,15 (DD, J = 14 et 7,5, 1H, 1H du $>NCH_2-$); 3,28 (Mt, J = 7 et 5,5, 2H, $-CONH-C\underline{H_2}-$); 4,29 (Mt, J = 7,5 -7 et 6, 1H $>N-CH<$); 6,6 (S, 2H, -CH=CH- du fumarate); 6,9 à 7,3 (Mt, 5H, aromatiques); 7,42 (D, J = 8, 1H, -H en 3); 7,54 (S, 1H, -H en 1); 8,48 (T, J = 5,5, 1H, -CONH-).

Spectre infra-rouge (KBr), bandes caractéristiques en $cm^{-1}$ :
3270, 3060, 3030, 2975, 2935, 2880, 2740, 2650, 2500, 1920, 1700, 1630, 1590, 1555, 1550, 1460, 1420, 1315, 1230, 990, 755, 640.

Le [diéthylamino-1 propyl-2 (2RS)]-10 N-éthyl phénothiazinecarbothioamide-2 peut être préparé de la manière suivante :

A une solution de 1,86 g de [diéthylamino-1 propyl-2-(2RS)]-10 phénothiazinecarbothioamide-2 dans 25 cm3 d'éthanol absolu, on ajoute 2,80 cm3 d'éthylamine en solution éthanolique 9N. Le mélange est porté à 150°C pendant 16 heures. Le mélange réac- tionnel est concentré à sec sous pression réduite (30 mm de Hg; 4 kPa). Le résidu est purifié par chromatographie sur une colonne (hauteur : 17,5 cm; diamètre; 2,8 cm) de gel de silice (0,040,06 mm) avec une légère surpression d'azote (40 kPa) en éluant avec des mélanges de cyclohexane et d'acétate d'éthyle 80-20 (1,5 litre) 50-50 (1,5 litre) (en volumes) puis par de l'acétate d'éthyle pur (1,5 litre) en recueillant des fractions de 100 cm3. Les fractions 9 à 39 sont réunies et concentrées à sec sous pression réduite (30 mm de Hg; 4 kPa) pour donner 0,93 g de [diéthylamino-1 propyl-2-(2RS)]-10 N-éthyl phénothiazinecarbothioamide-2.

Spectre de RMN du proton (250 MHz, DMSO, δ en ppm, J en Hz) :

1,23 (T, J = 7, 3H, $-NHCH_2C\underline{H_3}$); 3,7 (Mt, $-CSNH-C\underline{H_2}-$); 6,9 à 7,25 (Mt, 5H, aromatiques).

## EXEMPLE 4

A une solution de 0,6 g de fumarate neutre de N-propyl [(pyrrolidinyl-1)-1 propyl-2-(2RS)]-10 phénothiazinecarbothioamide-2 dans 10 cm3 d'acide acétique, on ajoute sous agitation 0,38 g d'acétate mercurique et on poursuit l'agitation pendant 4 heures 30 minutes à une température voisine de 20°C. Le mélange réactionnel noir est dilué avec 25 cm3 d'eau distillée et 50 cm3 d'acétate d'éthyle puis filtré et alcalinisé sous agitation avec une solution aqueuse de soude 4N jusqu'à pH 13. Après décantation, la phase organique est séparée et la phase aqueuse est extraite avec 20 cm3 d'acétate d'éthyle. Les phases organiques réunies sont lavées successivement avec 50 cm3 d'une solution aqueuse saturée de chlorure de sodium. Après séchage sur sulfate de magnésium, filtration et concentration à sec sous pression réduite (30 mm de Hg; 4 kPa) à 40°C, l'huile jaune résiduelle (0,43 g) est dissoute dans 2 cm3 d'éthanol puis additionnée d'une solution de 0,14 g d'acide fumarique dans 5 cm3 d'éthanol, concentré à mi-volume sous pression réduite (30 mm de Hg; 4 kPa) à 40°C et additionnée de 20 cm3 d'éther éthylique. Après 2 heures d'agitation à une température voisine de 20°C, on essore le solide formé et sèche sous pression réduite (5 mm de Hg; 0,7 kPa) à 40°C. On obtient ainsi 0,38 g de fumarate acide de N-propyl [(pyrrolidinyl-1)-1 propyl-2-(2RS)]-10 phénothiazinecarboxamide-2 sous forme d'un solide jaune pâle fondant à 75-80°C (fusion pâteuse).

RMN du proton (250 MHz, DMSO, δ en ppm, J en Hz).

0,88 (T, J = 7, 3H, $-CH_3$ propyle); 1,55 (Mt, 2H, $-C\underline{H_2}-CH_3$ propyle); 1,65 (D, J = 6,5, 3H, $-CH_3$); 1,73 (Mt, 4H, $-CH_2-$ de la pyrrolidine); 2,75 (Mt, 4H, $>N-CH_2-$ de la pyrrolidine); 3,20 (Mt, 4H, $>N-CH_2-$ et $-CONHC\underline{H_2}-$); 4,35 (Mt, 1H, $>N-CH<$); 6,55 (S, 2H, -CH=CH- du fumarate); 6,9 à 7,3 (Mt, 5H, aromatiques); 7,42 (D, J = 8, 1H, -H en 3); 7,50 (S, 1H, -H en 1); 9,46 (T, J = 5,5, 1H, -CONH-).

Spectre infra-rouge (KBr), bandes caractéristiques en $cm^{-1}$ :

3300, 3060, 2960, 2930, 2870, 2600, 2480, 1900, 1705, 1635, 1590, 1555, 1540, 1460, 1410, 1375, 1305, 1230, 980, 830, 750, 635.

Le fumarate neutre N-propyl (pyrrolidinyl-1)-1 propyl-2-(2RS)]-10 phénothiazinecarbothioamide-2 peut être préparé de la manière suivante :

On sature d'acide sulfhydrique une solution de 0,9 g de [(pyrrolidinyl-1)-1 propyl-2-(2RS)]-10 phénothiazinecarbothioamide-2 et de 3 cm3 de propylamine dans 18 cm3 d'éthanol absolu et on porte le mélange à une température voisine de 100°C pendant 16 heures. Après refroidissement, on concentre à sec sous pression réduite (30 mm de Hg; 4 kPa) à 40°C pour obtenir une huile jaune. Cette huile est purifiée par chromatographie sur une colonne (hauteur : 25 cm; diamètre ; 2,5 cm) de gel de silice (0,04-0,063 mm) sous une légère surpression d'azote (40 kPa) en éluant successivement avec 100 cm3 de chlorure de méthylène puis 300 cm3 d'un mélange de chlorure de méthylène et de méthanol (95-5 en volumes) et en recueillant des fractions de 50 cm3. Les fractions 3 à 5 sont réunies et concentrées à sec sous pression réduite (30 mm de Hg; 4 kPa) à 40°C. L'huile jaune résiduelle (1 g) est dissoute dans 9 cm3 d'éthanol au reflux et additionnée d'une solution bouillante de 0,29 g d'acide fumarique dans 5 cm3 d'éthanol. On laisse refroidir et on maintient pendant 4 heures à une température voisine de 5°C. Les cristaux formés sont essorés, lavés avec 2 cm3 d'éthanol glacé et séchés sous pression réduite (5 mm de Hg; 0,7 kPa) à 35°C. On obtient ainsi 1,12 g de fumarate neutre de N-propyl [(pyrrolidinyl-1)-1 propyl-2-(2RS)]-10 phénothiazinecarbothioamide-2 sous forme de cristaux jaunes fondant à 150-152°C.

EXEMPLE 5

A une solution de 9,5 g de N-propyl [(pyrrolidinyl-1)-1 propyl-2]-10 phénothiazinecarbothioamide-2, série L, dans 100 cm3 d'éthanol, on ajoute 2,7 g d'acide fumarique. La solution obtenue est concentrée à sec sous pression réduite (30 mm de Hg; 4 kPa) à 40°C. Le résidu jaune meringué est repris par 200 cm3 d'acide acétique. A la solution obtenue, on ajoute 7,3 g d'acétate mercurique et on agite pendant 16 heures à une température voisine de 20°C. La suspension noire obtenue est diluée par 200 cm3 d'eau distillée, filtrée. Le filtrat jaune est concentré sous pression réduite (30 mm de Hg; 4 kPa) à 40°C. Le résidu est repris par 250 cm3 d'acétate d'éthyle et 50 cm3 d'eau distillée, puis additionné de soude (d = 1,33) jusqu'à pH 13. La phase aqueuse est décantée et extraite par 250 cm3 d'acétate d'éthyle. Les phases organiques sont réunies, lavées successivement par 2 fois 100 cm3 d'eau distillée et par 100 cm3 d'une solution aqueuse saturée de chlorure de sodium et séchées sur sulfate de magnésium. Après filtration, le filtrat jaune est concentré sous pression réduite (30 mm de Hg; 4 kPa) à 40°C et on obtient ainsi 9,2 g d'huile jaune brute. Ce résidu est purifié par chromatographie sur une colonne (hauteur 22 cm; diamètre : 4 cm) de gel de silice (0,2-0,063 mm) en éluant avec un mélange de chlorure de méthylène et de méthanol (95 -5 en volumes). Les 1500 premiers cm3 sont éliminés et les 1500 cm3 suivants sont concentrés sous pression réduite (30 mm de Hg; 4 kPa) à 40°C pour donner 7,3 g de N-propyl [(pyrrolidinyl-1)-1 propyl-2]-10 phénothiazinecarboxamide-2, série L, sous forme d'une gomme jaune.

$[\alpha]_D^{20}$ = +23,4 ± 12°C (0,4 %; méthanol).

A une solution de 7,2 g de N-propyl [(pyrrolidinyl-1)-1 propyl-2]-10 phénothiazinecarboxamide-2, série L, dans 80 cm3 d'acétate d'éthyle anhydre, on ajoute goutte à goutte en 5 minutes, 6,2 cm3 d'une solution d'acide chlorhydrique 3,3N dans l'oxyde d'isopropyle. Le produit se dépose sur les parois et cristallise par grattage. La suspension obtenue est maintenue une heure à une température voisine de 5°C. Le solide est essoré, lavé par 3 fois 5 cm3 d'acétate d'éthyle anhydre et séché sous pression réduite (5 mm de Hg; 0,7 kPa) à 40°C pour donner 7,1 g de chlorhydrate de N-propyl [(pyrrolidinyl-1)-1 propyl-2]-10 phénothiazinecarboxamide-2, série L, sous la forme d' un solide blanc fondant à 190°C.

$[\alpha]_D^{20}$ = + 19,4 ± 0,6° (0,85%, diméthylformamide).

RMN proton (250 MHz, DMSO, δ en pmm, J en Hz).

0,9 (T, J = 7,5, 3H, $-CH_2-C\underline{H}_3$); 1,57 (Mt, 2H, $-C\underline{H}_2CH_3$); 1,79 (D, J = 7, 3H, $-CH_3$); 1,75 à 2 (Mt, 4H, $-C\underline{H}_2-C\underline{H}_2-$ de la pyrrolidine); 2,85 - 3,10 - 3,60 et 3,75 (4Mf de 1H chacun,

$$-C\underline{H}_2-\overset{}{\underset{|}{N}}-C\underline{H}_2-$$

de la pyrrolidine); 3,24 (Mt, 2H, $-CONH-C\underline{H}_2-$; 3,77 (AB, 2H, >N-$CH_2$-); 4,76 (Mt, 1H, >N-CH<); 7 à 7,4 (Mt, 5H, aromatiques); 7,53 (S, 1H, -H en 1); 7,55 (D, J = 8, 1H, -H en 3); 8,66 (T, J = 5,5, 1H, -CONH-); 10,7 (Mf, 1H, $-NH^+$).

Spectre infra-rouge (KBr), bandes caractéristiques en $cm^{-1}$ :

3260, 3060, 2965, 2935, 2880, 2670, 2570, 2470, 1645, 1595, 1535, 1465, 1415, 1380, 1360, 1235, 875,

835, 755.

Le N-propyl [(pyrrolidinyl-1)-1 propyl-2]-10 phénothiazine carbothioamide-2, série L, peut être préparé de la manière suivante :

Un mélange de 10,3 g de (pyrrolidinyl-1)-1 propyl-2]-10 phénothiazinecarbothioamide-2, série L, et de 32 cm3 de propylamine dans 150 cm3 d'éthanol est saturé avec de l'acide sulfhydrique puis chauffé 16 heures à 105°C en autoclave. Après refroidissement, la solution est concentrée à sec sous pression réduite (30 mm de Hg; 4 kPa) à 40°C. On obtient 10,2 g d'une huile orangée qui est purifiée par chromatographie sur colonne de silice (0,2-0,063 mm) (diamètre : 4 cm; hauteur : 25 cm) en éluant par un mélange de chlorure de méthylène et de méthanol 95-5 (2 litres) (en volumes) et en recueillant des fractions de 100 cm3. Les fractions 13 à 17 sont réunies et concentrées sous pression réduite (30 mm de Hg ; 4 kPa) à 40°C. On obtient 9,5 g de N-propyl [(pyrrolidinyl-1)-1 propyl-2]-10 phénothiazinecarbothioamide-2, série L, sous forme d'une huile jaune.

$[\alpha]_D^{20}$ = +30,4 ± 0,6° (1%; méthanol).

Le [(pyrrolidinyl-1)-1 propyl-2]-10 phénothiazinecarbothio amide-2, série L, peut être préparé de la manière suivante :

Un mélange de 11,2 g de [(pyrrolidinyl-1)-1 propyl-2]-10 phénothiazinecarbonitrile-2, série L et de 4,7 cm3 de triéthylamine dans 225 cm3 de pyridine anhydre est saturé par bullage d'acide sulfhydrique pendant une heure à 25°C. On agite pendant 20 heures à 25°C. Le mélange réactionnel est dégazé par barbottage d'azote puis dilué par 500 cm3 d'acétate d'éthyle et lavé par 500 cm3 d'eau distillée. La phase aqueuse est extraite à nouveau par 250 cm3 d'acétate d'éthyle. Les phases organiques réunies sont lavées par 2 fois 200 cm3 d'eau et 200 cm3 de solution aqueuse saturée de chlorure de sodium, séchées sur sulfate de magnésium et concentrées sous pression réduite (30 mm de Hg; 4 kPa) à 40°C. On obtient 14,4 g d'une huile orangée qui est purifiée par chromatographie sur colonne de silice (0,2 - 0,063 mm) (diamètre : 4 cm; hauteur : 30 cm) en éluant par un mélange de chlorure de méthylène et de méthanol 95-5 (3 litres) (en volumes) et en recueillant des fractions de 120 cm3. Les fractions 12 à 27 sont réunies et concentrées sous pression réduite (30 mm de Hg; 4 kPa) à 40°C. On obtient 10,3 g de [(pyrrolidinyl-1)-1 propyl-2]-10 phénothiazinecarbothioamide-2, série L, sous forme d'une meringue orangée.

$[\alpha]_D^{20}$ = -43 ± 0,7° (1%; chloroforme).

Le [(pyrrolidinyl-1)-1 propyl-2]-10 phénothiazinecarbonitrile-2, série L, peut être préparé de la manière suivante :

Un mélange de 25 g de méthanesulfonate de (cyano-2 phénothiazinyl-10)-2 propyle-1, série L, et de 26,6 cm3 de pyrrolidine dans 250 cm3 de toluène est chauffé pendant 55 heures à une température voisine de 90°C. Le mélange réactionnel est concentré à sec sous pression réduite (30 mm de Hg; 4 kPa) à 40°C. Le résidu est repris par 500 cm3 d'éther éthylique et extrait par 2 fois 100 cm3 par une solution aqueuse d'acide méthanesulfonique 2N. La phase aqueuse est alcalinisée par de la lessive de soude à une température voisine de 5°C et extraite par 2 fois 250 cm3 d'éther éthylique. Les phases organiques réunies sont lavées successivement par 100 cm3 d'éther éthylique. Les phases organiques réunies sont lavées successivement par 100 cm3 d'eau distillée et par 100 cm3 d'une solution aqueuse saturée de chlorure de sodium, séchées sur sulfate de magnésium, filtrées et le filtrat jaune est concentré à sec sous pression réduite (30 mm de Hg; 4 kPa) à 40°C. Les 17,1 g de l'huile orangée ainsi obtenue sont chromatographiés sur une colonne (hauteur : 45 cm; diamètre : 4 cm) de gel de silice (0,063-0,2 mm) en éluant par 1 litre d'un mélange de chlorure de méthylène et de méthanol (95-5 en volumes) et en recueillant des fractions de 100 cm3. Les fractions 3 à 7 sont réunies et concentrées à sec sous pression réduite (30 mm de Hg; 4 kPa) à 40°C. On obtient ainsi 11,2 g de [(pyrrolidinyl-1)-1 propyl-2]-10 phénothiazinecarbonitrile-2, série L, sous forme d'une huile jaune.

$[\alpha]_D^{20}$ = + 9,7 ± 0,3° (1,2%; chloroforme).

Le méthanesulfonate de (cyano-2 phénothiazinyl-10)-2 propyle-1, série L, peut être préparé de la manière suivante :

A une solution de 12,6 g de (hydroxy-1 propyl-2)-10 phénothiazinecarbonitrile-2, série L, dans 126 cm3 de chlorure de méthylène refroidie à une température voisine de 5°C, on ajoute, sous agitation, 10 cm3 de triéthylamine puis on verse, goutte à goutte pendant 25 minutes, une solution de 5,6 cm3 de chlorure de méthanesulfonyle dans 56 cm3 de chlorure de méthylène et on poursuit l'agitation pendant 1 heure 15 minutes à une température voisine de 10-15°C. Le mélange réactionnel est lavé successivement par deux fois 100 cm3 d'eau distillée et par 100 cm3 d'une solution aqueuse saturée de chlorure de sodium, séché sur sulfate de magnésium, filtré et concentré à sec sous pression réduite (30 mm de Hg; 4 kPa) à 40°C. On obtient ainsi 16,2 g de méthanesulfonate de (cyano-2 phénothiazinyl-10)-2 propyle-1, série L, sous forme d'une huile orangée ($[\alpha]_D^{20}$ = +29,9 ± 0,3°; 2,4%; chloroforme) qui est utilisée sans autre purification pour la suite des synthèses.

L'(hydroxy-1 propyl-2)-10 phénothiazinecarbonitrile, série L, peut être préparé de la manière suivante :

A une solution de 42 g de phtalate de (+)-(cyano-2 phénothiazinyl-10)-2 propyle et de phényl-1 éthylammonium-(R) dans 420 cm3 d'éthanol au reflux, on ajoute 84,9 cm3 d'une solution de potasse alcoolique 1,97M et on poursuit le reflux sous agitation pendant 15 minutes. Le mélange réactionnel est alors versé sur 500 g de glace pilée et extrait par 500 cm3 puis deux fois 250 cm3 d'acétate d'éthyle. Les phases organiques sont réunies, lavées successivement par 200 cm3 d'une solution aqueuse d'acide chlorhydrique 0,5N, par 100 cm3 d'une solution aqueuse d'acide chlorhydrique 0,1N, par deux fois 250 cm3 d'une solution aqueuse saturée d'hydrogénocarbonate de sodium et par 100 cm3 d'une solution aqueuse saturée de chlorure de sodium, séchées sur sulfate de magnésium, filtrées et concentrées à sec sous pression réduite (30 mm de Hg; 4 kPa) à 40°C. Le solide jaune résiduel est repris par 100 cm3 d'oxyde d'isopropyle, trituré, essoré, lavé par 10 cm3 d'oxyde d'isopropyle et séché sous pression réduite (5 mm de Hg; 0,7 kPa) à 40°C. On obtient ainsi 17,8 g de (hydroxy-1 propyl-2)-10 phénothiazinecarbonitrile-2, série L, sous forme de cristaux jaunes fondant à 136°C.

$[\alpha]_D^{20}$ = -13 ± 0,4° (1,2%; chloroforme).

Le phtalate de (+)-(cyano-2 phénothiazinyl-10)-2 propyle et de phényl-1 éthylammonium-(1R) peut être préparé de la manière suivante :

On porte à reflux pendant 6 heures, sous agitation, une suspension de 56,5 g d'[(hydroxy-1 propyl-2-(2RS)]-10 phénothiazinecarbonitrile-2 et de 32,6 g d'anhydride phtalique dans 100 cm3 de pyridine anhydre. Après refroidissement, le mélange réactionnel est dilué par 500 cm3 de chlorure de méthylène, lavé par 4 fois 100 cm3 d'eau distillée, séché sur sulfate de magnésium, filtré et concentré à sec sous pression réduite (30 mm de Hg; 4 kPa) à 40°C. Le résidu est agité avec 500 cm3 d'une solution aqueuse N d'acide chlorhydrique puis décanté et dissous dans 500 cm3 d'acétate d'éthyle. La solution est lavée par 2 fois 100 cm3 d'une solution aqueuse N d'acide chlorhydrique puis par 100 cm3 d'une solution aqueuse de chlorure de sodium. La phase organique est séchée sur sulfate de magnésium, filtrée et concentrée à sec sous pression réduite (30 mm de Hg; 4 kPa) à 50°C. On obtient ainsi 102 g d'une huile épaisse contenant de l'acide [cyano-2 phénothiazinyl-10)-2 propyl-1 -(2RS)] oxycarbonyl-2 benzènecarboxylique et utilisés tels quels ultérieurement.

On dissout 102 g de l'huile précédemment obtenue et contenant l'acide [(cyano-2 phénothiazinyl-10)-2 propyl-1 -(2RS)]oxycarbonyl-2 benzènecarboxylique dans 500 cm3 d'acétate d'éthyle et on ajoute, sous agitation, à une température voisine de 20°C, une solution de 24,2 g de (-)-phényl-1 éthylamine-(1S) dans 360 cm3 d'acétate d'éthyle. Après 2 jours d'agitation à une température voisine de 20°C, le solide formé est filtré et conservé.

Le filtrat est concentré à sec sous pression réduite (30 mm de Hg; 4 kPa) à 40°C. Le résidu est repris par 500 cm3 d'une solution aqueuse N d'acide chlorhydrique et extrait par 2 fois 250 cm3 d'acétate d'éthyle. Les phases organiques réunies sont concentrées à sec sous pression réduite (30 mm de Hg; 4 kPa) à 40°C. Le résidu (50 g) est dissous dans 500 cm3 d'acétate d'éthyle et on ajoute 14 g de (+)-phényl-1 éthylamine-(1R). Après 16 heures d'agitation à une température voisine de 20°C, le solide formé est essoré et dissous dans 450 cm3 d'acétate d'éthyle au reflux. Après refroidissement, le solide formé est essoré, lavé par 40 cm3 d'acétate d'éthyle et séché sous pression réduite (50 mm de Hg; 4 kPa) à 40°C. On obtient ainsi 44,3 g de phtalate de (+)-(cyano-2 phénothiazinyl-10)-2 propyle et de phényl-1 éthylammonium-(1R) sous forme de cristaux jaunes clairs fondant à 154-155°C.

$[\alpha]_D^{20}$ = +20,8 ± 0,5° (1,1%; chloroforme).

EXEMPLE 6

A une solution de 2,5 g de [(perhydroazépinyl-1)-1 propyl-2-(2RS)]-10 N-propyl phénothiazinecarbothioamide-2 dans 25 cm3 d'acide acétique, on ajoute en 20 minutes 1,81 g d'acétate mercurique en solution dans 35 cm3 d'acide acétique glacial et on agite pendant 45 minutes à une température voisine de 20°C. La suspension noire obtenue est filtrée sur verre fritté colmaté par de la célite et le filtrat jaune est concentré sous pression réduite (30 mm de Hg; 4 kPa) à 40°C. Le résidu est repris par 250 cm3 d'acétate d'éthyle et 50 cm3 d'eau distillée. La phase organique est lavée successivement par 2 fois 100 cm3 de soude normale, 3 fois 100 cm3 d'eau distillée et par 100 cm3 d'une solution aqueuse saturée de chlorure de sodium et séchée sur sulfate de magnésium. Après filtration, le filtrat jaune est concentré sous pression réduite (30 mm de Hg; 4 kPa) à 40°C et on obtient ainsi 2,1 g d'huile jaune brute. Ce résidu est repris par 80 cm3 d'oxyde d'isopropyle bouillant. Le léger insoluble est éliminé par filtration et la cristallisation est amorcée dans le filtrat refroidissant maintenu sous agitation. Les cristaux sont filtrés sur verre fritté pour donner 1,29 g de [(perhydroazépinyl-1)-1 propyl-2-(2RS)]-10 N-propyl phénothiazinecarboxamide-2 sous forme d'un solide blanc cassé fondant à 113°C.

RMN du proton (250 MHz, DMSO, δ en ppm, et J en Hz).

0,9 (T, J = 7,5, 3H, $-CH_3$ du propyle); 1,48 (Mf, 8H, $-CH_2-$ du perhydroazépinyl); 1,52 (Mt, 2H, $-CH_2C\underline{H}_2CH_3$); 1,6 (D, J = 7, 3H, $-CH_3$); 2,56 (Mt, environ 4H,

$$-C\underline{H}_2-N-C\underline{H}_2-);$$

2,78 (DD, J = 14 et 6, 1H, 1H du >N-$CH_2$-); 3,06 (DD, J = 14 et 6, 1H, 1H du>N-$CH_2$-); 3,06 (DD, J = 14 et 7,5, 1H, 1H du >N-$CH_2$-); 3,22 (Mt, 2H, -CONH-$CH_2$-); 4,12 (Mt, J = 7,5 -7 et 6, 1H, N-CH ); 6,9 à 7,25 (Mt, 5H, aromatiques); 7,4 (D large, J = 8, 1H, -H en 3); 7,52 (S large, 1H, -H en 1); 8,44 (T, J = 5,5, 1H, -CONH-).

Spectre infra-rouge (KBr), bandes caractéristiques en $cm^{-1}$ ;

3450, 3370, 3060, 2960, 2930, 2880, 2860, 2820, 1650, 1595, 1555, 1520, 1460, 1410, 1380, 1310, 1230, 820.

Le [(perhydroazépinyl-1)-1 propyl-2-(2RS)]-10 N-propyl phénothiazinecarbothioamide-2 peut être obtenu de la manière suivante :

A une solution de 3,2 g de [(perhydroazépinyl-1)-1 propyl-2-(2RS)]-10 phénothiazinecarbothioamide-2 dans 80 cm3 d'éthanol absolu, on ajoute 3,3 cm3 de n-propylamine. Le mélange est saturé d'acide sulfhydrique puis porté à 150°C pendant 16 heures. Le mélange réactionnel est concentré à sec sous pression réduite (30 mm de Hg; 4 kPa). Le résidu est dilué par 150 cm3 d'acétate d'éthyle, lavé par 3 fois 100 cm3 d'eau distillée et par 100 cm3 de solution saturée de chlorure de sodium, séché sur sulfate de magnésium puis concentré à sec sous pression réduite (30 mm de Hg; 4 kPa) avant d'être purifié par chromatographie sur une colonne (hauteur : 32 cm; diamètre : 4 cm) de gel de silice (0,06-0,2 mm) en éluant avec un mélange de cyclohexane et d'acétate d'éthyle 60-40 en volumes (1000 cm3) en recueillant des fractions de 60 cm3. Les fractions 3 à 11 sont réunies et concentrées à sec à 50°C sous pression réduite (30 mm de Hg; 4 kPa) pour donner 2,5 g de [(perhydroazépinyl-1)-1 propyl-2-(2RS)]-10 N-propyl phénothiazinethiocarboxamide-2 sous la forme d'une huile orangée limpide.

Le [(perhydroazépinyl-1)-1 propyl-2-(2RS)]-10 phénothiazinecarbothioamide-2 peut être obtenu de la manière suivante :

Un mélange de 7,1 g de [(perhydroazépinyl-1)-1 propyl-2-(2RS)]-10 phénothiazinecarbonitrile-2 et de 2,74 cm3 de triéthylamine dans 75 cm3 de pyridine anhydre est saturé par bullage d'acide sulfhydrique pendant 5 heures à 25°C. La solution limpide obtenue est maintenue sous agitation pendant 12 heures à 25°C, et dégazée par bullage d'azote pendant 1 heure. Le mélange réactionnel est dilué par 150 cm3 d'acétate d'éthyle et lavé par 6 fois 100 cm3 d'eau distillée, et par 3 fois 120 cm3 de solution saturée de chlorure de sodium. La phase organique est séchée sur sulfate de magnésium, filtrée et le filtrat est concentré à sec sous pression réduite (30 mm de Hg; 4 kPa). Le résidu est purifié par chromatographie sur colonne de gel de silice (0,06-0,2 mm) (hauteur : 29 cm; diamètre : 4,5 cm) en éluant par des mélanges de cyclohexane et d'acétate d'éthyle 30-70 (1 litre) et 40-60 (2 litres) (en volumes) et en recueillant des fractions de 250 cm3. Les fractions 4 à 6 sont réunies et concentrées à sec à 50°C sous pression réduite (30 mm de Hg; 4 kPa) pour donner 5,2 g de [(perhydroazépinyl-1)-1 propyl-2-(2RS)]-10 phénothiazinecarbothioamide-2 sous la forme d'une meringue orangée.

Le [(perhydroazépinyl-1)-1 propyl-2-(2RS)]-10 phénothiazinecarbonitrile-2 peut être préparé de la manière suivante :

On porte une suspension de 9 g de mésylate de (cyano-2 phénothiazinyl-10)-2-(2RS) propyle-1 dans 25 cm3 d'hexaméthylèneimine à 89°C. Le mélange est maintenu à cette température pendant 12 heures. Après refroidissement, le mélange est concentré à sec à 50°C sous pression réduite (30 mm de Hg; 4 kPa). Le résidu est dilué par 350 cm3 d'acétate d'éthyle, filtré et le filtrat est lavé par 150 cm3 d'eau distillée et 2 fois 100 cm3 de solution saturée de chlorure de sodium. Après décantation, la phase organique est extraite par 350 cm3 d'acide chlorhydrique en solution 3N. Les extraits aqueux acides réunis sont extraits par 200 cm3 d'acétate d'éthyle puis alcalinisés avec de la soude concentrée et extraits à nouveau, par 3 fois 200 cm3 d'acétate d'éthyle. La phase organique est lavée par 2 fois 200 cm3 d'eau distillée puis séchée sur sulfate de magnésium, filtrée et concentrée à sec sous pression réduite (30 mm de Hg; 4 kPa) pour donner un résidu qui est purifié par chromatographie sur colonne (hauteur : 35 cm; diamètre : 4,2 cm) de gel de silice (0,06-0,2 mm) en éluant par un mélange de cyclohexane et d'acétate d'éthyle 70-30 en volumes (500 cm3) et en recueillant des fractions de 100 cm3. Les 200 premiers cm3 sont éliminés et les fractions 1 à 3 sont réunies et concentrées à sec à 50°C sous pression réduite (30 mm de Hg; 4 kPa) pour donner 7,2 g de [(perhydroazépinyl-1)-1 propyl-2-(2RS)]-10 phénothiazinecarbonitrile-2 sous la forme d'une meringue jaune.

## EXEMPLE 7

A une solution de 0,76 g de N-butyl [diéthylamino-1 propyl-2-(2RS)]-10 phénothiazinecarbothioamide-2 dans 9 cm3 d'acide acétique glacial, on ajoute goutte à goutte une solution de 0,57 g d'acétate mercurique dans 9 cm3 d'acide acétique glacial pendant une période de 10 minutes. Le mélange réactionnel est agité 90 minutes à 25°C puis filtré sur verre fritté garni de célite. La célite est lavée par 2 fois 3 cm3 d'acide acétique et les filtrats réunis sont concentrés à sec sous pression réduite (30 mm de Hg; 4 kPa) à 40°C pour donner un résidu qui est dilué dans 50 cm3 d'acétate d'éthyle. La phase organique est lavée par 25 cm3 de soude normale, 3 fois 25 cm3 d'eau distillée puis par une fois 25 cm3 de saumure, séchée sur sulfate de magnésium,

filtrée et concentrée à sec sous pression réduite (30 mm de Hg; 4 kPa) à 40°C pour donner un résidu qui est purifié par chromatographie sur une colonne (hauteur : 12 cm; diamètre : 1,8 cm) de gel de silice (0,04-0,06 mm) avec une légère surpression d'azote (40 kPa) en éluant avec un mélange de cyclohexane et d'acétate d'éthyle 50-50 (750 cm3) (en volumes) en recueillant des fractions de 25 cm3. Les fractions 5 à 25 sont réunies et concentrées sous pression réduite (30 mm de Hg; 4 kPa) à 50°C pour donner 0,68 g de N-butyl [diéthylamino-1 propyl-2-(2RS)]-10 phénothiazinecarboxamide-2.

A une solution de 0,46 g de N-butyl [diéthylamino-1 propyl-2-(2RS)]-10 phénothiazinecarboxamide-2 dans 5 cm3 de propanol-2 bouillant, on ajoute 0,13 g d'acide fumarique dissous dans 5 cm3 de propanol-2 au reflux. La cristallisation est amorcée par grattage et le mélange est agité pendant 5 heures à 25°C. Les cristaux sont filtrés sur verre fritté, lavés par 2 fois 3 cm3 de propanol-2 glacé et séchés sous pression réduite (5 mm de Hg; 0,7 kPa) pour donner 0,34 g de fumarate de N-butyl [diéthylamino-1 propyl-2 -(2RS)]-10 phénothiazinecarboxamide-2 fondant à 129°C.

RMN du proton (250 MHz, DMSO, δ en ppm, J en Hz).

0,92 (Mt, 9H, $-N(CH_2C\underline{H_3})_2$ et $-CH_2C\underline{H_3}$); 1,35 (Mt, 2H, $-C\underline{H_2}CH_3$); 1,52 (Mt, 2H, $-CH_2CH_2CH_3$); 1,65 (D, J = 7, 3H, $-CH_3$); 2,53 (Mt masqué, 4H, $-N(C\underline{H_2}CH_3)_2$); 2,75 (DD, J = 13 et 6, 1H, 1H du $>NCH_2-$); 3,07 (DD, J = 13 et 6,5; 1H, 1H du $>NCH_2-$); 3,26 (Mt, J = 7 et 5,5, 2H, $-CONHC\underline{H_2}(CH_2)_2CH_3$); 4,20 (Mt, J = 7, 6,5 et 6, 1H,$>N-CH<$); 6,63 (S, 2H, -CH=CH- du fumarate); 6,9 à 7,3 (Mt, 5H, aromatiques); 7,42 (DD, J = 8 et 1, 1H, -H en 3); 7,54 (D, J = 1, 1H, -H en 1); 8,43 (T, J = 5,5, 1H, -CONH-).

Spectre infra-rouge (KBr), bandes caractéristiques en $cm^{-1}$ :

3260, 3050, 2960, 2930, 2870, 2660, 2490, 1910, 1700, 1630, 1590, 1555, 1460, 1415, 1380, 1305, 1230, 985, 880, 840, 755, 635.

Le N-butyl [diéthylamino-1 propyl-2-(2RS)]-10 phénothiazinecarbothioamide-2 peut être préparé de la manière suivante :

A une solution de 1,86 g de [diéthylamino-1 propyl-2-(2RS)]-10 phénothiazinecarbothioamide-2 dans 25 cm3 d'éthanol absolu, on ajoute 2,4 cm3 de n-butylamine. Le mélange est porté à 150°C pendant 16 heures puis concentré à sec sous pression réduite (30 mm de Hg; 4 kPa) à 40°C. Le résidu obtenu est purifié par chromatographie sur une colonne (hauteur 17 cm; diamètre : 2,6 cm) de gel de silice (0,04-0,06 mm) avec une légère surpression d'azote (40 kPa) en éluant avec des mélanges de cyclohexane et d'acétate d'éthyle 90-10 (250 cm3) 80-20 (500 cm3) 50-50 (750cm3) (en volumes) puis par de l'acétate d'éthyle pur (750 cm3) en recueillant des fractions de 50 cm3. Les fractions 10 à 18 sont réunies et concentrées à sec sous pression réduite (30 mm de Hg; kPa) à 40°C pour donner 0,96 g de N-butyl [diéthylamino-1 propyl-2-(2RS)]-10 phénothiazinecarbothioamide-2.

Spectre de RMN du proton (250 MHz, DMSO, δ en ppm, J en Hz) :

1,37 (Mt, 2H, $-(CH_2)_2C\underline{H_2}-CH_3$); 1,69 (Mt, 2H, $-C\underline{H_2}-CH_2-CH_3$); 3,70 (Mt, 2H, $-CSNH-C\underline{H_2}-$); 7 à 7,35 (Mt, 5H, aromatiques).

EXEMPLE 8

On dissout 0,6 g de N-butyl [N'-éthyl N'-méthyl-amino-1 propyl-2-(2RS)]-10 phénothiazinecarbothioamide-2 dans une solution de 0,17 g d'acide fumarique dans 10 cm3 d'éthanol puis on concentre à sec sous pression réduite (30 mm de Hg; 4 kPa) à 40°C. On reprend la meringue orangée résiduelle (0,77 g) par 10 cm3 d'acide acétique, on ajoute 0,48 g d'acétate mercurique et on agite la suspension orangée obtenue pendant 2 heures à une température voisine de 20°C. La suspension noire obtenue est concentrée à sec sous pression réduite (30 mm de Hg; 4 kPa) à 60°C et le résidu est repris par 15 cm3 d'eau distillée. On filtre et lave le solide avec 5 cm3 d'eau distillée. Le filtrat et l'eau de lavage sont réunis et alcalinisés par de la lessive de soude (d = 1,33) jusqu'à pH 13. On extrait par 2 fois 10 cm3 d'acétate d'éthyle et les phases organiques réunies sont lavées par 10 cm3 d'une solution aqueuse saturée de chlorure de sodium, séchées sur sulfate de magnésium, filtrées et concentrées à sec sous pression réduite (30 mm de Hg; 4 kPa) à 40°C. L'huile orangée résiduelle (0,5 g) est dissoute dans le minimum d'acétate d'éthyle (3,6 cm3) et on ajoute 28,5 cm3 d'oxyde d'isopropyle, puis, sous agitation, 4 cm3 d'une solution 0,3N d'acide chlorhydrique dans l'oxyde d'isopropyle. On poursuit l'agitation pendant 30 minutes à une température voisine de 5°C. Le précipité formé est essoré, lavé par 3 fois 4 cm3 d'oxyde d'isopropyle et séché sous pression réduite (5 mm de Hg; 0,7 kPa) à 40°C. On obtient ainsi 0,4 g de chlorhydrate de N-butyl [N'-éthyl N'-méthyl-amino-1 propyl-2-(2RS)]-10 phénothiazinecarboxamide-2 sous forme d'un solide beige fondant à 130-135°C (fusion pâteuse).

RMN du proton (250 MHz, en DMSO, δ en ppm, J en Hz) :

0,9 (T, J = 7, J = 7, 3H, $-CH_2C\underline{H_3}$); 1,32 (Mt, 2H, $-C\underline{H_2}-CH_3$); 1,51 (Mt, 2H, $-C\underline{H_2}CH_2CH_3$); 1,8 (D, J = 7, 3H, $-CH_3$); 2,73 (S, 3H, $>N-CH_3$); 3,25 (Mt, 2H, $-CONH-C\underline{H_2}-$); 4,8 (Mt, 1H, N-CH); 7 à 7,4 (Mt, 5H, aromatiques); 7,51 (S, 1H, -H en 1); 7,53 (D, J = 8, 1H, -H en 3); 8,62 (Mf, 1H, -CONH-); 10,45 (Mf, 1H, $NH^+$).

Spectre infra-rouge (KBr), bandes caractéristiques en $cm^{-1}$ :

3280, 3060, 2960, 2930, 2870, 2660, 2600, 2580, 1640, 1590, 1540, 1460, 1415, 1380, 1310, 1230, 870, 845, 830, 755.

Le N-butyl [N'-éthyl N'-méthyl-amino-1 propyl-2-(2RS)]-10 phénothiazinecarbothioamide-2 peut être préparé de la manière suivante :

Un mélange de 2,1 g de [N-éthyl N-méthyl-amino-1 propyl-2-(2RS)]-10 phénothiazinecarbothioamide-2 et de 8,5 cm3 de butylamine dans 30 cm3 d'éthanol absolu est saturé d'acide sulfhydrique et chauffé pendant 16 heures à une température voisine de 100°C. Après refroidissement, la solution orangée obtenue est concentrée à sec sous pression réduite (30 mm de Hg; 4 kPa) à 40°C. L'huile brune résiduelle est purifiée par chromatographie sur une colonne (hauteur : 4 cm; diamètre : 3 cm) de gel de silice (0,2-0,063 mm) en éluant successivement par 2 litres de chlorure de méthylène et par 1 litre d'un mélange de chlorure de méthylène et de méthanol (90-10 en volumes) et en recueillant des fractions de 60 cm3. Les fractions 36 à 39 sont réunies et concentrées à sec sous pression réduite (30 mm de Hg; 4 kPa) à 40°C. L'huile résiduelle orangée (1,8 g) est à nouveau purifiée par chromatographie sur une colonne (hauteur : 25 cm; diamètre : 4 cm) de gel de silice (0,04-0,063 cm) sous une légère surpression d'azote (40 kPa) en éluant par 1 litre d'un mélange d'acétate d'éthyle et de cyclohexane (75-25 en volumes) et en recueillant des fractions de 60 cm3. Les fractions 5 à 12 sont réunies et concentrées à sec sous pression réduite (30 mm de Hg; 4 kPa) à 40°C. On obtient ainsi 1,25 g de N-butyl [N'-éthyl N'-méthyl-amino-1 propyl-2-(2RS)]-10 phénothiazinecarbothioamide-2 sous forme d'une huile jaune.

Le [N-éthyl N-méthyl-amino-1 propyl-2-(2RS)]-10 phénothiazinecarbothioamide-2 peut être préparé de la manière suivante :

Une solution de 3 g de [(N-éthyl N-méthyl-amino-1 propyl-2-(2RS)]-10 phénothiazinecarbonitrile-2 et de 1,3 cm3 de triéthylamine dans 60 cm3 de pyridine anhydre est additionnée d'acide sulfhydrique, en excès, puis on poursuit l'agitation pendant 16 heures à une température voisine de 20°C. La solution obtenue est purgée avec un courant d'azote pendant 1 heure, versée dans 100 cm3 d'acétate d'éthyle, lavée par 3 fois 200 cm3 d'eau distillée, séchée sur sulfate de magnésium, filtrée et concentrée à sec sous pression réduite (30 mm de Hg; 4 kPa) à 40°C. L'huile brune résiduelle est purifiée par chromatographie sur une colonne (hauteur : 40 cm; diamètre : 3 cm) de gel de silice (0,2-0,06 mm) en éluant successivement par 1 litre de chlorure de méthylène, 1 litre d'un mélange de chlorure de méthylène et de méthanol (95-5 en volumes) et 3 litres d'un mélange de chlorure de méthylène et de méthanol (80-20 en volumes) et en recueillant des fractions de 150 cm3. Les fractions 26 à 30 sont réunies et concentrées à sec sous pression réduite (30 mm de Hg; 4 kPa) à 40°C. On obtient ainsi 2,15 g de [N-éthyl N-méthyl-amino-1 propyl-2-(2RS)]-10 phénothiazinecarbothioamide-2 sous forme d'une huile orangée.

Le [N-éthyl N-méthyl-amino-1 propyl-2-(2RS)]-10 phénothiazinecarbonitrile-2 peut être préparé de la manière suivante :

Un mélange de 4,4 g d'[éthylamino-1 propyl-2-(2RS)]-10 phénothiazinecarbonitrile-2, de 2,25 g de carbonate de sodium et de 0,9 cm3 d'iodométhane dans 60 cm3 de diméthylformamide est chauffé pendant 6 heures à une température voisine de 150°C. Le mélange réactionnel est ensuite concentré à sec sous pression réduite (5 mm de Hg; 0,7 kPa) à 40°C et le résidu est repris par 100 cm3 d'eau distillée et extrait par 2 fois 100 cm3 d'acétate d'éthyle. Les phases organiques réunies sont extraites par 2 fois 50 cm3 d'une solution aqueuse d'acide chlorhydrique N. Les phases aqueuses réunies sont alcalinisées avec de la soude (d = 1,33) jusqu'à pH 13 et extraites par 2 fois 100 cm3 d'acétate d'éthyle. Les phases organiques réunies sont lavées successivement avec 50 cm3 d'eau distillée et avec 50 cm3 d'une solution aqueuse saturée de chlorure de sodium, séchées sur sulfate de magnésium, filtrées et concentrées à sec sous pression réduite (30 mm de Hg; 4 kPa) à 40°C. On obtient ainsi 3 g de [N-éthyl N-méthyl-amino-1 propyl-2-(2RS)]-10 phénothiazinecarbonitrile-2 sous forme d'une huile orangée.

L'[éthylamino-1 propyl-2-(2RS)]-10 phénothiazinecarbonitrile-2 peut être préparé de la manière suivante :

Une solution de 50 g de méthanesulfonate de (cyano-2 phénothiazinyl-10)-2 propyle-(2RS) et de 100 cm3 d'éthylamine dans 600 cm3 de toluène est chauffée pendant 24 heures à une température voisine de 105°C. Après refroidissement, on concentre à sec sous pression réduite (30 mm de Hg; 4 KPa) à 40°C. Le résidu est repris par 250 cm3 d'eau distillée et on extrait successivement par 500 cm3 et 250 cm3 d'acétate d'éthyle. Les phases organiques réunies sont extraites par 2 fois 500 cm3 d'une solution aqueuse d'acide chlorhydrique N. Les phases aqueuses sont alcalinisées avec de la lessive de soude (d = 1,33) jusqu'à pH 13 et extraites successivement par 500 cm3 et par 250 cm3 d'acétate d'éthyle. Les phases organiques réunies sont lavées par 250 cm3 d'une solution aqueuse saturée de chlorure de sodium, séchées sur sulfate de magnésium, filtrées et concentrées à sec sous pression réduite (30 mm de Hg; 4 kPa) à 40°C. On obtient ainsi 30,4 g d'[éthylamino-1 propyl-2-(2RS)]-10 phénothiazinecarbonitrile-2 sous forme d'une huile orangée.

EXEMPLE 9

Une suspension de 1,2 g de N-butyl [(pyrrolidinyl-1)-1 propyl-2-(2RS)]-10 phénothiazinecarbothioamide-2 et de 1 g d'acétate mercurique dans 15 cm3 d'acide acétique est agitée pendant 5 heures à une température voisine de 20°C. La suspension noire obtenue est diluée par 25 cm3 d'éther éthylique et filtrée. Le solide est lavé par 2 fois 10 cm3 d'éther éthylique. Les phases organiques réunies sont concentrées à sec sous pression réduite (30 mm de Hg; 4 kPa) à 50°C. Le résidu est repris par 25 cm3 d'acétate d'éthyle et 25 cm3 d'eau distillée. La phase organique est séparée, filtrée, séchée sur sulfate de magnésium, filtrée à nouveau et concentrée à sec sous pression réduite (30 mm de Hg; 4 kPa) à 40°C. L'huile résiduelle très visqueuse (1,1 g) est reprise par 12 cm3 d'un mélange d'acétate d'éthyle et de méthanol (83-17 en volumes) tiédi vers 50°C. Après 1 heure à une température voisine de 20°C, on essore le solide formé, le lave par 2 fois 10 cm3 d'oxyde de diéthyle et le sèche sous pression réduite (5 mm de Hg; 0,7 kPa) à 40°C. On obtient ainsi 0,4 g de N-butyl [(pyrrolidinyl-1)-1 propyl-2 -(2RS)]-10 phénothiazinecarboxamide-2 sous forme de cristaux crèmes fondant à 218°C.

RMN du proton (250 MHz, $CDCl_3$, 27°C, $\delta$ en ppm, J en Hz).

0,97 (T, J = 7,5, 3H, $-CH_3$ du N-butyl); 1,43 (Mt, 2H, $-C\underline{H_2}-CH_3$); 1,64 (Mt, 2H, $-C\underline{H_2}CH_2-CH_3$); 1,87 (D, J = 7, 3H, $-CH_3$); 2,07 (Mf, 4H, $-CH_2-CH_2-$ de la pyrrolidine); 2,6 à 3,35 et 3,5 à 4,1 (2Mf étalé, de 2H chacun,

$$-CH_2-\underset{|}{N}-CH_2-$$

de la pyrrolidine); 3,45 (Mt, 3H, $-CONH-C\underline{H_2}-$ et 1H du $>N-CH_2-$); 3,72 (DD, J = 14 et 8, 1H, 1H du $>NCH_2-$); 5,24 (Mt, 1H, $>N-CH<$); 6,9 à 7,3 (Mt, 6H, aromatiques et -CONH-); 7,39 (DD, J = 8 et 1, 1H, -H en 3); 7,52 (D, J = 1, 1H, -H en 1); 12,3 (Mf étalé, 1H, - $NH^+$).

Spectre infra-rouge (KBr), bandes caractéristiques en $cm^{-1}$ :

3280, 3060, 2960, 2930, 2870, 2680, 2610, 2480, 1645, 1595, 1535, 1460, 1410, 1380, 1305, 1235, 845, 750.

Le N-butyl [(pyrrolidinyl-1)-1 propyl-2-(2RS)]-10 phénothiazinecarbothioamide-2 peut être préparé de la manière suivante :

Un mélange de 1 g de [(pyrrolidinyl-1)-1 propyl-2-(2RS)]-10 phénothiazinecarbothioamide-2 et de 1,5 cm3 de butylamine dans 20 cm3 d'éthanol absolu est saturé avec de l'acide sulfhydrique puis chauffé pendant 24 heures à une température voisine de 120°C. Après refroidissement, le mélange réactionnel est concentré à sec sous pression réduite (30 mm de Hg; 4 kPa) à 40°C. Le résidu est repris par 10 cm3 d'eau distillée et 25 cm3 d'acétate d'éthyle. Après extraction, la phase organique est séparée, lavée par 10 cm3 d'eau distillée puis par 20 cm3 d'une solution aqueuse saturée de chlorure de sodium, séchée sur carbonate de potassium, filtrée et concentrée à sec sous pression réduite (30 mm de Hg; 4 kPa) à 40°C. On obtient ainsi 1,15 g de N-butyl [(pyrrolidinyl-1)-1 propyl-2-(2RS)]-10 phénothiazinecarbothioamide-2 sous forme d'une huile jaune visqueuse utilisée telle quelle ultérieurement.

EXEMPLE 10

A une solution de 2 g de chlorhydrate de N-butyl [(pyrrolidinyl-1)-1 propyl-2]-10 phénothiazinecarbothioamide-2, série L, dans 40 cm3 d'acide acétique, on ajoute 1,45 g d'acétate mercurique et on agite pendant 16 heures à une température voisine de 20°C. La suspension orangée obtenue est diluée par 50 cm3 d'eau distillée, filtrée et le filtrat jaune est concentré à sec sous pression réduite (30 mm de Hg; 4 kPa) à 40°C. Le résidu est repris par 100 cm3 d'acétate d'éthyle et 50 cm3 d'eau distillée puis additionné de soude (d = 1,33) jusqu'à pH 13. La phase organique est lavée successivement par 2 fois 25 cm3 d'eau distillée et par 25 cm3 d'une solution aqueuse saturée de chlorure de sodium et séchée sur sulfate de magnésium. Après filtration, le filtrat jaune est concentré à sec sous pression réduite (30 mm de Hg; 4 kPa) à 40°C et on obtient ainsi 1,12 g d'une huile jaune. Ce produit est purifié par chromatographie sur une colonne (hauteur : 30 cm; diamètre : 1 cm) de gel de silice (0,2-0,063 mm) en éluant avec un mélange de chlorure de méthylène et de méthanol (95-5 en volumes) et en recueillant des fractions de 20 cm3. Les fractions 7 à 19 sont réunies et concentrées à sec sous pression réduite (30 mm de Hg; 4 kPa) à 40°C. On obtient 0,63 g d'une gomme jaune. Ce produit est dissous dans un mélange de 2,5 cm3 d'acétate d'éthyle et 40 cm3 d'oxyde d'isopropyle et additionné de 0,45 cm3 d'une solution d'acide chlorhydrique 3,3N dans l'oxyde d'isopropyle. Le précipité formé est essoré, lavé par 3 fois 2 cm3 d'oxyde d'isopropyle et séché à 40°C sous pression réduite(5 mm de Hg; 0,7 kPa). On obtient ainsi 0,35 g de chlorhydrate de N-butyl [(pyrrolidinyl-1)-1 propyl-2]-10 phénothiazinecarboxamide-2, série L, sous forme d'un solide blanc fondant à 200°C.

$[\alpha]_D^{20}$ = +14,7 ±1° (0,5%; diméthylformamide).

RMN du proton (250 MHz, DMSO, δ en ppm, J en Hz).

0,92 (T, J = 7, 3H, $-CH_3$ du N-butyle); 1,35 (Mt, 2H, $-CH_2-CH_3$); 1,53 (Mt,2H, $-CH_2CH_2CH_3$); 1,8 (D, J = 6,5; 3H, $-CH_3$); 1,7 à 2 (Mt, 4H, $-CH_2-CH_2-$); 2,8 - 3,1 - 3,6 et 3,75 (4Mt, 1H chacun,

$-CH_2-N-CH_2-$);

3,28 (Mt, 2H, $-CONH-CH_2-$); 3,6 à 3,9 (Mt, 2H, >N-$CH_2$-); 4,68 (Mt, 1H, >N-CH<); 7 à 7,4 (Mt, 5H, aromatiques); 7,52 (S, 1H, -H en 1); 7,54 (D, J = 8, 1H,-H en 3); 8,58 (T, J = 5,5, 1H, -CONH-); 10,2 (Mf, 1H, $-NH^+$).

Spectre infra-rouge (KBr), bandes caractéristiques en $cm^{-1}$ :

3270, 3060, 2950, 2930, 2865, 2670, 2580, 2470, 1645, 1590, 1535, 1460, 1410, 1380, 1305, 1230, 845, 755.

Le chlorhydrate de N-butyl [(pyrrolidinyl-1)-1 propyl-2]-10 phénothiazinecarbothioamide-2, série L, peut être préparé de la manière suivante :

A une solution de 3,7 g de [(pyrrolidinyl-1)-1 propyl-2]-10 phénothiazinecarbothioamide-2, série L, dans 55 cm3 d'éthanol absolu, on ajoute 9,6 cm3 de butylamine et on sature cette solution avec de l'acide sulfhydrique. Le mélange est ensuite porté à une température voisine de 105°C pendant 16 heures. Après refroidissement, la solution est concentrée à sec sous pression réduite (30 mm de Hg; 4 kPa) à 40°C. On obtient une huile orangée qui est purifiée par chromatographie sous une légère surpression d'azote (40 kPa) sur une colonne (hauteur : 25 cm; diamètre : 4 cm) de gel de silice (0,2-0,063 mm) en éluant par un litre d'un mélange d'acétate d'éthyle et de cyclohexane (70-30 en volumes) et en recueillant des fractions de 60 cm3. Les fractions 7 à 12 sont réunies et concentrées à sec sous pression réduite (30 mm de Hg; 4 kPa) à 40°C. On obtient ainsi 3,4 g d'une huile orangée. Ce produit est dissous dans 150 cm3 d'oxyde d'isopropyle et on ajoute 2,4 cm3 d'une solution d'acide chlorhydrique 3,3N dans l'oxyde d'isopropyle. Le précipité formé est essoré, lavé par 3 fois 10 cm3 d'oxyde d'isopropyle et séché à 40°C sous pression réduite (5 mm de Hg; 0,7 kPa). On obtient 3,16 g de chlorhydrate de N-butyl [(pyrrolidinyl-1)-1 propyl-2]-10 phénothiazinecarbothioamide-2, série L, sous forme d'un solide jaune fondant à 125-130°C (fusion pâteuse).

$[\alpha]_D^{20} = +27,5 \pm 0,6°$ (1%; diméthylformamide).

RMN du proton (250 MHz, $CDCl_3$, δ en ppm, J en Hz).

1 (T, J = 7,5, 3H, $-CH_3$ du butyle); 1,49 (Mt, 2H, $-CH_2CH_3$); 1,85 (D, J = 7, 3H, $-CH_3$); 1,86 (Mt, 2H, $-CH_2-CH_2CH_3$); 1,9 à 2,25 (Mt, 4H, $-CH_2-CH_2-$ de la pyrrolidine); 2,82 - 2,98 - 3,85 et 4,08 (4Mf, de 1H chacun,

$-CH_2-N-CH_2-$

de la pyrrolidine); 3,45 (D large, J = 13, 1H, 1H du >N-$CH_2$-); 3,65 à 3,95 (Mt, 3H, autre H du >N-$CH_2$- et $-CSNH-CH_2-$); 5,6 (Mt, 1H, >CH-N<); 6,9 à 7,25 (Mt, 5H, aromatiques); 7,33 (D, J = 1, 1H, -H en 1); 7,5 (DD, J = 8 et 1, 1H, -H en 3); 8,93 (Mf, 1H, -CSNH-); 12,25 (Mf, 1H, $-NH^+$).

EXEMPLE 11

A une solution de 1,12 g de [diéthylamino-1 propyl-2-(2RS)]-10 N-(méthyl-2 propyl) phénothiazinecarbothioamide-2 dans 13 cm3 d'acide acétique glacial, on ajoute goutte à goutte une solution de 0,84 g d'acétate mercurique dans 13 cm3 d'acide acétique glacial pendant une période de 10 minutes. Le mélange réactionnel est agité 90 minutes à 25°C puis filtré sur verre fritté garni de célite. La célite est lavée par 2 cm3 d'acide acétique et les filtrats réunis sont concentrés à sec sous pression réduite (30 mm de Hg; 4 kPa) à 40°C pour donner un résidu qui est alors dilué dans 50 cm3 d'acétate d'éthyle La phase organique est lavée par 25 cm3 de lessive de soude normale, 3 fois 25 cm3 d'eau distillée puis par une fois 25 cm3 de saumure, séchée sur sulfate de magnésium, filtrée et concentrée à sec sous pression réduite (30 mm de Hg; 4 kPa) à 40°C pour donner un résidu qui est purifié par chromatographie sur une colonne (hauteur : 17,2 cm; diamètre : 2,4 cm) de gel de silice (0,04-0,06 mm) avec une légère surpression d'azote (40 kPa) en éluant avec un mélange de cyclohexane et d'acétate d'éthyle 50-50 (1 litre) (en volumes) en recueillant des fractions de 60 cm3. Les fractions 4 à 14 sont réunies et concentrées à sec sous pression réduite (30 mm de Hg; 4 kPa) à 40°C pour donner 0,83 g de [diéthylamino-1 propyl-2-(2RS)]-10 N-(méthyl-2 propyl) phénothiazinecarboxamide-2.

A une solution de 0,62 g de [diéthylamino-1 propyl-2-(2RS)]-10 N-(méthyl-2 propyl) phénothiazinecarboxamide-2 dans 7,5 cm3 de propanol-2 bouillant, on ajoute 0,18 g d'acide fumarique dissous dans 7,5 cm3 de propanol-2 au reflux. Le mélange est concentré à sec sous pression réduite (30 mm de Hg; 4 kPa) à 40°C et le résidu est agité dans 50 cm3 d'éther d'isopropyle. Le solide est séparé par filtration sur verre fritté, lavé par 10 cm3 d'éther d'isopropyle et séché sous pression réduite (5 mm de Hg; 0,7 kPa) pour donner 0,64 g de

fumarate de [diéthylamino-1 propyl-2-(2RS)]-10 N-(méthyl-2 propyl) phénothiazinecarboxamide-2 fondant à 110°C.

RMN du proton (250 MHz, DMSO, δ en ppm, J en Hz).

0,9 (Mt, 12H, $-N(CH_2C\underline{H}_3)_2$ et $-CH(C\underline{H}_3)_2$); 1,65 (D, J = 7, 3H, $-CH_3$); 1,84 (Mt, 1H, $-C\underline{H}(CH_3)_2$; 2,58 (Mt, 4H, $-N(C\underline{H}_2CH_3)_2$); 2,8 (DD, J = 14 et 5,5,1H, 1H du $>NCH_2-$); 3,07 (Mt, 2H, $-CONH-C\underline{H}_2-$); 3,10 (DD, J = 14 et 7,5, 1H $>N-CH_2-$); 4,25 (Mt, J = 7,5 -7 et 5,5, 1H, N-CH ); 6,57 (S, 2H, -CH=CH- du fumarate); 6,9 à 7,3 (Mt, 5H, aromatiques); 7,42 (D, J = 8, 1H, -H en 3); 7,52 (S, 1H, -H en 1); 8,46 (T, J = 5,5, 1H, -CONH-).

Spectre infra-rouge (KBr), bandes caractéristiques en $cm^{-1}$ :

3300, 3060, 2950, 2920, 2865, 2620, 2500, 1900, 1705, 1635, 1590, 1555, 1540, 1460, 1410, 1380, 1310, 1230, 980, 870, 825, 750, 635.

Le [diéthylamino-1 propyl-2-(2RS)-10 N-(méthyl-2 propyl) phénothiazinecarbothioamide-2 peut être préparé de la manière suivante :

A une solution de 1,86 g de [diéthylamino-1 propyl-2-(2RS)]-10 phénothiazinecarbothioamide-2 dans 25 cm3 d'éthanol absolu, on ajoute 2,50 cm3 d'isobutylamine. Le mélange est porté à 150°C pendant 16 heures puis concentré à sec sous pression réduite (30 mm de Hg; 4 kPa) à 50°C. Le résidu obtenu est purifié par chromatographie sur une colonne (hauteur : 16,5 cm; diamètre : 2,8 cm) de gel de silice (0,04-0,06 mm) avec une légère surpression d'azote (40 kPa) en éluant avec des mélanges de cyclohexane et d'acétate d'éthyle 80-20 (1,5 litre) 50-50 (1 litre) (en volumes) en recueillant des fractions de 50 cm3. Les fractions 4 à 15 sont réunies et concentrées à sec sous pression réduite (30 mm de Hg; 4 kPa) à 40°C pour donner 1,3 g de [diéthylamino-1 propyl-2-(2RS)]-10 N-(méthyl-2 propyl) phénothiazinecarbothioamide-2.

Spectre du RMN du proton (400 MHz, DMSO, δ en ppm, J en Hz).

0,95 (D, J = 7, 6H, $-CH(C\underline{H}_3)_2$); 2,17 (Mt, 1H, $-C\underline{H}(CH_3)_2$); 3,55 (Mt, 2H, $-CSNH-C\underline{H}_2-$); 7,05 à 7,35 (Mt, 5H, aromatiques).

## EXEMPLE 12

A une solution de 2 g de (diéthylamino-1 propyl-2)-10 N-(méthyl-2 propyl) phénothiazinecarbothioamide-2, série L, dans 20 cm3 de méthanol, on ajoute 0,54 g d'acide fumarique et on concentre à sec sous pression réduite (30 mm de Hg; 4 kPa) à 40°C. Le résidu meringué orangé est dissous dans 40 cm3 d'acide acétique, additionné de 1,6 g d'acétate mercurique et agité pendant 5 heures à une température voisine de 20°C. Le mélange réactionnel est alors dilué avec 50 cm3 d'eau distillée, filtré et le filtrat jaune est concentré à sec sous pression réduite (30 mm de Hg; 4 kPa) à 40°C. L'huile jaune résiduelle est reprise par 50 cm3 d'acétate d'éthyle et la solution obtenue est lavée successivement par 50 cm3 de soude N, 25 cm3 d'eau distillée et 25 cm3 d'une solution aqueuse saturée de chlorure de sodium, séchée sur sulfate de magnésium en présence de noir 3S, filtrée et concentrée à sec sous pression réduite (30 mm de Hg; 4 kPa) à 40°C. L'huile jaune résiduelle (1,8 g) est purifiée par chromatographie sur colonne (hauteur : 25 cm; diamètre : 4 cm) de gel de silice (0,063-0,04 mm) sous légère surpression d'azote (300 mm de Hg; 40 kPa) en éluant successivement avec 1 litre de chlorure de méthylène, 2 litres d'un mélange de chlorure de méthylène et de méthanol (98-2 en volumes) et 1 litre d'un mélange de chlorure de méthylène et de méthanol (95-5 en volumes) et en recueillant des fractions de 60 cm3. Les fractions 14 à 60 sont réunies et concentrées à sec sous pression réduite (30 mm de Hg, 4 kPa) à 40°C pour donner 1,6 g d'une huile orangée. Ce produit est dissous dans 110 cm3 d'un mélange d'oxyde d'isopropyle et d'acétate d'éthyle (91-9 en volumes) et on ajoute, goutte à goutte, sous agitation et à une température voisine de 5°C, 6,2 cm3 d'une solution 0,64N d'acide chlorhydrique dans l'oxyde d'isopropyle. Le précipité formé est essoré, lavé par 20 cm3 d'oxyde d'isopropyle et séché sous pression réduite (5 mm de Hg; 0,7 kPa) à 40°C. On obtient ainsi 1,3 g de chlorhydrate de (diéthylamino-1 propyl-2)-10 N-(méthyl-2 propyl) phénothiazinecarbothioamide-2, série L, sous forme d'un solide blanc fondant à 115-120°C (fusion pâteuse).

RMN du proton (250 MHz, DMSO, δ en ppm et J en Hz).

En solution dans le DMSO, on observe deux formes dues à la salification de l'azote.

0,9 (D, J = 7, 6H, $-CH(C\underline{H}_3)_2$); 0,98 et 1,18 (2T, J = 7, 6H, $-N(CH_2C\underline{H}_3)_2$); 1,85 (Mt, 4H, $-CH_3$ et $-C\underline{H}(CH_3)_2$); 3,10 (Mt, 2H, $-CONH-C\underline{H}_2-$); 3,17 (Mf, 4H, $-N(C\underline{H}_2CH_3)_2$); 3,4 et 3,73 (2Mt, 1H chacun, $>N-CH_2-$); 4,77 (Mt, 1H, $>N-CH<$); 7 à 7,4 (Mt, 5H, aromatiques); 7,54 (S large, 1H, -H en 1); 7,57 (D large, J = 8, 1H, -H en 3); 8,6 (T, J = 5,5, 1H, -CONH-); 9,95 (Mf, 1H, $NH^+$).

Spectre infra-rouge (KBr), bandes caractéristiques en $cm^{-1}$ :

3270, 3060, 2960, 2930, 2870, 2580, 2480, 1645, 1590, 1555, 1535, 1460, 1415, 1390, 1370, 1315, 1230, 870, 830, 755.

EXEMPLE 13

A une solution de 1,5 g de N-(méthyl-2 propyl) [(pyrrolidinyl-1)-1 propyl-2-(2RS)]-10 phénothiazinecarbothioamide-2 dans 25 cm3 d'acide acétique, on ajoute, sous agitation, 1,4 g d'acétate mercurique et la suspension obtenue est agitée pendant 1,5 heure à une température voisine de 20°C. La suspension grise est filtrée et le filtrat est concentré à sec sous pression réduite (30 mm de Hg; 4 kPa) à 50°C. L'huile trouble résiduelle est reprise par 20 cm3 d'eau distillée et 50 cm3 d'éther éthylique puis alcalinisée avec de la soude (d = 1,33) jusqu'à pH 13. La phase organique est séparée, lavée par 15 cm3 d'une solution aqueuse saturée de chlorure de sodium, séchée sur carbonate de potassium, filtrée et concentrée à sec sous pression réduite (30 mm de Hg; 4 kPa) à 50°C. La meringue pâteuse résiduelle (1 g) est purifiée par chromatographie sur une colonne (hauteur ; 30 cm; diamètre : 2,6 cm) de gel de silice 0,2-0,063 mm) en éluant successivement par 100 cm3 d'un mélange d'acétate d'éthyle et de méthanol (95-5 en volumes) et par 100 cm3 d'un mélange d'acétate d'éthyle et de méthanol (85-15 en volumes) et en recueillant des fractions de 15 cm3. Les fractions 9 à 11 sont réunies et concentrées à sec sous pression réduite (30 mm de Hg; 4 kPa) à 40°C. L'huile jaune résiduelle (0,55 g) est dissoute dans 20 cm3 d'oxyde d'isopropyle et additionnée goutte à goutte sous agitation de 0,5 cm3 d'une solution 3,3N d'acide chlorhydrique dans l'oxyde d'isopropyle. Le précipité formé est essoré, lavé par 3 fois 5 cm3 d'oxyde d'isopropyle et séché sous pression réduite (5 mm de Hg; 0,68 kPa) à 40°C. On obtient ainsi 0,51 g de chlorhydrate de N-(méthyl-2 propyl) [(pyrrolidinyl-1)-1 propyl-2-(2RS)]-10 phénothiazinecarboxamide-2 sous forme d'une poudre jaune pâle fondant à 155-160°C (fusion pâteuse).

RMN du proton (250 MHz, $CDCl_3$, $\delta$ en ppm, J en Hz).

0,98 (D, J = 7, 6H, $-CH(C\underline{H}_3)_2$); 1,87 (D, J = 7, 3H, $-CH_3$); 1,98 (Mt, 1H, $-C\underline{H}(CH_3)_2$); 2,1 (Mf, 4H, $-C\underline{H}_2-C\underline{H}_2-$ de la pyrrolidine); 2,80 - 2,92 - 3,78 et 4 (4Mt, de 1H chacun, $-C\underline{H}_2-N-C\underline{H}_2-$ de la pyrrolidine); 3,26 (Mt, 2H, $-CONH-C\underline{H}_2-$); 3,47 (D large, J = 13, 1H, 1H du $NCH_2$-); 3,7 (Mt, 1H, 1H du $N-CH_2$-); 5,25 (Mt, 1H, N-CH ); 6,95 à 7,25 (Mt, 5H, aromatiques); 7,38 (D, J = 8, 1H, -H en 3); 7,51 (S,1H, -H en 1); 12,35 (Mf, 1H, -CONH-).

Spectre infra-rouge (KBr), bandes caractéristiques en $cm^{-1}$ :

3280, 3060, 2950, 2920, 2860, 2770, 2670, 2580, 1640, 1590, 1530, 1460, 1415, 1385, 1365, 1230, 870, 825, 755.

Le N-(méthyl-2 propyl) [(pyrrolidinyl-1)-1 propyl-2-(2RS)]-10 phénothiazinecarbothioamide-2 peut être préparé de la manière suivante :

Une suspension de 2 g de [(pyrrolidinyl-1)-1 propyl-2-(2RS)]-10 phénothiazinecarbothioamide-2 et de 3 cm3 de méthyl-2 propylamine dans 20 cm3 d'éthanol absolu est saturée pendant 15 minutes avec de l'acide sulfhydrique puis chauffée pendant 23 heures à une température voisine de 115°C. Après refroidissement, on concentre à sec sous pression réduite (30 mm de Hg; 4 kPa) à 30°C. La pâte résiduelle est reprise par 30 cm3 d'acétate d'éthyle et 20 cm3 d'eau distillée. La phase organique est séparée, lavée par 20 cm3 d'une solution aqueuse saturée de chlorure de sodium, séchée sur carbonate de potassium, filtrée et concentrée à sec sous pression réduite (30 mm de Hg; 4 kPa) à 50°C. L'huile visqueuse jaune résiduelle (2,5 g) est purifiée par chromatographie sur une colonne (hauteur : 35 cm; diamètre : 2,6 cm) de gel de silice (0,2-0,063 mm) en éluant par 200 cm3 d'un mélange d'acétate d'éthyle et de méthanol (92-8 en volumes) et en recueillant des fractions de 15 cm3. Les fractions 5 à 11 sont réunies et concentrées à sec sous pression réduite (30 mm de Hg; 4 kPa) à 40°C. On obtient ainsi 2,04 g de N-(méthyl-2 propyl) [(pyrrolidinyl-1)-1 propyl-2-(2RS)]-10 phénothiazinecarbothioamide-2 sous forme d'une huile visqueuse jaune.

EXEMPLE 14

A une solution de 1,36 g de chlorhydrate de N-(méthyl-2 propyl) [(pyrrolidinyl-1)-1 propyl-2]-10 phénothiazinecarbothioamide-2, série L, dans 27 cm3 d'acide acétique, on ajoute 0,94 g d'acétate mercurique et on agite pendant 16 heures à une température voisine de 20°C. La suspension orangée obtenue est filtrée et le filtrat est concentré à sec sous pression réduite (30 mm de Hg; 4 kPa) à 40°C. Le résidu est repris par 100 cm3 d'acétate d'éthyle et la solution obtenue est lavée par 2 fois 50 cm3 d'une solution aqueuse saturée d'hydrogénocarbonate de sodium puis par 50 cm3 d'une solution aqueuse saturée de chlorure de sodium et séchée sur sulfate de magnésium en présence de noir 3S. Après filtration, le filtrat jaune est concentré à sec sous pression réduite (30 mm de Hg; 4 kPa) à 40°C et on obtient ainsi 1,15 g d'une huile orangée qui est purifiée par chromatographie sur une colonne (hauteur : 40 cm; diamètre : 2 cm) de gel de silice (0,063-0,04 mm) sous légère surpression d'azote (40 kPa) en éluant avec successivement 500 cm3 de chlorure de méthylène, 750 cm3 d'un mélange de chlorure de méthylène et de méthanol (98,7-1,3 en volumes) puis 2 litres d'un mélange de chlorure de méthylène et de méthanol (97,5-2,5 en volumes) et en recueillantdes fractions de 50 cm3 pour le premier litre d'éluat puis des fractions de 100 cm3. Les fractions 11 à 23 sont réunies et concentrées à sec sous pression réduite (30 mm de Hg; 4 kPa) à 40°C. On obtient ainsi 0,95 g d'une huile orangée. Ce produit est dissous dans

50 cm3 d'éther éthylique et on ajoute 10 cm3 d'une solution 0,23N d'acide chlorhydrique dans l'éther éthylique. Le précipité formé est essoré, lavé par 3 fois 10 cm3 d'éther éthylique et séché sous pression réduite (5 mm de Hg; 0,7 kPa) à 40°C. On obtient ainsi 0,75 g de chlorhydrate de N-(méthyl-2 propyl) [(pyrrolidinyl-1)-1 propyl-2]-10 phénothiazinecarboxamide-2, série L, sous forme d'un solide blanc fondant à 200-205°C (fusion pâteuse).

RMN du proton (250 MHz, DMSO, δ en ppm et J en Hz).

En solution dans le DMSO, on observe deux formes dues à la salification de l'azote.

0,9 (D, J = 7,5, 6H, $-CH(C\underline{H}_3)_2$); 1,78 (D, J = 7, 3H, $-CH_3$); 1,88 (Mt, 1H, $-C\underline{H}(CH_3)_2$); 1,75 à 2 (Mt, 4H, $-C\underline{H}_2-C\underline{H}_2-$ de la pyrrolidine); 2,82 - 3,09 - 3,60 et 3,75 (4Mt, 1H chacun,

$$-C\underline{H}_2-\underset{|}{N}-C\underline{H}_2-$$

de la pyrrolidine); 3,09 (Mt, 2H, $-CONH-C\underline{H}_2-$); 3,75 (AB limite, 2H, $>N-CH_2-$); 4,72 (Mt, 1H, $>N-CH<$); 7 à 7,35 (Mt, 5H aromatiques); 7,52 (S large, 1H, -H en 1); 7,55 (D large, J = 8, 1H, -H en 3); 8,60 (T, J = 5,5, 1H, -CONH-); 10,48 (Mf, 1H, $-NH^+$).

Spectre infra-rouge (KBr), bandes caractéristiques en $cm^{-1}$ :

3280, 3060, 2960, 2925, 2875, 2680, 2590, 2475, 1640, 1595, 1540, 1460, 1415, 1385, 1370, 1315, 1230, 875, 830, 755.

Le chlorhydrate de N-(méthyl-2 propyl) phénothiazinecarbothioamide-2, série L, peut être obtenu de la manière suivante :

Un mélange de 2 g de [(pyrrolidinyl-1)-1 propyl-2]-10 phénothiazinecarbothioamide-2, série L, et de 2,75 cm3 de méthyl-2 propylamine dans 40 cm3 d'éthanol est saturé d'acide sulfhydrique puis chauffé pendant 16 heures à 100°C. Après refroidissement, la solution est concentrée à sec sous pression réduite (30 mm de Hg; 4 kPa) à 40°C et on obtient 2,7 g d'une huile orangée qui est purifiée par chromatographie sur une colonne (hauteur : 40 cm; diamètre : 4 cm) de gel de silice (0,063-0,04 mm) sous légère surpression d'azote (300 mm de Hg; 40 kPa) en éluant successivement avec 500 cm3 de chlorure de méthylène puis 500 cm3 d'un mélange de chlorure de méthylène et de méthanol (97,5-2,5 en volumes) et en recueillant des fractions de 60 cm3. Les fractions 12 à 16 sont réunies et concentrées à sec sous pression réduite (30 mm de Hg; 4 kPa) à 40°C et on obtient 1,72 g d'une huile orangée. Ce produit est dissous dans 50 cm3 d'éther éthylique et la solution est traitée au noir 3S et filtrée; à ce filtrat, on ajoute 1,5 cm3 d'une solution 3N d'acide chlorhydrique dans l'éther éthylique. Le précipité formé est essoré, lavé par 3 fois 10 cm3 d'éther éthylique et séché sous pression réduite (5 mm de Hg; 0,7 kPa) à 40°C. On obtient ainsi 1,4 g de chlorhydrate de N-(méthyl-2 propyl) [(pyrrolidinyl-1)-1 propyl-2]-10 phénothiazinecarbothioamide-2, série L, sous forme d'un solide jaune fondant vers 120°C (fusion pâteuse).

$[\alpha]_D^{20}$ = +24 ±0,5° (1%; diméthylformamide).

EXEMPLE 15

A une solution de 0,95 g de chlorhydrate de N-(méthyl-2 propyl) [(pyrrolidinyl-1)-1 propyl-2]-10 phénothiazinecarbothioamide-2, série D, dans 19 cm3 d'acide acétique, on ajoute 0,65 g d'acétate mercurique et on agite pendant 24 heures à une température voisine de 20°C. La suspension orangée obtenue est filtrée et le filtrat est concentré à sec sous pression réduite (30 mm de Hg; 4 kPa) à 40°C. Le résidu est repris par 100 cm3 d'acétate d'éthyle et la solution obtenue est lavée par 2 fois 50 cm3 d'une solution aqueuse saturée d'hydrogénocarbonate de sodium puis par 50 cm3 d'une solution aqueuse saturée de chlorure de sodium et séchée sur sulfate de magnésium en présence de noir 3S. Après filtration, le filtrat jaune est concentré à sec sous pression réduite (30 mm de Hg; 4 kPa) à 40°C et on obtient ainsi 0,76 g d'une huile orangée. Ce produit est purifié par chromatographie sur une colonne (hauteur : 40 cm; diamètre : 2 cm) de gel de silice (0,063-0,04 mm) sous légère surpression d'azote (40 kPa) en éluant avec successivement 500 cm3 de chlorure de méthylène, 750 cm3 d'un mélange de chlorure de méthylène et de méthanol (98,7-1,3 en volumes) puis 1 litre d'un mélange de chlorure de méthylène et de méthanol (97,5-2,5 en volumes) et en recueillant des fractions de 50 cm3 pour le premier litre d'éluat puis desdes fractions de 80 cm3. Les fractions 15 à 28 sont réunies et concentrées à sec sous pression réduite (30 mm de Hg; 4 kPa) à 40°C. On obtient ainsi 0,47 g d'une huile orangée. Ce produit est dissous dans 30 cm3 d'éther éthylique et on ajoute 5 cm3 d'une solution 0,23N d'acide chlorhydrique dans l'éther éthylique. Le précipité formé est essoré, lavé par 3 fois 5 cm3 d'éther éthylique et séché sous pression réduite (5 mm de Hg; 0,7 kPa) à 40°C. On obtient ainsi 0,4 g de chlorhydrate de N-(méthyl-2 propyl) [(pyrrolidinyl-1)-1 propyl-2]-10 phénothiazinecarboxamide-2, série D, sous forme d'un solide blanc fondant à 190-200°C (fusion pâteuse) et dont le spectre de RMN est identique à celui du produit décrit à l'exemple 14.

$[\alpha]_D^{20}$ = -16,7 ± 0,5° (1%; diméthylformamide).

Spectre infra-rouge (KBr), bandes caractéristiques en $cm^{-1}$ :

3280, 3060, 2960, 2925, 2875, 2685, 2610, 2480, 1640, 1595, 1540, 1460, 1415, 1385, 1370, 1320, 1230, 875, 830, 755.

Le chlorhydrate de N-(méthyl-2 propyl) [(pyrrolidinyl-1)-1 propyl-2]-10 phénothiazinecarbothioamide-2, série D, peut être préparé de la manière suivante :

Un mélange de 2 g de [(pyrrolidinyl-1)-1 propyl-2]-10 phénothiazinecarbothioamide-2, série D, et de 2,75 cm3 de méthyl-2 propylamine dans 40 cm3 d'éthanol est saturé d'acide sulfhydrique puis chauffé pendant 16 heures à 100°C. Après refroidissement, la solution est concentrée à sec sous pression réduite (30 mm de Hg; 4 kPa) à 40°C, on obtient 2,3 g d'une huile orangée. Ce produit est purifié par chromatographie sur une colonne (hauteur : 40 cm; diamètre : 4 cm) de gel de silice (0,063-0,04 mm) sous légère surpression d'azote (40 kPa) en éluant successivement avec 500 cm3 de chlorure de méthylène puis 500 cm3 d'un mélange de chlorure de méthylène et de méthanol (97,5-2,5 en volumes) et en recueillant des fractions de 60 cm3. Les fractions 12 à 16 sont réunies et concentrées à sec sous pression réduite (30 mm de Hg; 4 kPa) à 40°C et on obtient 1,25 g d'une huile orangée. Ce produit est dissous dans 50 cm3 d'éther éthylique et la solution est traitée au noir 3S et filtrée; à ce filtrat, on ajoute 1 cm3 d'une solution 3N d'acide chlorhydrique dans l'éther éthylique. Le précipité formé est essoré, lavé par 3 fois 10 cm3 d'éther éthylique séché sous pression réduite (5 mm de Hg; 0,7 kPa) à 40°C. On obtient ainsi 1 g de chlorhydrate de N-(méthyl-2 propyl) [(pyrrolidinyl-1)-1 propyl-2]-10 phénothiazinecarbothioamide-2, série D, sous forme d'un solide jaune fondant vers 120°C (fusion pâteuse).

EXEMPLE 16

Une suspension de 1,7 g de N-pentyl [(pyrrolidinyl-1)-1 propyl-2-(2RS)]-10 phénothiazinecarbothioamide-2 et de 1,4 g d'acétate mercurique dans 25 cm3 d'acide acétique est agitée 16 heures à une température voisine de 20°C. La suspension noire obtenue est diluée par 15 cm3 d'éther éthylique, filtrée et le filtrat est concentré à sec sous pression réduite (30 mm de Hg; 4 kPa) à 40°C. Le résidu brun est repris par 20 cm3 d'eau distillée, alcalinisé jusqu'à pH 13 par de la soude (d = 1,33) et extrait par 25 cm3 d'acétate d'éthyle. La phase organique est lavée par 10 cm3 d'eau distillée, séchée sur carbonate de potassium, filtrée et concentrée à sec sous pression réduite (30 mm de Hg; 4 kPa) à 50°C. L'huile visqueuse brun clair résiduelle (1,5 g) est purifiée par chromatographie sur une colonne (hauteur : 45 cm; diamètre : 3,2 cm) de gel de silice (0,04-0,063 mm) sous une légère surpression d'azote (40 kPa) en éluant par 800 cm3 d'un mélange d'acétate d'éthyle et de méthanol (90-10 en volumes) et en recueillant des fractions de 25 cm3. Les fractions 13 à 23 sont réunies et concentrées à sec sous pression réduite (30 mm de Hg; 4 kPa) à 40°C. L'huile visqueuse résiduelle est dissoute dans 25 cm3 d'éther éthylique et on ajoute, goutte à goutte sous agitation, 1 cm3 d'une solution 3,3N d'acide chlorhydrique dans l'oxyde d'isopropyle. Le précipité formé est essoré, lavé par 2 fois 5 cm3 d'oxyde d'isopropyle et séché sous pression réduite (5 mm de Hg; 0,7 kPa) à 40°C. On obtient ainsi 0,9 g de chlorhydrate de N-pentyl [(pyrrolidinyl-1)-1 propyl-2-(2RS)]-10 phénothiazinecarboxamide-2 sous forme d'une poudre blanche fondant à 214°C.

RMN du proton (250 MHz, DMSO, δ en ppm, J en Hz).

0,9 (T, J = 7, 3H, $-CH_2-C\underline{H}_3$); 1,32 (Mt, 4H, $-C\underline{H}_2-C\underline{H}_2-CH_3$); 1,55 (Mt, 2H, $-C\underline{H}_2-(CH_2)_2-CH_3$); 1,8 (D, J = 7, 3H, $-CH_3$); 1,75 à 2 (Mt, 4H, $-CH_2-$ de la pyrrolidine); 2,85 - 3,10 - 3,6 et 3,75 (4Mt, de 1H chacun, $>NCH_2-$ de la pyrrolidine); 3,26 (Mt, 2H, $-CONH-C\underline{H}_2-$); 3,77 (Mt, 2H, $>N-CH_2-$); 4,77 (Mt, 1H, $>N-CH<$); 7 à 7,4 (Mt, 5H, aromatiques); 7,53 (S, 1H, -H en 1); 7,55 (D, J = 8, 1H, -H en 3); 8,66 (T, J = 5,5, 1H, -CONH-); 10,80 (Mf, 1H, $-NH^+$).

Spectre infra-rouge (KBr), bandes caractéristiques en $cm^{-1}$ :

3270, 3060, 2950, 2920, 2860, 2850, 2670, 2610, 2580, 1645, 1590, 1535, 1460, 1410, 1375, 1305, 1230, 860, 830, 750.

Le N-pentyl [(pyrrolidinyl-1)-1 propyl-2-(2RS)]-10 phénothiazinecarbothioamide-2 peut être obtenu de la manière suivante :

Une suspension de 2 g de [(pyrrolidinyl-1)-1 propyl-2-(2RS)]-10 phénothiazinecarbothioamide-2 et de 3 cm3 de pentylamine dans 30 cm3 d'éthanol absolu est saturée avec de l'acide sulfhydrique puis chauffée pendant 2 heures à une température voisine de 100°C. Après refroidissement, on concentre à sec sous pression réduite (30 mm de Hg; 4 kPa) à 40°C. Le résidu pâteux est repris par 50 cm3 d'éther éthylique et 15 cm3 d'eau distillée. La phase organique est séparée, séchée sur carbonate de potassium et concentrée à sec sous pression réduite (30 mm de Hg; 4 kPa) à 40°C. L'huile jaune visqueuse résiduelle est purifiée par chromatographie sur une colonne (hauteur : 35 cm; diamètre : 2,6 cm) de gel de silice (0,2-0,063 mm) en éluant par 300 cm3 d'acétate d'éthyle et en recueillant des fractions de 25 cm3. Les fractions 3 à 9 sont réunies et concentrées à

sec sous pression réduite (30 mm de Hg; 4 kPa) à 40°C. On obtient ainsi 2,25 g de N-pentyl [(pyrrolidinyl-1)-1 propyl-2-(2RS)]-10 phénothiazinecarbothioamide-2 sous forme d'une huile jaune.

RMN du proton (250 MHz, DMSO, δ en ppm, J en Hz).

0,92 (T, J = 7, 3H, $-CH_3$ du pentyle); 1,35 (Mt, 4H, $-C\underline{H}_2-C\underline{H}_2-CH_3$); 1,7 (Mt, 2H, $-C\underline{H}_2-(CH_2)_2CH_3$); 3,55 à 3,90 (Mt, 4H, $N-CH_2-$ et $-CSNH-C\underline{H}_2-$); 7 à 7,35 (Mt, 5H, aromatiques); 7,43 (DD, J = 8 et 1, 1H, -H en 3); 7,57 (D, J = 1, 1H, -H en 1).

EXEMPLE 17

On dissout 1 g de N-[méthyl-2 butyl-(2RS)] [N'-éthyl N'-méthyl-amino-1 propyl-2-(2RS)]-10 phénothiazinecarbothioamide-2 dans une solution de 0,27 g d'acide fumarique dans 20 cm3 d'éthanol absolu et on concentre à sec sous pression réduite (30 mm de Hg; 4 kPa) à 40°C. Le résidu est dissous dans 16,7 cm3 d'acide acétique et on ajoute 0,78 g d'acétate mercurique. La suspension obtenue est agitée pendant 16 heures à une température voisine de 20°C pour donner une suspenson noire qui est diluée avec 25 cm3 d'eau distillée et filtrée. Le filtrat orangé est alcalinisé avec de la lessive de soude (d = 1,33) jusqu'à pH 13 et extrait par 2 fois 50 cm3 d'acétate d'éthyle. Les phases organiques réunies sont lavées successivement par 2 fois 25 cm3 d'eau distillée et par 25 cm3 d'une solution aqueuse saturée de chlorure de sodium, séchées sur sulfate de magnésium en présence de noir 3S, filtrées et concentrées à sec sous pression réduite (30 mm de Hg; 4 kPa) à 40°C. L'huile jaune résiduelle (1 g) est purifiée par chromatographie sur une colonne (hauteur : 25 cm; diamètre : 2 cm) de gel de silice (0,04-0,063 mm) sous une légère surpression d'azote (40 kPa) en éluant successivement par 500 cm3 d'acétate d'éthyle et par 1,2 litre d'un mélange d'acétate d'éthyle et de méthanol (90-10 en volumes) et en recueillant des fractions de 50 cm3. Les fractions 21 à 30 sont réunies et concentrées à sec sous pression réduite (30 mm de Hg; 4 kPa) à 40°C. On obtient 0,46 g d'une huile jaune qui cristallise. Ce produit est dissous dans 10 cm3 d'oxyde d'isopropyle au reflux puis laissé au repos 1 heure à une température voisine de 5°C. Les cristaux formés sont essorés, lavés par 2 fois 2 cm3 d'oxyde d'isopropyle et séchés sous pression réduite (5 mm de Hg; 0,7 kPa) à 40°C. On obtient ainsi 0,23 g de N-[méthyl-2 butyl-(2RS)] [N'-éthyl N'-méthyl-amino-1 propyl-2 -(2RS)]-10 phénothiazinecarboxamide-2 sous forme de cristaux blanc cassé fondant à 95°C.

RMN du proton (250 MHz, DMSO, δ en ppm et J en Hz).

0,9 (Mt, 9H, $2-CH_3$ du méthyl-2 butyle, $>NCH_2C\underline{H}_3$); 1,14 et 1,44 (2Mt, 1H chacun, $-C\underline{H}_2-CH_3$); 1,62 (D, J = 7, 3H, $-CH_3$); 1,65 (Mt,1H, ⟍CH- du méthyl-2 butyle); 2,23 (S, 3H, ⟍$N-CH_3$); 2,41 (Mt, 2H, $>N-C\underline{H}_2-CH_3$); 2,67 (DD, J = 14 et 6,5, 1H, 1H du $>N-CH_2-$); 2,97 (DD, J = 14 et 6, 1H, 1H du $>NCH_2-$); 3 à 3,3 (Mt, 2H, $-CONH-C\underline{H}_2-$); 4,17 (Mt, J = 7 - 6,5 et 6, 1H, $>N-CH<$); 6,90 à 7,3 (Mt, 5H, aromatiques); 7,42 (DD, J = 8 et 1, 1H, -H en 3); 7,55 (D, J = 1, 1H, -H en 1); 8,4 (T, J = 5,5, 1H, -CONH-).

Spectre infra-rouge (KBr), bandes caractéristiques en $cm^{-1}$ ;

3320, 3060, 2965, 2930, 2875, 2845, 2790, 1630, 1590, 1580, 1540, 1460, 1415, 1380, 1320 1240, 825, 750.

Le N-[méthyl-2 butyl-(2RS)] [(N'-éthyl N'-méthyl-amino-1 propyl-2-(2RS)]-10 phénothiazinecarbothioamide-2 peut être préparé de la manière suivante :

Une solution de 1 g de [N-éthyl N-méthyl-amino-1 propyl-2-(2RS)]-10 phénothiazinecarbothioamide-2 et de 4,9 cm3 de méthyl-2 butylamine-(2RS) dans 15 cm3 d'éthanol absolu est saturée avec de l'acide sulfhydrique et chauffée pendant 16 heures à une température voisine de 100°C. Après refroidissement, on concentre à sec sous pression réduite (30 mm de Hg; 4 kPa) à 40°C. Le résidu huileux orangé est purifié par chromatographie sur une colonne (hauteur : 25 cm, diamètre : 2 cm) de gel de silice (0,04-0,063 mm) sous une légère surpression d'azote (40 kPa) en éluant par 500 cm3 d'un mélange d'acétate d'éthyle et de cyclohexane (80-20 en volumes) et en recueillant des fractions de 40 cm3. Les fractions 4 à 8 sont réunies et concentrées à sec sous pression réduite (30 mm de Hg; 4 kPa) à 40°C. On obtient 1,88 g de N-[méthyl-2 butyl-(2RS)] [N'-éthyl N'-méthyl-amino-1 propyl-2-(2RS)]-10 phénothiazinecarbothioamide-2 sous forme d'une huile jaune.

EXEMPLE 18

Dans une solution de 1,4 g de N-[méthyl-2 butyl-(2RS) [(pyrrolidinyl-1)-1 propyl-2-(2RS)]-10 phénothiazinecarbothioamide-2 dans 25 cm3 d'acide acétique, on ajoute 1,3 g d'acétate mercurique et on agite la suspension obtenue pendant 2 heures à une température voisine de 20°C. Le précipité noir formé est essoré et lavé par 2 fois 5 cm3 d'acide acétique et le filtrat est concentré à sec sous pression réduite (30 mm de Hg; 4 kPa) à 60°C. Le résidu brun est repris par 15 cm3 d'eau distillée et 25 cm3 d'acétate d'éthyle, alcalinisé par une solution aqueuse de soude N. La phase organique est séparée, filtrée sur supercel, séchée sur carbonate de potassium, filtrée à nouveau et concentrée à sec sous pression réduite (30 mm de Hg; 4 kPa) à 20°C. La

laque visqueuse jaune pâle obtenue (1,3 g) est dissoute dans 50 cm3 d'oxyde d'isopropyle et additionnée de 1 cm3 d'une solution d'acide chlorhydrique 3,3N dans l'oxyde d'isopropyle. Le précipité est essoré, lavé par 3 fois 10 cm3 d'oxyde d'isopropyle et séché sous pression réduite (5 mm de Hg; 0,7 kPa) à 40°C. On obtient ainsi 1,2 g de chlorhydrate de N-[méthyl-2 butyl-(2RS) [(pyrrolidinyl-1)-1 propyl-2-(2RS)]-10 phénothiazinecarboxamide-2 sous forme d'une poudre blanc cassé fondant à 155-160°C (fusion pâteuse).

RMN du proton (250 MHz, DMSO, δ en ppm, J en Hz).

0,89 et 0,90 (D et T, J = 7, 6H, respectivement CH-C<u>H</u>$_3$ et -$CH_2$C<u>H</u>$_3$); 1,13 et 1,43 (2Mt, de 1H chacun, -C<u>H</u>$_2$$CH_3$); 1,7 (Mt, 1H,>CH- du méthyl-2 butyle); 1,79 (D, J = 7, 3H, -$CH_3$); 1,75 à 2 (Mt, 4H, -C<u>H</u>$_2$-C<u>H</u>$_2$- de la pyrrolidine); 2,85 - 3,10 - 3,60 et 3,75 (4Mf, de 1H chacun, 2 >N-$CH_2$- de la pyrrolidine); 3 à 3,3 (Mt, 2H, -CONH-C<u>H</u>$_2$-); 3,65 à 3,90 (Mt, 2H, >N-$CH_2$-); 4,8 (Mt, 1H, >N-CH<); 7 à 7,4 (Mt, 5H, aromatiques); 7,55 (S, 1H, -H en 1); 7,57 (D, J = 8, 1H, -H en 3); 8,65 (T, J = 5,5, 1H, -CONH-); 10,85 (Mf, 1H, -$NH^+$).

Spectre infra-rouge (KBr), bandes caractéristiques en $cm^{-1}$ :

3290, 3060, 2980, 2925, 2875, 2670, 2585, 2475, 1645, 1595, 1535, 1460, 1410, 1380, 1305, 1230, 830, 755.

Le N-[méthyl-2 butyl-(2RS)] [(pyrrolidinyl-1)-1 propyl-2-(2RS)]-10 phénothiazinecarbothioamide-2 peut être préparé de la manière suivante :

On sature d'acide sulfhydrique une suspension agitée de 2 g de [(pyrrolidinyl-1)-1 propyl-2-(2RS)]-10 phénothiazinecarbothioamide-2 et de 3 cm3 de méthyl-2 butylamine dans 15 cm3 d'éthanol anhydre et on chauffe pendant 1 heure à une température voisine de 115°C. Après refroidissement, on concentre à sec sous pression réduite (30 mm de Hg; 4 kPa) à 50°C. La pâte jaune résiduelle est reprise par 30 cm3 d'acétate d'éthyle et 20 cm3 d'eau distillée.

La phase organique est séparée, lavée par 20 cm3 d'une solution aqueuse saturée de chlorure de sodium, séchée sur sulfate de magnésium, filtrée et concentrée à sec sous pression réduite (30 mm de Hg; 4 kPa) à 40°C. L'huile jaune visqueuse résiduelle (2,2 g) est purifiée par chromatographie sur une colonne (hauteur : 40 cm, diamètre : 3,2 cm) de gel de silice (0,04-0,063 mm) en éluant avec 300 cm3 d'un mélange d'acétate d'éthyle et de méthanol (92-8 en volumes) et en recueillant des fractions de 25 cm3. Les fractions 6 à 11 sont réunies et concentrées à sec sous pression réduite (30 mm de Hg; 4 kPa) à 40°C. On obtient ainsi 1,8 g de N-[méthyl-2 butyl-(2RS)] [(pyrrolidinyl-1)-1 propyl-2-(2RS)]-10 phénothiazinecarbothioamide-2 sous forme d'une huile jaune visqueuse.

## EXEMPLE 19

A une solution de 2,5 g de chlorhydrate de N-[méthyl-2 butyl-(2S)] [(pyrrolidinyl-1)-1 propyl-2]-10 phénothiazinecarbothioamide-2, série L, dans 50 cm3 d'acide acétique, on ajoute 2,2 g d'acétate mercurique et on agite pendant 16 heures à une température voisine de 20°C. La suspension orangée obtenue est diluée par 100 cm3 d'eau distillée, filtrée et le filtrat est concentré à sec sous pression réduite (30 mm de Hg; 4 kPa) à 40°C. Le résidu est repris par 200 cm3 d'acétate d'éthyle et 100 cm3 d'eau distillée. On ajoute de la soude (d = 1,33) jusqu'à pH 13. La phase organique est décantée, lavée successivement par 2 fois 50 cm3 d'eau distillée et par 50 cm3 d'une solution aqueuse saturée de chlorure de sodium et séchée sur sulfate de magnésium. Après filtration, le filtrat jaune est concentré à sec sous pression réduite (30 mm de Hg; 4 kPa) à 40°C; on obtient ainsi 1,9 g d'une huile orangée. Ce produit est dissous dans 10 cm3 d'acétate d'éthyle et on ajoute, goutte à goutte et sous agitation, 1,4 cm3 d'une solution 3,3N d'acide chlorhydrique dans l'oxyde d'isopropyle. Les cristaux formés sont séparés par filtration, lavés par 3 fois 10 cm3 d'oxyde d'isopropyle et séchés sous pression réduite (5 mm de Hg; 0,7 kPa). On obtient ainsi 1,05 g de chlorhydrate de N-[méthyl-2 butyl-(2S)] [(pyrrolidinyl-1)-1 propyl-2]-10 phénothiazinecarboxamide-2, série L, sous forme d'un solide beige fondant à 190°C dont le spectre de RMN est identique à celui du produit décrit à l'exemple 18.

Spectre infra-rouge (KBr), bandes caractéristiques en $cm^{-1}$ :

3280, 3060, 2960, 2930, 2875, 2680, 2590, 2480, 1640, 1595, 1540, 1460, 1415, 1380, 1310, 1235, 860, 830, 755.

Le chlorhydrate de N-[méthyl-2 butyl-(2S)] [(pyrrolidinyl-1)-1 propyl-2]-10 phénothiazinecarbothioamide-2, série L, peut être obtenu de la manière suivante :

Un mélange de 4 g de [(pyrrolidinyl-1)-1 propyl-2]-10 phénothiazinecarbothioamide-2, série L, et de 5 g de méthyl-2 butylamine-(2S) dans 60 cm3 d'éthanol est saturé d'acide sulfhydrique puis chauffé pendant 16 heures à 100°C. Après refroidissement, la solution est concentrée à sec sous pression réduite (30 mm de Hg; 4 kPa) à 40°C. On obtient 5,3 g d'une huile orangée que l'on purifie par chromatographie sur colonne (hauteur : 48 cm; diamètre : 4 cm) de gel de silice (0,063-0,2 mm) en éluant par 2 litres d'un mélange de chlorure de méthylène et de méthanol (97,5-2,5 en volumes) et en recueillant des fractions de 60 cm3. Les fractions 23 à 29 sont réunies et concentrées à sec sous pression réduite (30 mm de Hg; 4 kPa) à 40°C. On obtient 4,1 g

d'une huile orangée. Ce produit est dissous dans 200 cm3 d'un mélange d'oxyde d'isopropyle et d'acétate d'éthyle (75-25 en volumes) puis on ajoute, sous agitation et à une température voisine de 5°C, 12,8 cm3 d'une solution 0,72N d'acide chlorhydrique dans l'oxyde d'isopropyle. Le précipité formé est essoré, lavé par 3 fois 20 cm3 d'oxyde d'isopropyle et séché sous pression réduite (5 mm de Hg; 0,7 kPa) à 40°C. On obtient ainsi 3,2 g de chlorhydrate de N-[méthyl-2 butyl-(2S)] phénothiazinecarbothioamide-2, série L, sous forme d'un solide jaune fondant à 135-140°C (fusion pâteuse) et dont le spectre de RMN est identique à celui du thioamide décrit à l'exemple 18.

$[\alpha]_D^{20}$= +28,3 ±0,6° (1%; diméthylformamide).

## EXEMPLE 20

A une solution de 0,8 g de N-[méthyl-2 butyl-(2RS)] [(pyrrolidinyl-1)-1 propyl-2]-10 phénothiazinecarbothioamide-2, série L, dans 16 cm3 d'acide acétique, on ajoute 0,7 g d'acétate mercurique et on agite pendant 16 heures à une température voisine de 20°C. La suspension orangée obtenue est diluée par 50 cm3 d'eau distillée, filtrée et le filtrat orangé est concentré à sec sous pression réduite (30 mm de Hg; 4 kPa) à 40°C, Le résidu est repris par 100 cm3 d'acétate d'éthyle et 25 cm3 d'eau distillée. On ajoute de la lessive de soude (d = 1,33) jusqu'à pH 13. La phase organique est décantée, lavée successivement par 2 fois 25 cm3 d'eau distillée et par 25 cm3 d'une solution aqueuse saturée de chlorure de sodium et séchée sur sulfate de magnésium. Après filtration, le filtrat jaune est concentré à sec sous pression réduite (30 mm de Hg; 4 kPa) à 40°C et on obtient ainsi 0,5 g d'une gomme jaune. Ce produit est dissous dans 25 cm3 d'oxyde d'isopropyle puis on ajoute goutte à goutte, sous agitation et à une température voisine de 5°C, 5,4 cm3 d'une solution 0,24N d'acide chlorhydrique dans l'oxyde d'isopropyle. Le précipité formé est séparé par filtration, lavé par 3 fois 10 cm3 d'oxyde d'isopropyle et séché sous pression réduite (5 mm de Hg; 0,68 kPa). On obtient ainsi 0,4 g de chlorhydrate de N-[méthyl-2 butyl-(2RS)] [(pyrrolidinyl-1)-1 propyl-2]-10 phénothiazinecarboxamide-2, série L, sous forme d'un solide blanc fondant à 178°C et dont le spectre de RMN est identique à celui du produit décrit à l'exemple 18.

$[\alpha]_D^{20}$ = +15,5 ±0,5° (1%; diméthylformamide).

Spectre infra-rouge (KBr), bandes caractéristiques en $cm^{-1}$ ;

3310, 3060, 2960, 2925, 2870, 2680, 2600, 2475, 1635, 1590, 1540, 1470, 1410, 1380, 1310, 1230, 870, 830, 755.

Le chlorhydrate de N-[méthyl-2 butyl-(2RS)] [(pyrrolidinyl-1)-1 propyl-2]-10 phénothiazinecarbothioamide-2, série L, peut être obtenu de la manière suivante :

Un mélange de 2 g de [(pyrrolidinyl-1)-1 propyl-2]-10 phénothiazinecarbothioamide-2, série L, et de 9,5 cm3 de méthyl-2 butylamine-(2RS) dans 30 cm3 d'éthanol est saturé d'acide sulfhydrique puis chauffé pendant 16 heures à 100°C. Après refroidissement, la solution est concentrée à sec sous pression réduite (30 mm de Hg; 4 kPa) à 40°C. On obtient 3,8 g d'une huile orangée que l'on purifie par chromatographie sur colonne (hauteur : 40 cm; diamètre : 3 cm) de gel de silice (0,063-0,2 mm) en éluant par 1,5 litre d'un mélange de chlorure de méthylène et de méthanol (97,5-2,5 en volumes) et en recueillant des fractions de 30 cm3. Les fractions 35 à 46 sont réunies et concentrées à sec sous pression réduite (30 mm de Hg; 4 kPa) à 40°C. On obtient 1,8 g d'une huile orangée. Ce produit est dissous dans 60 cm3 d'un mélange d'oxyde d'isopropyle et d'acétate d'éthyle (80-20 en volumes) puis on ajoute, sous agitation et à une température voisine de 5°C, 11 cm3 d'une solution 0,37N d'acide chlorhydrique dans l'oxyde d'isopropyle. Le précipité formé est essoré, lavé par 3 fois 10 cm3 d'oxyde d'isopropyle et séché sous pression réduite (5 mm de Hg; 0,7 kPa) à 40°C. On obtient ainsi 1,4 g de chlorhydrate de N-[méthyl-2 butyl-(2RS)] [(pyrrolidinyl-1)-1 propyl-2]-10 phénothiazinecarbothioamide-2, série L, sous forme d'un solide jaune fondant à 120-125°C (fusion pâteuse) et dont le spectre de RMN est identique à celui du thioamide décrit à l'exemple 18.

$[\alpha]_D^{20}$ = +2 ,3 ±0,8° (0,7%; diméthylformamide).

## EXEMPLE 21

Une solution de 2 g de (diéthylamino-1 propyl-2)-10 N-(méthyl-3 butyl-1) phénothiazinecarbothioamide-2, série L, et de 0,52 g d'acide fumarique dans 20 cm3 d'éthanol est concentré à sec sous pression réduite (30 mm de Hg; 4 kPa) à 40°C. Le résidu est repris sous agitation par 35 cm3 d'acide acétique puis on ajoute 1,5 g d'acétate mercurique et on poursuit l'agitation à une température voisine de 20°C pendant 16 heures. La suspension grise obtenue est diluée par 60 cm3 d'eau distillée et filtrée. Le filtrat orangé est additionné de lessive de soude (d = 1,33) jusqu'à pH 13 et extrait par 200 cm3 d'acétate d'éthyle. La phase organique est lavée successivement par deux fois 50 cm3 d'eau distillée et par 50 cm3 d'une solution aqueuse saturée de chlorure

de sodium et séchée sur sulfate de magnésium, en présence de noir 3S. Après filtration, le filtrat jaune est concentré à sec sous pression réduite (30 mm de Hg; 4 kPa) à 40°C et on obtient ainsi 1,55 g d'une huile orangée. Ce produit est purifié par chromatographie sur une colonne (hauteur : 25 cm; diamètre : 2,6 cm) de gel de silice (0,04-0,063 mm) sous légère surpression d'azote (40 kPa) en éluant avec 750 cm3 d'acétate d'éthyle puis avec 1500 cm3 d'un mélange d'acétate d'éthyle et de méthanol (90-10 en volumes) et en recueillant des fractions de 50 cm3. Les fractions 12 à 44 sont réunies et concentrées à sec sous pression réduite (30 mm de Hg; 4 kPa) à 40°C. On obtient 1,1 g d'une huile jaune ($[\alpha]_D^{20}$ = +15,6°; 0,64%; chloroforme). Ce produit est dissous dans 100 cm3 d'oxyde d'isopropyle puis on ajoute sous agitation, à une température voisine de 5°C, 5,8 cm3 d'une solution 0,45N d'acide chlorhydrique dans l'oxyde d'isopropyle et on poursuit l'agitation pendant 30 minutes. Le précipité formé est essoré, lavé par 2 cm3 d'oxyde d'isopropyle et séché à 40°C sous pression réduite (5 mm de Hg; 0,7 kPa). On obtient ainsi 0,88 g de chlorhydrate de (diéthylamino-1 propyl-2)-10 N-(méthyl-3 butyl-1) phénothiazinecarboxamide-2, série L, sous forme d'un solide blanc fondant à 100-105°C (fusion pâteuse).

$[\alpha]_D^{20}$ = +15,7° (0,9%; diméthylformamide).

RMN du proton (250 MHz, DMSO, δ en ppm, J en Hz).

0,93 (D, J = 7, 6H, $-CH(C\underline{H_3})_2$); 0,98 et 1,17 (2T, J = 7, 3H chacun, $-N(CH_2C\underline{H_3})_2$); 1,44 (Q, J = 7, 2H, >N-$CH_2C\underline{H_2}$-); 1,62 (Mt, 1H, >CH- du méthyl-3 butyle); 1,85 (D, J = 7, 3H, $-CH_3$); 3,17 (Mt, 4H, $-N(C\underline{H_2}CH_3)_2$); 3,3 (Mt, 2H, -CONH-$C\underline{H_2}$-); 3,4 (Mt, 1H, 1H du >N-$CH_2$-); 3,76 (DD, J = 14 et 7,5, 1H, 1H du N-$CH_2$-); 4,81 (Mt, 1H, >N-CH<); 7 à 7,4 (Mt, 5H, aromatiques); 7,52 (S, 1H, -H en 1); 7,53 (D, J = 7,5, 1H, -H en 3); 8,6 (T, J = 5,5, 1H, -CONH-); 10,13 (Mf, 1H, $-NH^+$).

Spectre infra-rouge (KBr), bandes caractéristiques en $cm^{-1}$ :

3270, 3060, 2955, 2940, 2870, 2640, 2580, 2480, 1640, 1590, 1535, 1460, 1415, 1395, 1385, 1365, 1310, 1230, 875, 830, 755.

Le (diéthylamino-1 propyl-2)-10 N-(méthyl-3 butyl-1) phénothiazinecarbothioamide-2, série L, peut être préparé de la manière suivante :

A une solution de 3 g de fumarate acide de (diéthylamino-1 propyl-2)-10 phénothiazinecarbothioamide-2, série L, dans 45 cm3 d'éthanol absolu, on ajoute 10,7 cm3 de méthyl-3 butylamine et on sature cette solution avec de l'acide sulfhydrique. Le mélange est ensuite porté à une température voisine de 105°C pendant 16 heures et concentré à sec sous pression réduite (30 mm de Hg; 4 kPa). L'huile orangée résiduelle est purifiée par chromatographie sur une colonne (hauteur : 35 cm; diamètre : 3 cm) de gel de silice (0,2-0,063 mm) en éluant avec 400 cm3 d'un mélange d'acétate d'éthyle et de cyclohexane (70-30 en volumes) en recueillant des fractions de 30 cm3. Les fractions 7 à 11 sont réunies et concentrées à sec sous pression réduite (30 mm de Hg; 4 kPa) à 50°C pour donner 3,1 g de (diéthylamino-1 propyl-2)-10 N-(méthyl-3 butyl-1) phénothiazinecarbothioamide-2, série L, sous forme d'une huile orangée dont les caractéristiques de RMN sont identiques à celles décrites ci-après à l'exemple 22.

Le fumarate acide de (diéthylamino-1 propyl-2)-10 phénothiazinecarbothioamide-2, série L, peut être préparé de la manière suivante :

Une solution de 5,2 g de (diéthylamino-1 propyl-2)-10 phénothiazinecarbonitrile-2, série L, et de 2,2 cm3 de triéthylamine dans 104 cm3 de pyridine anhydre est saturée d'acide sulfhydrique à 20°C pendant 1 heure sous agitation puis agitée à 20°C pendant 17 heures. Le mélange réactionnel est purgé à l'azote pendant 1 heure, versé dans 500 cm3 d'eau distillée et extrait par 2 fois 250 cm3 d'acétate d'éthyle. Les phases organiques réunies sont lavées successivement par 3 fois 100 cm3 d'eau distillée et par 100 cm3 d'une solution aqueuse saturée de chlorure de sodium, séchées sur sulfate de magnésium et filtrées. Le filtrat jaune est concentré à sec sous pression réduite (30 mm de Hg; 4 kPa) à 40°C pour donner 6,5 g d'une huile orangée. Ce produit est purifié par chromatographie sur une colonne (hauteur : 44 cm; diamètre : 3,4 cm) de gel de silice (0,2-0,063 mm) en éluant par 3 litres d'un mélange de cyclohexane et d'acétate d'éthyle (30-70 en volumes) et en recueillant des fractions de 150 cm3. Les fractions 8 à 17 sont réunies et concentrées à sec sous pression réduite (30 mm de Hg; 4 kPa) à 40°C. On obtient 5,08 g d'une huile orangée ($[\alpha]_D^{20}$ = -33,7° ±0,6°; 1,006%; chloroforme). Ce produit est dissous à une température voisine de 60°C dans 20 cm3 d'éthanol et cette solution est versée dans une solution de 1,56 g d'acide fumarique dans 20 cm3 d'éthanol à une température voisine de 60°C puis laissée au repos pendant 16 heures à une température voisine de 5°C. Les cristaux formés sont essorés, lavés avec 2 fois 2 cm3 d'éthanol et séchés à 40°C sous pression réduite (5 mm de Hg; 0,7 kPa). On obtient ainsi 5,5 g de fumarate acide de (diéthylamino-1 propyl-2)-10 phénothiazinecarbothioamide-2, série L, sous forme de cristaux jaunes fondant à 186°C.

$[\alpha]_D^{20}$ = +29,1 ±0,6° (1%; diméthylformamide).

Le [(diéthylamino-1 propyl-2)-10 phénothiazinecarbonitrile-2], série L, peut être préparé de la manière suivante :

A une solution de 7 g d'(éthylamino-1 propyl-2)-10 phénothiazinecarbonitrile-2, série L, dans 86 cm3 de diméthylformamide, on ajoute 3,2 g de carbonate de sodium et 2,3 cm3 d'iodoéthane puis on porte ce mélange à une température voisine de 150°C pendant 6 heures. Après refroidissement, le mélange réactionnel est concentré à sec sous pression réduite (30 mm de Hg; 4 kPa) à 40°C et le résidu est repris par 250 cm3 d'acétate d'éthyle. La solution obtenue est lavée successivement par 2 fois 100 cm3 d'eau distillée et par 100 cm3 d'une solution aqueuse saturée de chlorure de sodium, séchée sur sulfate de magnésium en présence de noir 3S et filtrée. Le filtrat jaune est concentré à sec sous pression réduite (30 mm de Hg; 4 kPa) à 40°C pour donner 6,65 g d'une huile orangée qui cristallise lentement. Ce résidu est dissous dans le minimum d'oxyde d'isopropyle au reflux, le léger insoluble est filtré à chaud et le filtrat est conservé pendant 3 jours à une température voisine de 5°C. Les cristaux formés sont essorés, lavés avec 2 fois 2 cm3 d'oxyde d'isopropyle et séchés à 40°C sous pression réduite (5 mm de Hg; 0,7 kPa). On obtient ainsi 2,9 g de (diéthylamino-1 propyl-2)-10 phénothiazinecarbonitrile-2, série L, sous forme de cristaux beiges fondant à 83°C ($[\alpha]_D^{20}$ = +9 ±0,3°; 0,978%; chloroforme). Le filtrat est alors concentré à sec sous pression réduite (30 mm de Hg; 4 kPa) à 40°C pour donner encore 2,3 g de (diéthylamino-1 propyl-2)-10 phénothiazinecarbonitrile-2, série L, sous forme d'un solide beige fondant à 81-82°C.

$[\alpha]_D^{20}$ = +8,7 ±0,3° (1,2%; chloroforme).

L'(éthylamino-1 propyl-2)-10 phénothiazinecarbonitrile-2, série L, peut être préparé de la manière suivante :

A une solution de 16 g de méthanesulfonate de (cyano-2 phénothiazinyl-10)-2 propyle-1, série L, dans 160 cm3 de toluène, on ajoute 30 cm3 d'éthylamine et on porte ce mélange à une température voisine de 105°C pendant 24 heures. Après refroidissement, le mélange réactionnel est concentré à sec sous pression réduite (30 mm de Hg; 4 kPa) à 40°C et le résidu est repris par 250 cm3 d'éther éthylique et 50 cm3 d'une solution aqueuse de soude N. Après agitation, la phase organique est séparée, lavée successivement par 50 cm3 d'eau distillée et par 50 cm3 d'une solution aqueuse saturée de chlorure de sodium, séchée sur sulfate de magnésium, filtrée et concentrée à sec sous pression réduite (30 mm de Hg; 4 kPa) pour donner 13,8 g d'une huile jaune. Ce résidu est dissous à une température voisine de 60°C dans 46 cm3 d'éthanol et cette solution est versée dans une solution à 60°C de 5,2 g d'acide fumarique dans 46 cm3 d'éthanol puis laissée pendant 16 heures à une température voisine de 5°C. Le précipité jaune formé est essoré, lavé par 2 fois 5 cm3 d'éthanol et séché à 40°C sous pression réduite (5 mm de Hg; 0,7 kPa). On obtient ainsi 9,7 g de fumarate d'(éthylamino-1 propyl-2)-10 phénothiazinecarbonitrile-2, série L, ($[\alpha]_D^{20}$ = +6,2 ±0,4°; 1,008%; diméthylformamide). Ce produit est mis en suspension dans 200 cm3 d'éther éthylique et on ajoute 100 cm3 d'une solution aqueuse de soude N. Après agitation, la phase organique est séparée et la phase aqueuse est extraite avec 50 cm3 d'éther éthylique. Les phases organiques sont réunies, lavées par 100 cm3 d'une solution aqueuse saturée de chlorure de sodium, séchées sur sulfate de magnésium, filtrées et concentrées à sec sous pression réduite (300 mm de Hg; 40 kPa puis 30 mm de Hg; 4 kPa) à 40°C. On obtient ainsi 7 g d'(éthylamino-1 propyl-2)-10 phénothiazinecarbonitrile-2, série L, sous forme d'une huile jaune.

$[\alpha]_D^{20}$ = +12 ±0,3° (2%; chloroforme).

## EXEMPLE 22

Une solution de 2 g de (diéthylamino-1 propyl-2)-10 N-(méthyl-3 butyl-1) phénothiazinecarbothioamide-2, série D, et de 0,52 g d'acide fumarique dans 20 cm3 d'éthanol est concentrée à sec sous pression réduite (30 mm de Hg; 4 kPa) à 40°C. Le résidu est repris sous agitation par 35 cm3 d'acide acétique puis additionné de 1,5 g d'acétate mercurique. L'agitation est poursuivie à une température voisine de 20°C pendant 16 heures. La suspension noire obtenue est diluée par 60 cm3 d'eau distillée et filtrée. Le filtrat orangé est additionné de lessive de soude (d = 1,33) jusqu'à pH 13 et extrait par 200 cm3 d'éther éthylique. La phase organique est lavée successivement par deux fois 50 cm3 d'eau distillée et par 50 cm3 d'une solution aqueuse saturée de chlorure de sodium et séchée sur sulfate de magnésium, en présence de noir 3S. Après filtration, le filtrat incolore est concentré à sec sous pression réduite (30 mm de Hg; 4 kPa) à 40°C; on obtient ainsi 2 g d'une huile jaune pâle. Ce produit est purifié par chromatographie sur une colonne (hauteur : 25 cm; diamètre : 2,6 cm) de gel de silice (0,04-0,063 mm) sous légère surpression d'azote (40 kPa) en éluant avec 1000 cm3 d'un mélange d'acétate d'éthyle et de méthanol (90-10 en volumes) et en recueillant des fractions de 50 cm3. Les fractions 3 à 9 sont réunies et concentrées à sec sous pression réduite (30 mm de Hg; 4 kPa) à 40°C. On obtient 1,5 g d'une huile jaune ($[\alpha]_D^{20}$ = -15 ±1°; 0,6%; chloroforme). Ce produit est dissous dans 100 cm3 d'oxyde d'isopropyle puis additionné sous agitation, à une température voisine de 5°C, de 5,8 cm3 d'une solution 0,45N d'acide chlorhydrique dans l'oxyde d'isopropyle. L'agitation est poursuivie pendant 30 minutes. Le précipité formé est essoré, lavé par 2 cm3 d'oxyde d'isopropyle et séché à 40°C sous pression réduite (5 mm de Hg;

0,7 kPa). On obtient ainsi 0,8 g de chlorhydrate de (diéthylamino-1 propyl-2)-10 N-(méthyl-3 butyl-1) phénothiazinecarboxamide-2, série D, sous forme d'un solide blanc fondant à 105-110°C (fusion pâteuse) dont le spectre de RMN est identique à celui du produit obtenu à l'exemple 21.

$[\alpha]_D^{20}$ = -14,6° ±0,5 (0,5%, diméthylformamide).

Spectre infra-rouge (KBr), bandes caractéristiques en $cm^{-1}$ :

3280, 3050, 2950, 2930, 2860, 2620, 2580, 2470, 1640, 1590, 1535, 1460, 1410, 1390, 1380, 1360, 1305, 1230, 870, 825, 750.

Le (diéthylamino-1 propyl-2)-10 N-(méthyl-3 butyl-1) phénothiazinecarbothioamide-2, série D, peut être préparé de la manière suivante :

A une solution de 3 g de fumarate acide de (diéthylamino-1 propyl-2)-10 phénothiazinecarbothioamide-2, série D, dans 45 cm3 d'éthanol absolu, on ajoute 10,7 cm3 de méthyl-3 butylamine et on sature cette solution avec de l'acide sulfhydrique. Le mélange est ensuite porté à une température voisine de 105°C pendant 16 heures et concentré à sec sous pression réduite (30 mm de Hg; 4 kPa) à 50°C. L'huile orangée résiduelle est purifiée par chromatographie sur une colonne (hauteur : 35 cm; diamètre : 3 cm) de gel de silice (0,2-0,063 mm) en éluant avec 400 cm3 d'un mélange d'acétate d'éthyle et de cyclohexane (75-25 en volumes) et en recueillant des fractions de 40 cm3. Les fractions 4 à 6 sont réunies et concentrées à sec sous pression réduite (30 mm de Hg; 4 kPa) à 50°C pour donner 3,1 g de (diéthylamino-1 propyl-2)-10 N-(méthyl-3 butyl-1) phénothiazinecarbothioamide-2, série D, sous forme d'une huile orangée.

Le fumarate acide de (diéthylamino-1 propyl-2)-10 phénothiazinecarbothioamide-2, série D, peut être obtenu de la manière suivante :

En opérant comme décrit à l'exemple 21 mais à partir de 3,4 g de (diéthylamino-1 propyl-2)-10 phénothiazinecarbonitrile-2, série D, et de 1,4 cm3 de triéthylamine dans 68 cm3 de pyridine anhydre, on obtient 4 g d'une huile orangée. Ce produit est dissous à une température voisine de 60°C dans 13 cm3 d'éthanol et cette solution est versée dans une solution de 1,16 g d'acide fumarique dans 13 cm3 d'éthanol à une température voisine de 5°C. Les cristaux formés sont essorés, lavés avec 2 fois 2 cm3 d'éthanol et séchés à 40°C sous pression réduite (5 mm de Hg; 0,7 kPa). On obtient ainsi 3,47 g de fumarate acide de (diéthylamino-1 propyl-2)-10 phénothiazinecarbothioamide-2, série D, sous forme d'un solide jaune fondant à 180°C.

$[\alpha]_D^{20}$ = -32,8 ±0,6° (0,9%; diméthylformamide).

Le (diéthylamino-1 propyl-2)-10 phénothiazinecarbonitrile-2, série D, peut être obtenu de la manière suivante :

On chauffe à une température voisine de 150°C pendant 6 heures un mélange de 4 g de fumarate acide d'(éthylamino-1 propyl-2)-10 phénothiazinecarbonitrile-2, série D, de 1,32 g de carbonate de sodium et de 1 cm3 d'iodoéthane dans 50 cm3 de diméthylformamide. Après refroidissement, on concentre à sec (30 mm de Hg; 4 kPa) à 50°C le mélange réactionnel et le résidu est repris par 100 cm3 d'acétate d'éthyle et lavé par successivement 50 cm3 d'eau distillée et par 50 cm3 d'une solution aqueuse saturée de chlorure de sodium. La phase organique est séchée sur sulfate de magnésium, filtrée et concentrée à sec sous pression réduite (30 mm de Hg; 4 kPa) à 40°C. On obtient ainsi 3,4 g de (diéthylamino-1 propyl-2)-10 phénothiazinecarbonitrile-2, série D, sous forme d'une huile jaune.

$[\alpha]_D^{20}$ = -6,8 ±0,4° (1,3%; chloroforme).

Le fumarate acide d'(éthylamino-1 propyl-2)-10 phénothiazinecarbonitrile-2, série D, peut être obtenu de la manière suivante :

A une solution de 10 g de méthanesulfonate de (cyano-2 phénothiazinyl-10)-2 propyle-1, série D, dans 100 cm3 de toluène, on ajoute 18,75 cm3 d'éthylamine et on porte ce mélange à une température voisine de 105°C pendant 24 heures. Après refroidissement, le mélange réactionnel est concentré à sec sous pression réduite (30 mm de Hg; 4 kPa) à 40°C. Le résidu est repris par 250 cm3 d'éther éthylique et extrait successivement par 2 fois 100 cm3 d'une solution aqueuse d'acide chlorhydrique N. Les phases aqueuses réunies sont alcalinisées par de la lessive de soude (d = 1,33) jusqu'à pH 13 et extraites par 2 fois 200 cm3 d'éther éthylique. Les phases organiques réunies sont lavées successivement par 50 cm3 d'eau distillée et par 50 cm3 d'une solution aqueuse saturée de chlorure de sodium, séchées sur sulfate de magnésium, filtrées et concentrés à sec sous pression réduite (30 mm de Hg; 4 kPa) à 40°C. On obtient ainsi 5 g d'(éthylamino-1 propyl-2)-10 phénothiazinecarbonitrile-2, série D, sous forme d'une huile orangée. Ce produit est dissous à une température voisine de 60°C dans 17 cm3 d'éthanol et cette solution est versée dans une solution de 1,9 g d'acide fumarique dans 17 cm3 d'éthanol et cettesolution est versée dans une solution de 1,9 g d'acide fumarique dans 17 cm3 d'éthanol à la même température puis on amorce la cristallisation et laisse pendant 16 heures à une température voisine de 20°C. Le solide est essoré, lavé par 2 fois 2 cm3 d'éthanol et séché sous pression réduite (5 mm de Hg; 0,7 kPa) à 40°C. On obtient ainsi 4 g de fumarate acide d'(éthylamino-1 propyl-2)-10 phénothiazinecarboni trile-2, série D, sous forme d'un solide jaune fondant à 208°C.

$[\alpha]_D^{20}$ = -5,2 ±0,5° (0,9%; diméthylformamide).

EXEMPLE 23

On dissout 0,3 g de N-(méthyl-3 butyl) [N'-éthyl N'-méthyl-amino-1 propyl-2-(2RS)]-10 phénothiazinecarbothioamide-2 dans une solution de 81 mg d'acide fumarique dans 5 cm3 d'éthanol absolu et on concentre à sec sous pression réduite (30 mm de Hg; 4 kPa) à 40°C. La meringue orangée résiduelle est dissoute dans 5 cm3 d'acide acétique et on ajoute 0,23 g d'acétate mercurique. La suspension orangée obtenue est agitée pendant 16 heures à une température voisine de 20°C. Le mélange réactionnel est concentré à sec sous pression réduite (30 mm de Hg; 4 kPa) à 60°C. Le résidu est repris par 10 cm3 d'eau distillée, filtré et le filtrat est alcalinisé avec de la lessive de soude (d = 1,33) jusqu'à pH 13 et extrait par 2 fois 10 cm3 d'acétate d'éthyle. Les phases organiques réunies sont lavées par 10 cm3 d'une solution aqueuse saturée de chlorure de sodium, séchées sur sulfate de magnésium, filtrées et concentrées à sec sous pression réduite (30 mm de Hg; 4 kPa) à 40°C. L'huile jaune résiduelle (0,15 g) est dissoute dans 2 cm3 d'acétate d'éthyle et on ajoute 20 cm3 d'oxyde d'isopropyle. La solution obtenue est refroidie à une température voisine de 5°C et on ajoute, goutte à goutte sous agitation, 5,1 cm3 d'une solution 0,065N d'acide chlorhydrique dans l'oxyde d'isopropyle. Le précipité formé est essoré, lavé par 3 fois 5 cm3 d'oxyde d'isopropyle et séché sous pression réduite (5 mm de Hg; 0,7 kPa) à 40°C. On obtient ainsi 0,13 g de chlorhydrate de N-(méthyl-3 butyl) [N'-éthyl N'-méthyl-amino-1 propyl-2-(2RS)]-10 phénothiazinecarboxamide-2 sous forme d'un solide blanc cassé fondant à 110-120°C (fusion pâteuse).

RMN du proton (250 MHz, DMSO, δ en ppm, J en Hz).

En solution dans le DMSO, on observe deux formes dues à la salification de l'azote.

0,92 (D, J = 7, 6H, $-CH(C\underline{H}_3)_2$); 1,06 et 1,20 (2T large, 3H, $>NCH_2C\underline{H}_3$); 1,45 (Mt, 2H, $-C\underline{H}_2CH<$); 1,63 (Mt, 1H, $>CH-$ du méthyl-3 butyle); 1,82 (D large, 3H, $-CH_3$); 2,77 (Mf, 3H, $>NCH_3$); 3,15 (Mf, 2H, $>NC\underline{H}_2-CH_3$); 3,29 (Mt, 2H, $-CONH-C\underline{H}_2-$); 3,48 et 3,75 (2Mf, 3,46 DD - J = 14 et 3 et 3,73 - DD - J = 14 et 8, 2H, $N-CH_2-$); 4,76 (Mf, 1H, $>N-CH<$); 7 à 7,4 (Mt, 5H, aromatiques); 7,53 (S, 1H, -H en 1); 7,54 (D, J = 8, 1H, -H en 3); 8,59 (Mf, J = 5,5, 1H, -CONH-); 10,20 (Mf, 1H, $-NH^+$).

Spectre infra-rouge (KBr), bandes caractéristiques en $cm^{-1}$ :

3270, 3060, 2950, 2930, 2870, 2580, 2480, 1645, 1590, 1540, 1460, 1415, 1385, 1365, 1310, 1230, 870, 845, 830, 755.

Le N-(méthyl-3 butyl) [N'-éthyl N'-méthyl-amino-1 propyl-2-(2RS)]-10 phénothiazinecarbothioamide-2 peut être préparé de la manière suivante :

Une solution de 1 g de [N-éthyl N-méthyl-amino-1 propyl-2-(2RS)]-10 phénothiazinecarbothioamide-2 et de 4,9 cm3 de méthyl-3 butylamine dans 15 cm3 d'éthanol absolu est saturée avec de l'acide sulfhydrique et chauffée pendant 16 heures à une température voisine de 100°C. Après refroidissement, la solution orangée obtenue est concentrée à sec sous pression réduite (30 mm de Hg; 4 kPa) à 40°C. L'huile orangée résiduelle est purifiée par chromatographie sur une colonne (hauteur : 25 cm, diamètre : 2 cm) de gel de silice (0,04-0,063 mm) sous une légère surpression d'azote (40 kPa) en éluant par 500 cm3 d'un mélange d'acétate d'éthyle et de cyclohexane (80-20 en volumes) et en recueillant des fractions de 30 cm3. Les fractions 4 à 8 sont réunies et concentrées à sec sous pression réduite (30 mm de Hg; 4 kPa) à 40°C. On obtient ainsi 1,3 g de N-(méthyl-3 butyl) [N'-éthyl N'-méthyl-amino-1 propyl-2 -(2RS)]-10 phénothiazinecarbothioamide-2 sous forme d'une huile jaune orangée.

EXEMPLE 24

A une suspension de 1 g de chlorhydrate de N-(méthyl-3 butyl) [(pyrolidinyl-1)-1 propyl-2-(2RS)]-10 phénothiazinecarbothioamide-2 dans 20 cm3 d'acide acétique, on ajoute 0,668 g d'acétate mercurique sous agitation et on laisse réagir 5 heures, 30 minutes à une température voisine de 20°C. Le mélange réactionnel est dilué avec 150 cm3 d'acétate d'éthyle et 50 cm3 d'eau distillée puis additionné de lessive de soude (d = 1,33) jusqu'à pH 13. La phase organique est séparée et la phase aqueuse est extraite à nouveau avec 2 fois 50 cm3 d'acétate d'éthyle. Les phases organiques réunies sont lavées avec 100 cm3 d'une solution aqueuse saturée d'hydrogénocarbonate de sodium puis avec 100 cm3 d'une solution aqueuse saturée de chlorure de sodium, séchées sur sulfate de magnésium, filtrées et concentrées à sec sous pression réduite (30 mm de Hg; 4 kPa) à 40°C. L'huile jaune résiduelle (0,53 g) est purifiée par chromatographie sur une colonne (hauteur : 30 cm; diamètre : 2,5 cm) de gel de silice (0,04-0,063 mm) sous une légère surpression d'azote (40 kPa) en éluant successivement par 100 cm3 d'un mélange de chlorure de méthylène et de méthanol (97,5-2,5 en volumes) puis par 200 cm3 d'un mélange de chlorure de méthylène et de méthanol (95-5 en volumes) et en recueillant

des fractions de 15 cm3. Les fractions 12 à 34 sont réunies et concentrées à sec sous pression réduite (30 mm de Hg; 4 kPa) à 40°C. L'huile obtenue est dissoute dans 10 cm3 d'acétate d'éthyle et on ajoute 0,5 cm3 d'une solution 3N d'acide chlorhydrique dans l'éther éthylique. Le solide formé est essoré, lavé par deux fois 2 cm3 d'éther éthylique et séché sous pression réduite (5 mm de Hg; 0,7 kPa) à 40°C. On obtient ainsi 0,37 g de chlorhydrate de N-(méthyl-3 butyl) [(pyrrolidinyl-1)-1 propyl-2-(2RS)]-10 phénothiazinecarboxamide-2 sous forme d'un solide jaune pâle fondant vers 100°C (fusion pâteuse) et dont le spectre de RMN est identique à celui du produit décrit ci-après à l'exemple 26.

Spectre infra-rouge (KBr), bandes caractéristiques en $cm^{-1}$ :

3280, 3060, 2960, 2930, 2875, 2680, 2610, 2480, 1660, 1650, 1595, 1540, 1460, 1415, 1385, 1365, 1310, 1235, 860, 830, 750.

Le chlorhydrate N-(méthyl-3 butyl) [(pyrrolidinyl-1)-1 propyl-2-(2RS)]-10 phénothiazinecarbothioamide-2 peut être préparé de la manière suivante :

On sature d'acide sulfhydrique une solution de 1,89 g de [(pyrrolidinyl-1)-1 propyl-2-(2RS)]-10 phénothiazinecarbothioamide-2 et de 8,9 cm3 de méthyl-3 butylamine dans 28 cm3 d'éthanol absolu et on chauffe ce mélange pendant 16 heures à une température voisine de 110°C. Après refroidissement, le mélange réactionnel est concentré à sec sous pression réduite (30 mm de Hg; 4 kPa) à 40°C et le résidu huileux jaune (2,8 g) est purifié par chromatographie sur une colonne (hauteur : 30 cm; diamètre : 2 cm) de gel de silice (0,04-0,063 mm) sous une légère surpression d'azote (40 kPa) en éluant successivement avec 100 cm3 de chlorure de méthylène puis 300 cm3 d'un mélange de chlorure de méthylène et de méthanol (95-5 en volumes) et en recueillant des fractions de 30 cm3. Les fractions 4 à 8 sont réunies et concentrées à sec sous pression réduite (30 mm de Hg; 4 kPa) à 40°C. Le résidu huileux jaune (2,1 g) est dissous dans 15 cm3 d'acétate d'éthyle et on ajoute 2 cm3 d'une solution 3N d'acide chlorhydrique dans l'éther éthylique. On maintient sous agitation pendant 1 heure à une température voisine de 5°C. Le précipité formé est essoré, lavé par 2 cm3 d'acétate d'éthyle et séché sous pression réduite (5 mm de Hg; 0,7 kPa) à 35°C. On obtient ainsi 2,1 g de chlorhydrate N-(méthyl-3 butyl) [(pyrrolidinyl-1)-1 propyl-2-(2RS)]-10 phénothiazinecarbothioamide-2 sous forme de cristaux jaunes fondant à 190°C.

EXEMPLE 25

A une solution de 4,57 g de N-(méthyl-3 butyl) [(pyrrolidinyl-1)-1 propyl-2]-10 phénothiazinecarbothioamide, série L, dans 100 cm3 d'éthanol absolu, on ajoute 1,5 g d'acide fumarique et on concentre à sec sous pression réduite (30 mm de Hg; 4 kPa) à 40°C. On reprend le résidu meringué jaune par 100 cm3 d'acide acétique, on ajoute sous agitation, 4,3 g d'acétate mercurique et on poursuit l'agitation pendant 16 heures à une température voisine de 20°C. La suspension noire obtenue est concentrée au quart de son volume sous pression réduite (30 mm de Hg; 4 kPa) à 50°C, reprise par 200 cm3 d'eau distillée et filtrée. Le filtrat est alcalinisé par de la lessive de soude (d = 1,33) jusqu'à pH 13 et extrait par deux fois 200 cm3 d'acétate d'éthyle. Les phases organiques réunies sont lavées successivement par 2 fois 100 cm3 d'eau distillée et par 100 cm3 d'une solution aqueuse saturée de chlorure de sodium, séchées sur sulfate de magnésium, filtrées et concentrées à sec sous pression réduite (30 mm de Hg; 4 kPa) à 40°C. On obtient ainsi 4,88 g de N-(méthyl-3 butyl) [(pyrrolidinyl-1)-1 propyl-2]-10 phénothiazinecarbothioamide-2, série L, sous forme d'une huile légèrement jaune. 3,3 g de cette huile sont dissous dans 25 cm3 d'acétate d'éthyle agités et additionnés de 2,4 cm3 d'une solution 3,3N d'acide chlorhydrique dans l'oxyde d'isopropyle puis agités à nouveau pendant 1 heure à une température voisine de 5°C. Le solide formé est essoré, lavé par 3 fois 10 cm3 d'éther éthylique et séché sous pression réduite (5 mm de Hg; 0,7 kPa) à 40°C. On obtient ainsi 2,55 g de chlorhydrate de N-(méthyl-3 butyl) [(pyrrolidinyl-1)-1 propyl-2]-10 phénothiazinecarboxamide-2, série L, sous forme d'un solide blanc fondant à 210°C et dont le spectre de RMN est identique à celui décrit à l'exemple 26.

$[\alpha]_D^{20}$ = +14 ±0,9° (0,5%; diméthylformamide).

Spectre infra-rouge (KBr), bandes caractéristiques en $cm^{-1}$ ;

3295, 3060, 2950, 2930, 2870, 2680, 2620, 2485, 1660, 1650, 1595, 1535, 1460, 1410, 1380, 1360, 1305, 1230, 855, 825, 750.

Le N-(méthyl-3 butyl) [(pyrrolidinyl-1)-1 propyl-2]-10 phénothiazinecarbothioamide-2, série L, peut être préparé de la manière suivante :

Une solution de 6 g de [(pyrrolidinyl-1)-1 propyl-2]-10 phénothiazinecarbothioamide-2, série L, et de 18,9 cm3 de méthyl-3 butylamine dans 90 cm3 d'éthanol absolu est saturée d'acide sulfhydrique, et portée pendant 16 heures à une température voisine de 105°C. Après refroidissement, le mélange réactionnel est concentré à sec sous pression réduite (30 mm de Hg; 4 kPa) à 40°C. Le résidu huileux brun est purifié par chromatographie sur une colonne (hauteur : 40 cm; diamètre : 4 cm) de gel de silice (0,02-0,063 mm) en éluant par 1 litre

de chlorure de méthylène puis par 1,5 litre d'un mélange de chlorure de méthylène et de méthanol (95-5 en volumes) et en recueillant des fractions de 80 cm3. Les fractions 23 à 36 sont réunies et concentrées à sec sous pression réduite (30 mm de Hg; 4 kPa) à 40°C. On obtient ainsi 6,76 g de N-(méthyl-3 butyl) [(pyrrolidinyl-1)-1 propyl-2]-10 phénothiazinecarbothioamide-2, série L, sous forme d'une huile orangée.

EXEMPLE 26

Une solution de 2 g de N-(méthyl-3 butyl) [(pyrrolidinyl-1)-1 propyl-2]-10 phénothiazinecarbothioamide-2, série D, et de 0,52 g d'acide fumarique dans 20 cm3 d'éthanol est concentrée à sec sous pression réduite (30 mm de Hg; 4 kPa) à 40°C. La meringue jaune résiduelle est reprise par 35 cm3 d'acide acétique puis additionnée de 1,5 g d'acétate mercurique et agitée à une température voisine de 20°C pendant 16 heures. La suspension noire obtenue est diluée par 50 cm3 d'eau distillée et filtrée. Le filtrat jaune est alcalinisé jusqu'à pH 13 par de la soude (d = 1,33) et extrait par 2 fois 100 cm3 d'acétate d'éthyle. Les phases organiques réunies sont lavées successivement par 2 fois 50 cm3 d'eau distillée et par 50 cm3 d'une solution aqueuse saturée de chlorure de sodium, séchées sur sulfate de magnésium en présence de noir 3S, filtrées et le filtrat incolore est concentré à sec sous pression réduite (30 mm de Hg; 4 kPa) à 50°C. On obtient ainsi 1 g de N-(méthyl-3 butyl) [(pyrrolidinyl-1)-1 propyl-2]-10 phénothiazinecarbothioamide-2, série D, sous forme d'une huile jaune. Ce produit est dissous dans 10 cm3 d'acétate d'éthyle et additionné de 0,7 cm3 d'une solution 3,3N d'acide chlorhydrique dans l'oxyde d'isopropyle. Le précipité formé est essoré, lavé par 3 fois 2 cm3 d'oxyde d'isopropyle et séché sous pression réduite (5 mm de Hg; 0,7 kPa) à 40°C. On obtient ainsi 0,75 g de chlorhydrate de N-(méthyl-3 butyl) [(pyrrolidinyl-1)-1 propyl-2]-10 phénothiazinecarboxamide-2, série D, sous forme d'un solide blanc fondant à 200°C.

$[\alpha]_D^{20}$ = -17,3 ±0,5° (1,1%; diméthylformamide).

RMN du proton (250 MHz, DMSO, δ en ppm, J en Hz).

0,92 (T, J = 7, 6H, -CH(C$\underline{H}_3$)$_2$); 1,43 (Q, J = 7, 2H, -NH-CH$_2$-C$\underline{H}_2$-); 1,62 (Mt, 1H, -CH- du méthyl-3 butyle); 1,78 (D, J = 7, 3H, -CH$_3$); 1,75 (Mt, 4H, -C$\underline{H}$-$_2$-C$\underline{H}_2$-); 2,82 - 3,10 - 3,55 et 3,75 (4Mf, 1H chacun,

$-CH_2-\underset{|}{N}-CH_2-$

de la pyrrolidine); 3,30 (Mt, 2H, -CONH-C$\underline{H}_2$); 3,75 (AB, 2H, >N-CH$_2$); 4,75 (Mt, 1H, >N-CH<); 7 à 7,4 (Mt, 5H, aromatiques); 7,53 (S, 1H, -H en 1); 7,55 (D, J = 8, 1H, -H en 3); 8,6 (T, J = 5, 1H, -CONH-); 10,58 (Mf, 1H, -NH$^+$).

Spectre infra-rouge (KBr), bandes caractéristiques en cm$^{-1}$ ;

3290, 3060, 2955, 2935, 2870, 2660, 2620, 2580, 1660, 1650, 1595, 1540, 1465, 1415, 1385, 1365, 1305, 1235, 860, 850, 830, 750.

Le N-(méthyl-3 butyl) [(pyrrolidinyl-1)-1 propyl-2]-10 phénothiazinecarbothioamide-2, série D, peut être préparé de la manière suivante :

A une solution de 4,1 g de [(pyrrolidinyl-1)-1 propyl-2]-10 phénothiazinecarbothiamide-2, série D, dans 61,5 cm3 d'éthanol, on ajoute 12,9 cm3 de méthyl-3 butylamine puis on sature d'acide sulfhydrique et on chauffe pendant 16 heures à une température voisine de 105°C. Après refroidissement, la solution est concentrée à sec sous pression réduite (30 mm de Hg; 4 kPa) à 40°C. On obtient une huile jaune qui est purifiée par chromatographie sur une colonne (hauteur : 30 cm; diamètre : 4 cm) de gel de silice (0,2-0,063 mm), en éluant par 1,5 litre d'un mélange de chlorure de méthylène et de méthanol (95-5 en volumes) et en recueillant des fractions de 80 cm3. Les fractions 14 à 17 sont réunies et concentrées à sec sous pression réduite (30 mm de Hg; 4 kPa) à 40°C. On obtient 4,7 g de N-(méthyl-3 butyl) [(pyrrolidinyl-1)-1 propyl-2]-10 phénothiazinecarbothioamide-2, série D, sous forme d'une huile orangée.

$[\alpha]_D^{20}$ = -3,2 ±0,2° (1,92%; chloroforme).

Le [(pyrrolidinyl-1)-1 propyl-2]-10 phénothiazinecarbothioamide-2, série D, peut être préparé de la manière suivante :

Un mélange de 11,7 g de [(pyrrolidinyl-1)-1 propyl-2]-10 phénothiazinecarbonitrile-2, série D, et de 4,9 cm3 de triéthylamine dans 234 cm3 de pyridine anhydre est saturé d'acide sulfhydrique pendant 1 heure à 25°C. On agite pendant 20 heures à 25°C puis le mélange réactionnel est dégazé par barbottage d'azote, dilué par 100 cm3 d'acétate d'éthyle, versé sur 1 litre d'eau distillée et extrait par 2 fois 500 cm3 d'acétate d'éthyle. Les phases organiques réunies sont lavées successivement par 2 fois 250 cm3 d'eau distillée et 250 cm3 d'une solution aqueuse saturée de chlorure de sodium, séchées sur sulfate de magnésium, filtrées et le filtrat jaune est concentré à sec sous pression réduite (30 mm de Hg; 4 kPa) à 40°C. On obtient 18 g d'une huile jaune qui est purifiée par chromatographie sur une colonne (hauteur : 45 cm; diamètre : 4 cm) de gel de silice (0,2-0,063 mm) en éluant par 1 litre de chlorure de méthylène puis par 5 litres d'un mélange de chlorure de méthylène

et de méthanol (95-5 en volumes) et en recueillant des fractions de 100 cm3. Les fractions 23 à 50 sont réunies et concentrées à sec sous pression réduite (30 mm de Hg; 4 kPa) à 40°C. On obtient ainsi 10,7 g de [(pyrrolidinyl-1)-1 propyl-2)-10 phénothiazinecarbothioamide-2, série D, sous forme d'une meringue jaune.

$[\alpha]_D^{20}$ = +40,7 ±0,6° (1,1%; chloroforme).

Le [(pyrrolidinyl-1)-1 propyl-2)-10 phénothiazinecarbonitrile-2, série D, peut être préparé de la manière suivante :

Un mélange de 25,5 g de méthanesulfonate de (cyano-2 phénothiazinyl-10)-2 propyle-1, série D, et de 29,6 cm3 de pyrrolidine dans 260 cm3 de toluène est chauffé pendant 52 heures à une température voisine de 90°C. Le mélange réactionnel est concentré à sec sous pression réduite (30 mm de Hg; 4 kPa) à 40°C. Le résidu est repris par 500 cm3 d'éther éthylique et extrait par 2 fois 100 cm3 d'une solution aqueuse 2N d'acide méthanesulfonique. La phase aqueuse est alcalinisée par de la lessive de soude (d = 1,33) jusqu'à pH 13 à une température voisine de 5°C et extraite successivement par 100 cm3 d'eau distillée et par 100 cm3 d'une solution aqueuse saturée de chlorure de sodium, séchées sur sulfate de magnésium en présence de noir 3S, filtrées et le filtrat jaune est concentré à sec sous pression réduite (30 mm de Hg; 4 kPa) à 40°C. Les 18,2 g d'huile orangée ainsi obtenus sont chromatographiés sur une colonne (hauteur : 45 cm; diamètre : 4 cm) de gel de silice (0,063-0,2 mm) en éluant par 2,5 litres d'un mélange de chlorure de méthylène et de méthanol (97,5-2,5 en volumes) et en recueillant des fractions de 150 cm3. Les fractions 8 à 16 sont réunies et concentrés à sec sous pression réduite (30 mm de Hg; 4 kPa) à 40°C. On obtient ainsi 11,7 g de [(pyrrolidinyl-1)-1 propyl-2]-10 phénothiazinecarbonitrile-2, série D, sous forme d'une huile jaune.

$[\alpha]_D^{20}$ = -9,8 ±0,4° (1,1%; chloroforme).

Le méthanesulfonate de (cyano-2 phénothiazinyl-10)-2 propyle-1, série D, peut être préparé de la manière suivante :

A une solution de 20 g d'(hydroxy-1 propyl-2)-10 phénothiazinecarbonitrile, série D, dans 200 cm3 de chlorure de méthylène refroidie à une température voisine de 5°C, on ajoute, sous agitation, 16,2 cm3 de triéthylamine puis on verse, goutte à goutte pendant 25 minutes, une solution de 8,9 cm3 de chlorure de méthanesulfonyle dans 89 cm3 de chlorure de méthylène et poursuit l'agitation pendant 50 minutes à une température voisine de 10°C. Le mélange réactionnel est lavé successivement par 2 fois 100 cm3 d'eau distillée et par 100 cm3 d'une solution aqueuse saturée de chlorure de sodium, séché sur sulfate de magnésium, filtré et concentré à sec sous pression réduite (30 mm de Hg; 4 kPa) à 40°C. On obtient ainsi 25,8 g de méthanesulfonate de (cyano-2 phénothiazinyl-10)-2 propyle-1, série D, sous forme d'une gomme orangée qui est utilisée sans autre purification pour la suite des synthèses.

$[\alpha]_D^{20}$ = -23,1 ±0,4° (1,4%; chloroforme).

L'(hydroxy-1 propyl-2)-10 phénothiazinecarbonitrile-2, série D, peut être préparé de la manière suivante :

A une solution de 23,5 g de potasse dans 1150 cm3 d'éthanol au reflux, on ajoute 95,4 g de phtalate de (-)-(cyano-2 phénothiazinyl-10)-2 propyle et de phényl-1 éthylammonium-(S) et poursuit le reflux sous agitation pendant 10 minutes. Le mélange réactionnel est alors jeté sur 1 litre d'eau glacée et extrait par 2 litres puis 500 cm3 d'acétate d'éthyle. Les phases organiques réunies sont lavées successivement par deux fois 500 cm3 d'une solution aqueuse d'acide chlorhydrique 0,1N, par 2 fois 500 cm3 d'une solution aqueuse saturée d'hydrogénocarbonate de sodium et par 500 cm3 d'une solution aqueuse saturée de chlorure de sodium, séchées sur sulfate de magnésium, filtrées et concentrées à sec sous pression réduite (30 mm de Hg; 4 kPa) à 40°C. Le solide jaune résiduel (44 g) est repris par 200 cm3 d'oxyde d'isopropyle au reflux et le produit cristallise à chaud. On laisse revenir à une température voisine de 20°C et on essore le solide, le lave par 20 cm3 d'oxyde d'isopropyle et le sèche sous pression réduite (5 mm de Hg; 0,7 kPa) à 20°C. On obtient ainsi 36,5 g d'(hydroxy-1 propyl-2)-10 phénothiazinecarbonitrile-2, série D, sous forme de cristaux jaunes fondant à 135°C.

$[\alpha]_D^{20}$ = +13,1 ±0,5° (1,0%; chloroforme).

Le phtalate de (-)-(cyano-2 phénothiazinyl-10)-2 propyle et de phényl-1 éthylammonium-(S) peut être obtenu comme décrit à l'exemple 5 pour la préparation du phtalate de (cyano-2 phénothiazinyl-10)-2 propyle-1, série L, et de phényl-1 éthylamonium-(1R), mais en reprenant le solide conservé à l'issu de la filtration. Le solide est dissous dans 600 cm3 d'acétate d'éthyle au reflux. Après refroidissement, le solide formé est essoré, lavé par 50 cm3 d'acétate d'éthyle et séché sous pression réduite (30 mm de Hg; 4 kPa) à 40°C. On obtient ainsi 44,2 g de phtalate de (-)-(cyano-2 phénothiazinyl-10)-2 propyle et de phényl-1 éthylammonium-(1S) sous forme de cristaux jaunes clairs fondant à 154°C.

$[\alpha]_D^{20}$ = -21,5° ±0,6° (1%; chloroforme).

EXEMPLE 27

A une solution de 2,32 g de N-(méthyl-3 butyl) [pipéridino-1 propyl-2-(2RS)]-10 phénothiazinecarbothioamide-2 dans 20 cm3 d'acide acétique glacial, on ajoute goutte une solution de 1,63 g d'acétate mercurique dans 20 cm3 d'acide acétique pendant une période de 10 minutes. Le mélange réactionnel est agité 60 minutes à 25°C puis filtré sur verre fritté garni de célite. La célite est lavée par 2 fois 10 cm3 d'acétate d'éthyle et les filtrats réunis sont concentrés à sec sous pression réduite (30 mm de Hg; 4 kPa) pour donner un résidu qui est dilué dans 100 cm3 d'acétate d'éthyle. La phase organique est lavée par 2 fois 50 cm3 de soude normale, 2 fois 50 cm3 d'eau distillée puis par une fois 50 cm3 de saumure, séchée sur sulfate de magnésium, filtrée et concentrée à sec sous pression réduite (30 mm de Hg; 4 kPa) à 50°C pour donner un résidu qui est purifié par chromatographie sur une colonne (hauteur : 36 cm; diamètre : 2 cm) de gel de silice (0,06-0,2 mm) en éluant avec des mélanges de cyclohexane et d'acétate d'éthyle 80-20 (500 cm3) 50-50 (600 cm3)) (en volumes) en recueillant des fractions de 50 cm3. Les fractions 8 à 20 sont réunies et concentrées à sec sous pression réduite (30 mm de Hg; 4 kPa) à 50°C pour donner 0,95 g de N-(méthyl-3 butyl) [pipéridino-1 propyl-2-(2RS)]-10 phénothiazinecarboxamide-2.

A une solution de 0,80 g de N-(méthyl-3 butyl) [pipéridino-1 propyl-2-(2RS)]-10 phénothiazinecarboxamide-2 dans 40 cm3 d'éther éthylique, on ajoute 0,83 cm3 d'une solution d'éther chorhydrique 2,2 N. La suspension est agitée pendant 12 heures à 25°C. Le précipité est filtré sur verre fritté, lavé par 2 fois 10 cm3 d'éther éthylique et séché à 40°C sous pression réduite (5 mm de Hg; 0,7 kPa) pour donner 0,67 g de chlorhydrate de N-(méthyl-3 butyl) [pipéridino-1 propyl-2-(2RS)]-10 phénothiazinecarboxamide-2 fondant à 228°C.

RMN du proton (250 MHz, DMSO, δ en ppm, J en Hz).

0,9 (D, J = 7, 6H, $-CH(C\underline{H}_3)_2$); 1,42 (Mt, 2H, $-NHCH_2C\underline{H}_2-$); 1,6 (Mt, 1H, >CH- du méthyl-3 butyle); 1,2 à 1,9 (Mt, 6H, $-CH_2-C\underline{H}_2-$de la pipéridine); 1,8 (D, J = 7, 3H, $-CH_3$); 2,7 à 3,8 (Mt, 8H,

$$-C\underline{H}_2-\underset{|}{N}-C\underline{H}_2-,$$

>N-$CH_2$-, $-CONHC\underline{H}_2-$); 4,8 (Mt, 1H, >N-CH<); 7 à 7,35 (Mt, 5H, aromatiques); 7,5 (S, 1H, -H en 1); 7,52 (D, J = 8, 1H, -H en 3); 8,55 (T, J = 5,5, 1H, -CONH-); 9,95 (Mf, 1H, $-NH^+$).

Spectre infra-rouge (KBr), bandes caractéristiques en $cm^{-1}$ :

3285, 3060, 2950, 2870, 2660, 2540, 1660, 1595, 1540, 1460, 1410, 1385, 1365, 1305, 1235, 860, 825, 745.

Le N-(méthyl-3 butyl) [pipéridino-1 propyl-2-(2RS)]-10 phénothiazinecarbothioamide-2 peut être obtenu de la manière suivante :

A une solution de 3,07 g de [pipéridino-1 propyl-2 -(2RS)]-10 phénothiazinecarbothioamide-2 dans 65 cm3 d'éthanol absolu, on ajoute 4,65 cm3 de méthyl-3 butylamine. Le mélange est porté à 150°C pendant 16 heures. Le mélange réactionnel est concentré à sec sous pression réduite (30 mm de Hg; 4 kPa) à 50°C. Le résidu est dilué par 150 cm3 d'acétate d'éthyle, la solution obtenue est lavée par 3 fois 50 cm3 d'eau distillée, séchée sur sulfate de magnésium puis concentrée à sec sous pression réduite (30 mm de Hg; 4 kPa), le résidu est purifié par chromatographie sur une colonne (hauteur : 23,2 cm; diamètre : 3,6 cm) de gel de silice (0,04-0,06 mm) avec une légère surpression d'azote (40 kPa) en éluant avec des mélanges de cyclohexane et d'acétate d'éthyle 80-20 (1 litre) 50-50 (2 litres) (en volumes) en recueillant des fractions de 60 cm3. Les fractions 5 à 13 sont réunies et concentrés à sec à 50°C sous pression réduite (30 mm de Hg; 4 kPa) pour donner 2,49 g de N-(méthyl-3 butyl) [pipéridino-1 propyl-2 -(2RS)]-10 phénothiazinecarbothioamide-2.

Le [pipéridino-1 propyl-2-(2RS)]-10 phénothiazinecarbothioamide-2 peut être obtenu de la manière suivante :

Un mélange de 8,74 g de [pipéridino-1 propyl-2-(2RS)]-10 phénothiazinecarbonitrile-2 et de 3,5 cm3 de triéthylamine dans 100 cm3 de pyridine anhydre est saturé par bullage d'acide sulfhydrique pendant 3 heures à 25°C. La solution limpide obtenue est maintenue sous agitation pendant 12 heures à 25°C, puis le mélange est dégazé par bullage d'azote pendant 2 heures. Le mélange réactionnel est dilué par 500 cm3 d'acétate d'éthyle et lavé par 10 fois 200 cm3 d'eau distillée. La phase organique est séchée sur sulfate de magnésium, filtrée et le filtrat est concentré à sec sous pression réduite (30 mm de Hg; 4 kPa). Le résidu est purifié par chromatographie sur colonne (hauteur : 54 cm; diamètre: 3,6 cm) de gel de silice (0,06-0,2 mm) en éluant par un mélange de cyclohexane et d'acétate d'éthyle 50-50 (1,25 litres) (en volumes) puis par de l'acétate d'éthyle pur (1,25 litres) et en recueillant des fractions de 125 cm3. Les fractions 4 à 18 sont réunies et concentrées à sec sous pression réduite (30 mm de Hg ; 4 kPa) à 50°C pour donner 9,36 g de [pipéridino-1 propyl-2-(2RS)]-10 phénothiazinecarbothioamide-2.

RMN du proton (250 MHz, DMSO, δ en ppm, J en Hz).

1,38 (Mt, 2H, $-CH_2-C\underline{H}_2-CH_2-$); 4,23 (Mt, J = 7 - 6,5 et 6, 1H, >N-CH<); 6,9 à 7,25 (Mt, 5H, aromatiques); 7,43 (D large, J = 8, 1H, -H en 3); 7,79 (S large,1H, -H en 1); 9,5 et 9,9 (2S, 1H chacun, $-CSNH_2$).

Le [pipéridino-1 propyl-2-(2RS)]-10 phénothiazinecarbonitrile-2 peut être obtenu de la manière suivante :

A une suspension de 36,05 g de méthanesulfonate de (cyano-2 phénothiazinyl-10)-2 propyle-1 dans 360

cm3 de xylène, on ajoute 19,8 cm3 de pipéridine. Le mélange est porté au reflux pendant 19 heures. Après refroidissement, le mélange est lavé par 6 fois 150 cm3 d'eau distillée. La phase organique est séchée sur sulfate de magnésium, filtrée et concentrée à sec à 50°C sous pression réduite (30 mm de Hg; 4 kPa). Le résidu est purifié par chromatographie sur colonne (hauteur : 96 cm; diamètre : 4,8 cm) de gel de silice (0,06-0,2 mm) en éluant par des mélanges de cyclohexane et d'acétate d'éthyle 90-10 (4 litres), 85-15 (4 litres), 80-20 (4 litres) et 75-25 (4 litres) (en volumes) en recueillant des fractions de 500 cm3. Les 9 premiers litres sont éliminés et les fractions 8 à 13 sont réunies et concentrées à sec à 50°C sous pression réduite (30 mm de Hg; 4 kPa) pour donner 13,6 g de [pipéridino-1 propyl-2-(2RS)]-10 phénothiazinecarbonitrile-2.

RMN du proton (250 MHz, DMSO, δ en ppm, J en Hz).

1,4 (Mt, 2H, $-CH_2-C\underline{H}_2-CH_2-$); 4,19 (Mt, J = 7 - 6,5 et 6, 1H, >N-C$\underline{H}$<); 6,9 à 7,35 (Mt, 5H, aromatiques); 7,39 (DD, J = 8 et 1, 1H, -H en 3); 7,79 (D, J = 1, 1H, -H en 1).

EXEMPLE 28

A une solution de 1,2 g de chlorhydrate de N-[méthyl-3 pentyl-(3RS)] [(pyrrolidinyl-1)-1 propyl-2-(2RS)]-10 phénothiazinecarbothioamide-2 dans 20,5 cm3 d'acide acétique, on ajoute 0,88 g d'acétate mercurique et on agite pendant 16 heures à une température voisine de 20°C. La suspension orangée obtenue est diluée par 50 cm3 d'eau distillée, filtrée et le filtrat jaune est concentré à sec sous pression réduite (30 mm de Hg; 4 kPa) à 40°C. Le résidu est repris par 100 cm3 d'acétate d'éthyle et 50 cm3 d'eau distillée. On ajoute de la lessive de soude (d = 1,33) jusqu'à pH 13. La phase organique est lavée successivement par 2 fois 25 cm3 d'eau distillée et par 25 cm3 d'une solution aqueuse saturée de chlorure de sodium et séchées sur sulfate de magnésium. Après filtration, le filtrat jaune est concentré à sec sous pression réduite (30 mm de Hg; 4 kPa) à 40°C et on obtient ainsi 0,8 g d'une meringue jaune. Ce produit est dissous dans 10 cm3 d'acétate d'éthyle, puis on ajoute 0,5 cm3 d'une solution 3,3N d'acide chlorhydrique dans l'oxyde d'isopropyle. Le précipité formé est séparé par filtration, lavé par 3 fois 2 cm3 d'oxyde d'isopropyle et séché sous pression réduite (5 mm de Hg; 0,7 kPa). On obtient ainsi 0,26 g de chlorhydrate de N-[méthyl-3 pentyl-(3RS)] [(pyrrolidinyl-1)-1 propyl-2 -(2RS)]-10 phénothiazinecarboxamide-2 fondant à 198°C.

RMN du proton (250 MHz, $CDCl_3$, δ en ppm, J en Hz).

0,91 (T, J = 7, 3H, $-CH_2-C\underline{H}_3$); 0,97 (D, J = 7, 3H, >CH-C$\underline{H}_3$ du méthyl-3 pentyle); 1,2 et 1,45 (2Mt, de 1H chacun, $-C\underline{H}_2CH_3$); 1,48 (Mt, 2H, $-NHCH_2C\underline{H}_2-$); 1,69 (Mt, 1H, >CH- du méthyl-3 pentyle); 1,89 (D, J = 7, 3H, $-CH_3$); 2,08 (Mt, 4H, $-C\underline{H}_2-C\underline{H}_2-$ de la pyrrolidine); 2,9 et 3,6 à 4 (2Mf, de 2H chacun,

$$-C\underline{H}_2-\underset{|}{N}-C\underline{H}_2-$$

de la pyrrolidine); 3,45 (Mt, 2H, $-CONH-C\underline{H}_2-$); 3,45 (D large, J = 14, 1H, 1H du >N-$CH_2$-); 3,72 (DD, J = 14 et 8, 1H, 1H du >$NCH_2$-); 5,25 (Mt, 1H, >N-CH<); 6,92 (T, J = 5,5, 1H, -CONH-); 7 à 7,25 (Mt, 5H, aromatiques); 7,37 (D large, J = 8, 1H, -H en 3); 7,5 (D, J = 1, 1H, -H en 1); 12,4 (Mf étalé, 1H, $-NH^+$).

Spectre infra-rouge (KBr), bandes caractéristiques en $cm^{-1}$ :

3280, 3060, 2960, 2930, 2885, 2760, 2610, 2480, 1660, 1595, 1530, 1460, 1415, 1380, 1300, 1235, 860, 850, 830, 750.

Le chlorhydrate de N-[méthyl-3 pentyl-1-(3RS)] [(pyrrolidinyl-1)-1 propyl-2-(2RS)]-10 phénothiazinecarbothioamide-2 peut être préparé de la manière suivante :

Un mélange de 3,7 g de [(pyrrolidinyl-1)-1 propyl-2-(2RS)]-10 phénothiazinecarbothioamide-2, de 3,3 g de chlorhydrate de [méthyl-3 pentyl-1-(3RS)] amine et de 3,4 cm3 de triéthylamine dans 55 cm3 d'éthanol est saturé d'acide sulfhydrique puis chauffé pendant 16 heures à 105°C. Après refroidissement, la solution est concentrée à sec sous pression réduite (30 mm de Hg; 4 kPa) à 40°C. On obtient 5,6 g d'une huile orangée qui est purifiée par chromatographie sous surpression d'azote (40 kPa) sur colonne (hauteur : 25 cm; diamètre : 4 cm) de gel de silice (0,063-0,2 mm) en éluant par 1 litre d'un mélange de chlorure de méthylène et de méthanol (95-5 en volumes) et en recueillant des fractions de 60 cm3. Les fractions 8 à 9 sont réunies et concentrées à sec sous pression réduite (30 mm de Hg; 4 kPa) à 40°C. On obtient 2,3 g d'une huile orangée. Ce produit est dissous dans un mélange de 10 cm3 d'acétate d'éthyle et de 100 cm3 d'oxyde d'isopropyle puis on ajoute 1,5 cm3 d'une solution 3,3N d'acide chlorhydrique dans l'oxyde d'isopropyle. Le précipité formé est essoré, lavé par 3 fois 10 cm3 d'oxyde d'isopropyle et séché sous pression réduite (5 mm de Hg; 0,7 kPa) à 40°C. On obtient ainsi 1,7 g de chlorhydrate de N-[méthyl-3 pentyl-1-(3RS) [(pyrrolidinyl-1)-1 propyl-2-(2RS)]-10 phénothiazinecarbothioamide-2 sous forme d'un solide jaune fondant à 135-140°C (fusion pâteuse).

RMN du proton (250 MHz, DMSO, δ en ppm, J en Hz).

0,88 (T, J = 7, 3H, $-CH_2-C\underline{H}_3$); 0,93 (D, J = 7, 3H, CH-C$\underline{H}_3$ du méthyl-3 pentyle); 1,2 et 1,4 (2Mt, 1H chacun,

-C$H_2$$CH_3$); 1,49 (Mt, 2H, -NHC$H_2$C$H_2$-); 1,72 (Mt, 1H, > CH- du méthyl-3 pentyle); 3,50 à 3,90 (Mt, 4H, N-$CH_2$ et -CSNH-C$H_2$-); 7 à 7,35 (Mt, 5H, aromatiques); 7,41 (D large, J = 8, 1H, -H en 3); 7,56 (S large, 1H, -H en 1); 10,4 (T, J = 5, 1H, -CSNH-).

Le [(pyrrolidinyl-1)-1 propyl-2-(2RS)]-10 phénothiazinecarbothioamide-2 peut être obtenu de la manière suivante :

Une solution de 5,3 g de [(pyrrolidinyl-1)-1 propyl 2-(2RS)]-10 phénothiazinecarbonitrile-2 et 2,2 cm3 de triéthylamine dans 106 cm3 de pyridine anhydre est saturée d'acide sulfhydrique et laissée sous agitation pendant 16 heures à une température voisine de 20°C. Le mélange réactionnel est versé sur 500 cm3 d'eau distillée et extrait successivement par 500 cm3 puis deux fois 250 cm3 d'acétate d'éthyle. Les phases organiques réunies sont lavées par 3 fois 200 cm3 d'eau distillée, séchées sur sulfate de magnésium, filtrées et concentrées à sec sous pression réduite (30 mm de Hg; 4 kPa) à 40°C. Les 6,5 g de l'huile orangée obtenue sont chromatographiés sur une colonne (hauteur : 30 cm; diamètre : 4 cm) de gel de silice (0,2-0,063 mm) sous surpression d'azote (40 kPa) en éluant par 1 litre d'un mélange de chlorure de méthylène et de méthanol (95-5 en volumes) puis par 1 litre d'un mélange de chlorure de méthylène et de méthanol (90-10 en volumes) et en recueillant des fractions de 100 cm3. Les fractions 8 à 16 sont réunies et concentrées à sec sous pression réduite (30 mm de Hg; 4 kPa) à 40°C. On obtient ainsi 5,1 g de [(pyrrolidinyl-1)-1 propyl-2-(2RS)]-10 phénothiazinecarbothioamide-2 sous forme d'un solide jaune. On dissout, à une température voisine de 60°C, 1,4 g de ce produit dans 25 cm3 d'isopropanol. Après refroidissement, on essore les cristaux formés, les lave par 5 cm3 d'isopropanol froid et sèche sous pression réduite (5 mm de Hg; 0,7 kPa) à 30°C. On obtient ainsi 1,1 g de [(pyrrolidinyl-1)-1 propyl-2-(2RS)]-10 phénothiazinecarbothioamide-2 sous forme de cristaux jaunes fondant à 150°C.

Le [(pyrrolidinyl-1)-1 propyl-2-(2RS)]-10 phénothiazinecarbonitrile-2 peut être obtenu de la manière suivante :

Un mélange de 10 g de méthanesulfonate de (cyano-2 phénothiazinyl-10)-2 propyle-1-(2RS) et de 11,6 cm3 de pyrrolidine dans 50 cm3 de toluène est porté à 90°C sous agitation pendant 24 heures. Après refroidissement, on concentre à sec sous pression réduite (30 mm de Hg; 4 kPa) à 40°C et on reprend le résidu avec 200 cm3 d'éther éthylique et 15 cm3 d'une solution aqueuse de soude 4N. Après avoir agité pendant 10 minutes, on décante et lave la phase organique avec 3 fois 25 cm3 d'une solution aqueuse saturée de chlorure de sodium, sèche sur sulfate de magnésium, filtre et concentre à sec sous pression réduite (30 mm de Hg; 4 kPa) à 20°C. Les 11,3 g d'huile résiduelle sont dissous dans 60 cm3 d'une solution aqueuse d'acide chlorhydrique 0,5N. Cette solution est lavée avec 100 cm3 d'étheréthylique puis alcalinisée avec un excès d'une solution aqueuse de soude N et extraite par 100 cm3 d'éther éthylique. La phase organique est lavée avec 50 cm3 d'une solution aqueuse saturée de chlorure de sodium, séchée sur sulfate de magnésium, filtrée et concentrée à sec sous pression réduite (30 mm de Hg; 4 kPa) à 20°C. L'huile jaune résiduelle (9,5 g) est purifiée sur une colonne (hauteur ; 30 cm; diamètre ! 5,8 cm) de gel de silice (0,04-0,063 mm) sous légère surpression d'azote (40 kPa) en éluant avec un litre d'un mélange de chlorure de méthylène et de méthanol (95-5 en volumes) et en recueillant des fractions de 100 cm3. Les fractions 3 à 10 sont réunies et concentrées à sec sous pression réduite (30 mm de Hg; 4 kPa) à 40°C. On obtient ainsi 5,45 g de [(pyrrolidinyl-1)-1 propyl-2-(2RS)]-10 phénothiazinecarbonitrile-2 sous forme d'une huile jaune.

Le méthanesulfonate de (cyano-2 phénothiazinyl-10)-2 propyle-1-(2RS) peut être obtenu de la manière suivante :

Sur une solution de 120,5 g d'[hydroxy-1 propyl-2-(2RS)]-10 phénothiazinecarbonitrile-2 dans 1280 cm3 de chlorure de méthylène refroidie à une température voisine de 5°C, on verse, sous agitation, 100 cm3 de triéthylamine puis, en 30 minutes, 55,9 cm3 de chlorure de méthanesulfonyle et on poursuit l'agitation pendant 15 minutes en maintenant la température vers 10-15°C. Le mélange réactionnel est dilué avec 500 cm3 d'eau distillée à 5°C et la phase organique est séparée, lavée avec 500 cm3 d'une solution aqueuse saturée de chlorure de sodium, séchée sur sulfate de sodium, filtrée et concentrée à sec sous pression réduite (30 mm de Hg; 4 kPa) à 40°C. L'huile résiduelle (164 g) est purifiée par chromatographie sur une colonne (hauteur : 54 cm; diamètre : 8,5 cm) de gel de silice (0,2-0,063 mm) en éluant par 4,4 litres de chlorure de méthylène puis par 7 litres d'un mélange de chlorure de méthylène et de méthanol (99-1 en volumes) et en recueillant des fractions de 1 litre. Les fractions 3 à 11 sont réunies et concentrées à sec sous pression réduite (30 mm de Hg; 4 kPa) à 40°C. On obtient ainsi 153,5 g d'une huile jaune que l'on reprend par 400 cm3 d'oxyde d'isopropyle au reflux. Par refroidissement, un produit cristallise et on poursuit l'agitation pendant 1 heure à une température voisine de 5°C. Le solide formé est essoré, lavé par 2 fois 50 cm3 d'oxyde d'isopropyle glacé et séché à 30°C sous pression réduite (30 mm de Hg; 0,4 kPa). On obtient ainsi 131,6 g de méthanesulfonate de (cyano-2 phénothiazinyl-10)-2 propyle-1-(2RS) sous forme de cristaux jaune clair fondant à 124°C.

L'[hydroxy-1 propyl-2-(2RS)]-10 phénothiazinecarbonitrile-2 peut être préparé de la manière suivante :

Dans une suspension de 52 g de borohydrure de sodium dans 1,4 litre de tétrahydrofuranne on verse, sous

agitation en 15 minutes et à une température voisine de 20°C, 113 cm3 d'éthanedithiol-1,2 puis, en 15 minutes dans les mêmes conditions, une solution de 296 g de (cyano-2 phénothiazinyl-10)-2 propionate d'éthyle-(2RS) dans 1,4 litre de tétrahydrofuranne. A la fin de l'addition, on chauffe le mélange réactionnel pendant 20 heures à une température voisine de 60°C. Après refroidissement à une température voisine de 5°C, on verse 1 litre d'une solution aqueuse de soude 4N pendant 1 heure : un vif dégagement gazeux est observé. Le mélange réactionnel est ensuite versé dans un mélange de 1 litre d'une solution aqueuse de soude 4N et de 3 litres de chlorure de méthylène sous agitation. On isole la phase organique et on extrait à nouveau la phase aqueuse avec 1 litre de chlorure de méthylène. Les phases organiques réunies sont lavées par 2 fois 1 litre d'une solution aqueuse saturée de chlorure de sodium, séchées sur sulfate de magnésium, filtrées et concentrées à sec sous pression réduite (30 mm de Hg; 4 kPa) à 30°C. L'huile visqueuse orangée (290 g) est purifiée sur sune colonne (hauteur : 50 cm; diamètre : 8,5 cm) de gel de silice (0,2-0,063 mm) en éluant successivement avec 3 litres de chlorure de méthylène, puis par 4 litres d'un mélange de chlorure de méthylène et de méthanol (97,5-2,5 en volumes) et par 10 litres d'un mélange de chlorure de méthylène et de méthanol (95-5 en volumes) et en recueillant des fractions de 1 litre. Les fractions 3 à 15 sont réunies et concentrées à sec sous pression réduite (30 mm de Hg; 4 kPa) à 30°C. On obtient ainsi 169,7 g d'[(hydroxy-1 propyl-2-(2RS)]-10 phénothiazinecarbonitrile-2 sous forme d'un solide jaune fondant à 123°C.

Le (cyano-2 phénothiazinyl-10)-2 propionate d'éthyle-(2RS) peut être obtenu de la manière suivante :

Dans une suspension de 24 g d'hydrure de sodium dans 1 litre de diméthylformamide à une température voisine de 25°C, on verse, sous agitation et en 2 heures 30 minutes, une solution de phénothiazinecarbonitrile-2 (224,5 g) dans 1 litre de diméthylformamide puis on laisse encore agiter 1 heure 15 minutes jusqu'à la fin du dégagement gazeux. La suspension fine obtenue est versée sous agitation et à une température voisine de 25°C, en 4 heures 30 minutes dans une solution de 255 cm3 de chloro-2 propionate d'éthyle-(2RS) dans 1 litre de diméthylformamide et on poursuit l'agitation pendant 16 heures. On verse alors, dans le mélange réactionnel, 100 cm3 d'éthanol, puis on verse le tout sur un mélange de 2 kg de glace dans 4 litres d'eau distillée : une gomme précipite puis cristallise. On essore le solide formé, le lave successivement avec 6 fois 500 cm3 d'eau distillée et 2 fois 500 cm3 d'éther de pétrole et le sèche à l'air. On obtient ainsi 296,5 g de (cyano-2 phénothiazinyl-10)-2 propionate d'éthyle-(2RS) sous la forme de cristaux kaki fondant à 117-118°C, utilisés tels quels à l'étape suivante.

## EXEMPLE 29

Un mélange de 1,2 g de chlorhydrate de N-(méthyl-4 pentyl) [(pyrrolidinyl-1)-1 propyl-2-(2RS)]-10 phénothiazinecarbothioamide-2 et de 0,88 g d'acétate mercurique dans 20,5 cm3 d'acide acétique est agité pendant 16 heures à une température voisine de 20°C. La suspension orangée obtenue est diluée avec 50 cm3 d'eau distillée, filtrée et concentrée à sec sous pression réduite (30 mm de Hg; 4 kPa) à 40°C. Le résidu est repris par 50 cm3 d'eau distillée et 100 cm3 d'acétate d'éthyle. On alcalinise jusqu'à pH 13 avec de la soude (d = 1,33). La phase organique est séparée puis lavée successivement avec 2 fois 25 cm3 d'eau distillée et 25 cm3 d'une solution aqueuse saturée de chlorure de sodium. La phase organique est ensuite séchée sur sulfate de magnésium, filtrée et concentrée à sec sous pression réduite (30 mm de Hg; 4 kPa) à 40°C. Le résidu huileux jaune (0,82 g) est dissous dans 10 cm3 d'acétate d'éthyle et additionné, sous agitation, 0,55 cm3 d'une solution 3,3N d'acide chlorhydrique dans l'oxyde d'isopropyle. Après 1 heure sous agitation à une température voisine de 5°C, le solide formé est essoré, lavé par 3 fois 10 cm3 d'éther éthylique et séché sous pression réduite (5 mm de Hg; 0,7 kPa) à 40°C. On obtient ainsi 0,6 g de chlorhydrate de N-(méthyl-4 pentyl) [(pyrrolidinyl-1)-1 propyl-2-(2RS)]-10 phénothiazinecarboxamide-2 sous forme d'un solide jaune très pâle fondant à 218°C.

RMN du proton (200 MHz, DMSO, $\delta$ en ppm, J en Hz).

0,9 (D, J = 7, 6H, $-CH(C\underline{H}_3)_2$); 1,24 (Mt, 2H, $-C\underline{H}_2CH(CH_3)_2$); 1,6 (Mt, 3H, $-CH_2-$ et $>$CH- du méthyl-4 pentyle); 1,8 (D, J = 7, 3H, $-CH_3$); 1,75 à 2 (Mt, 4H, $-C\underline{H}_2C\underline{H}_2-$ de la pyrrolidine); 2,88 - 3,13 - 3,60 et 3,75 (4Mf, de 1H chacun,

$$-CH_2-\underset{|}{N}-CH_2-$$

de la pyrrolidine); 3,26 (Mt, 2H, $-CONHC\underline{H}_2-$); 3,75 (Mt, 2H, $>NCH_2-$); 4,78 (Mt, 1H, $>N-CH<$); 7 à 7,4 (Mt, 5H, aromatiques); 7,55 (S, 1H, -H en 1); 7,57 (D, J = 8, 1H, -H en 3); 8,68 (T, J = 5,5, 1H, -CONH-); 10,75 (Mf étalé, 1H, $-NH^+$).

Spectre infra-rouge (KBr), bandes caractéristiques en $cm^{-1}$ :

3285, 3060, 2955, 2930, 2870, 2680, 2620, 2485, 1650, 1595, 1540, 1460, 1415, 1385, 1365, 1235, 865,

830, 750.

Le chlorhydrate de N-(méthyl-4 pentyl) [(pyrrolidinyl-1)-1 propyl-2-(2RS)]-10 phénothiazinecarbothioamide-2 peut être préparé de la manière suivante :

Une solution de 2 g de [(pyrrolidinyl-1)-1 propyl-2-(2RS)]-10 phénothiazinecarbothioamide-2 et de 7,4 g de chlorhydrate de méthyl-4 pentylamine dans 30 cm3 d'éthanol absolu est saturée d'acide sulfhydrique. Le mélange réactionnel est ensuite porté pendant 16 heures à une température voisine de 105°C. Après refroidissement, on concentre à sec sous pression réduite (30 mm de Hg; 4 kPa) à 40°C. Le résidu pâteux (5 g) est purifié par chromatographie sur une colonne (hauteur : 35 cm; diamètre : 3 cm) de gel de silice (0,2-0,063 mm) en éluant successivement par 500 cm3 de chlorure de méthylène puis par 500 cm3 d'un mélange de chlorure de méthylène et de méthanol (95-5 en volumes) et en recueillant des fractions de 30 cm3. Les fractions 25 à 28 sont réunies et concentrées à sec sous pression réduite (30 mm de Hg; 4 kPa) à 40°C. L'huile orangée résiduelle (2,16 g) est dissoute dans 10 cm3 d'acétate d'éthyle et on ajoute sous agitation 1,5 cm3 d'une solution 3,3N d'acide chlorhydrique dans l'oxyde d'isopropyle puis de l'éther éthylique jusqu'à formation d'un trouble persistant. Après 1 heure à une température voisine de 5°C, le solide formé est essoré, lavé par 3 fois 10 cm3 d'éther éthylique et séché sous pression réduite (5 mm de Hg; 0,7 kPa) à 40°C. On obtient ainsi 1,7 g de chlorhydrate N-(méthyl-4 pentyl) [(pyrrolidinyl-1)-1 propyl-2-(2RS)]-10 phénothiazinecarbothioamide-2 sous forme d'un solide jaune fondant à 186°C.

RMN du proton (250 MHz, DMSO, δ en ppm, J en Hz).

0,90 (D, J = 7, 6H, $-CH(C\underline{H}_3)_2$); 1,26 (Mt, 2H, $-C\underline{H}_2-CH(CH_3)_2$); 1,58 (Mt, 1H, >CH- du méthyl-4 pentyle); 1,7 (Mt, 2H, $-NHCH_2-C\underline{H}_2-$); 3,72 (Mt, 2H, $-CSNHC\underline{H}_2-$); 7 à 7,35 (Mt, 5H, aromatiques); 7,45 (D large, J = 8, 1H, -H en 3); 7,55 (S large, 1H, -H en 1); 10,52 (T, J = 5, 1H, -CSNH-).

EXEMPLE 30

Une suspension de 2,45 g de chlorhydrate de N-(diméthyl-3,3 butyl) [(pyrrolidinyl-1)-1 propyl-2-(2RS)]-10 phénothiazinecarbothioamide-2 et de 1,6 g d'acétate mercurique dans 49 cm3 d'acide acétique est agitée pendant 16 heures à une température voisine de 20°C puis reprise par 50 cm3 d'eau distillée et filtrée. Le filtrat est concentré à sec sous pression réduite (30 mm de Hg; 4 kPa) à 40°C. Le résidu est repris par 100 cm3 d'acétate d'éthyle et alcalinisé par une solution aqueuse de soude N. La phase organique est séparée, lavée par 50 cm3 d'une solution aqueuse saturée de chlorure de sodium, séchée sur sulfate de magnésium, filtrée et concentrée à sec sous pression réduite (30 mm de Hg; 4 kPa) à 40°C. La meringue jaune résiduelle (1,6 g) est purifiée par chromatographie sur une colonne (hauteur : 40 cm; diamètre : 1,8 cm) de gel de silice (0,2-0,063 mm) en éluant avec 100 cm3 de chlorure de méthylène puis avec 500 cm3 d'un mélange de chlorure de méthylène et de méthanol (93-7 en volumes) et en recueillant des fractions de 50 cm3. Les fractions 3 à 1 sont réunies et concentrées à sec sous pression réduite (30 mm de Hg; 4 kPa) à 40°C. Le résidu (1,75 g) est dissous dans 10 cm3 d'acétate d'éthyle et additionné de 1,5 cm3 d'une solution 3N d'acide chlorhydrique dans l'éther éthylique. Le solide formé est essoré (1,5 g) et repris par 20 cm3 d'eau distillée et 20 cm3 d'acétate d'éthyle et alcalinisé par une solution aqueuse 0,1N de soude. La phase organique est séparée, lavée par 10 cm3 d'eau distillée, séchée sur sulfate de magnésium, filtrée et concentrée à sec sous pression réduite (30 mm de Hg; 4 kPa) à 40°C. Le résidu (1,35 g) est purifié par chromatographie sur une colonne (hauteur : 40 cm; diamètre : 1,8 cm) de gel de silice (0,2-0,063 mm) en éluant par 700 cm3 d'acétate d'éthyle et en recueillant des fractions de 50 cm3. Les fractions 4 à 10 sont réunies et concentrées à sec sous pression réduite (30 mm de Hg; 4 kPa) à 40°C. On obtient 1,31 g de solide blanc résiduel. On reprend 1,05 g de ce résidu par 20 cm3 d'oxyde d'isopropyle au reflux sous agitation pendant 10 minutes et on laisse refroidir sous agitation. Le solide est essoré, lavé par 5 cm3 d'oxyde d'isopropyle glacé et séché sous pression réduite (5 mm de Hg; 0,7 kPa) à 40°C. On obtient ainsi 0,68 g de N-(diméthyl-3,3 butyl) [(pyrrolidinyl-1)-1 propyl-2 -(2RS)]-10 phénothiazinecarboxamide-2 sous forme de cristaux blancs fondant à 138°C.

RMN du proton (250 MHz, DMSO, δ en ppm, J en Hz).

0,98 (S, 9H, $-C(CH_3)_3$); 1,47 (Mt, 2H, $-C\underline{H}_2-C(CH_3)_3$); 1,61 (D, J = 7, 3H, $-CH_3$); 1,7 (Mt, 4H, $-C\underline{H}_2-C\underline{H}_2-$ de la pyrrolidine); 2,55 (Mt masqué, 4H,

$$-CH_2-N-CH_2-$$

de la pyrrolidine); 2,91 (DD, J = 14 et 7,5, 1H, 1H du $>NCH_2-$); 3 (DD, J =14 et 5,5, 1H, 1H du $>N-CH_2-$); 3,28 (Mt, 2H, $-CONHC\underline{H}_2-$); 4,2 (Mt, J = 7,5 - 7 et 5,5, 1H, $>N-CH<$); 6,9 à 7,3 (Mt, 5H, aromatiques); 7,4 (D, J = 8, 1H -H en 3); 7,55 (S, 1H, -H en 1); 8,4 (T, J = 5,5, 1H, -CONH-).

Spectre infra-rouge (KBr), bandes caractéristiques en $cm^{-1}$ :

3305, 3060, 2950, 2900, 2860, 2790, 1630, 1590, 1580, 1540, 1460, 1410, 1390, 1360, 1320, 1230, 875, 845, 825, 745.

Le chlorhydrate de N-(diméthyl-3,3 butyl) phénothiazinecarbothioamide-2 peut être préparé de la manière suivante :

Un mélange de 3 g de [(pyrrolidinyl-1)-1 propyl-2-(2RS)]-10 phénothiazinecarbothioamide-2 et de 5,7 g de diméthyl-3,3 butylamine dans 30 cm3 d'éthanol absolu est porté à une température voisine de 100°C pendant 16 heures. Après refroidissement on concentre à sec sous pression réduite (30 mm de Hg; 4 kPa) à 40°C. Le résidu est dissous dans 100 cm3 d'acétate d'éthyle et cette solution est lavée par 3 fois 20 cm3 d'eau distillée, séchée sur sulfate de magnésium en présence de noir 3S et concentrée à sec sous pression réduite (30 mm de Hg; 4 kPa) à 40°C. Le résidu (5 g) est purifié par chromatographie sur une colonne (hauteur : 30 cm; diamètre : 5 cm) de gel de silice (0,04-0,063 mm) en éluant par 2,5 litres d'un mélange de chlorure de méthylène et de méthanol (97-3 en volumes) et en recueillant des fractions de 100 cm3. Les fractions 18 à 24 sont réunies et concentrées à sec sous pression réduite (30 mm de Hg; 4 kPa) à 40°C. Le résidu (3,68 g) est dissous dans 35 cm3 d'acétate d'éthyle. Cette solution est filtrée et on ajoute 3,5 cm3 d'une solution 3N d'acide chlorhydrique dans l'éther éthylique. On laisse reposer 2 heures à une température voisine de 5°C et le solide formé est essoré, lavé par 5 cm3 d'éther éthylique et séché sous pression réduite (5 mm de Hg; 0,7 kPa) à 35°C. On obtient ainsi 3,2 g de chlorhydrate de N-(diméthyl-3,3 butyl) [(pyrrolidinyl-1)-1 propyl-2-(2RS)]-10 phénothiazinecarbothioamide-2 sous forme de cristaux jaunes fondant à 199-200°C.

RMN du proton (250 MHz, DMSO, δ en ppm, J en Hz).

0,97 (S, 9H, $-C(CH_3)_3$); 1,61 (Mt, 2H, $-C\underline{H_2}-C(C\underline{H_3})_3$); 1,78 (D, J = 7, 3H, $-CH_3$); 3,5 à 3,9 (Mt, 4H, $N-CH_2$ et $-CSNHC\underline{H_2}-$); 7 à 7,35 (Mt, 5H, aromatiques); 7,42 (D, J = 8, 1H, -H en 3); 7,53 (S, 1H, -H en 1); 10,45 (T, J = 5, 1H, -CSNH-).

EXEMPLE 31

Un mélange de 2,4 g de [(N-méthyl N-éthyl-amino)-1 propyl-2-(2RS)]-10 phénothiazinecarbothioamide-2 et de 8,27 cm3 de propylamine dans 48 cm3 d'éthanol est chauffé pendant 16 heures à une température voisine de 100°C. Après refroidissement, on concentre à sec sous pression réduite (30 mm de Hg; 4 kPa) à 40°C. L'huile brune résiduelle (3 g) est purifiée par chromatographie sur une colonne (hauteur : 25 cm; diamètre : 4 cm) de gel de silice (0,04-0,063 mm) avec une légère surpression d'azote (40 kPa) en éluant par 2 litres d'un mélange de chlorure de méthylène et de méthanol (95-5 en volumes) et en recueillant des fractions de 60 cm3. Les fractions 8 à 20 sont réunies et concentrées à sec sous pression réduite (30 mm de Hg; 4 kPa) à 40°C. L'huile résiduelle (1,68 g) est dissoute dans 50 cm3 d'éther éthylique en présence de noir 3S. On filtre et le filtrat jaune est additionné, sous agitation de 1,3 cm3 d'une solution d'acide chlorhydrique 3,3N dans l'éther éthylique et on poursuit l'agitation pendant 2 heures à une température voisine de 20°C. Le précipité formé est essoré, lavé par 5 cm3 d'éther éthylique et séché sous pression réduite (5 mm de Hg; 0,68 kPa) à 35°C. On obtient ainsi 1,35 g de chlorhydrate de [(N-méthyl N-éthyl-amino)-1 propyl-2-(2RS)]-10 N-propyl phénothiazinecarbothioamide-2 sous forme d'un solide jaune fondant 110 - 115°C (fusion pâteuse).

RMN du proton (250 MHz, DMSO, δ en ppm, J en Hz).

En solution dans le DMSO, on observe deux formes dues à la salification de l'azote, ce phénomène disparaît par ajout de $CD_3COOD$.

0,97 (T, J = 7,5, 3H, $-(CH_2)_2C\underline{H_3}$); 1,06 et 1,20 (2T, J = 7,5, 3H, $>NCH_2C\underline{H_3}$); 1,74 (Mt, 2H, $-CH_2-C\underline{H_2}-CH_3$); 1,82 (Mt, 3H, $-CH_3$); 2,8 (Mt, 3H, $>N-CH_3$); 3,18 (Mt, 2H, $>N-C\underline{H_2}CH_3$); 3,54 et 3,80 (2Mt, 1H chacun, $>N-CH_2-$); 3,7 (Mt, 2H, $-CSNH-C\underline{H_2}-$); 7,05 à 7,4 (Mt, 5H, aromatiques); 7,45 (D large, J = 8, 1H, H en 3); 7,6 (S large, 1H, -H en 1); 10,31 et 10,52 (2Mt, 1H chacun, $-NH^+$ et -CSNH-).

Spectre infra-rouge (KBr), bandes caractéristiques en $cm^{-1}$ :

3210, 2970, 2940, 2880, 2650, 2480, 1590, 1535, 1465, 880, 820, 750.

Une solution de 1,1 g de [N'-éthyl N'-méthyl amino-1 propyl-2 -(2RS)]-10 N-propyl phénothiazinecarbothioamide-2 et de 0,32 g d'acide fumarique dans 20 $cm^3$ de méthanol est concentrée à sec sous pression réduite (309 mm de Hg; 4 kPa) à 40°C. La meringue orangée résiduelle est reprise par 22 cm3 d'acide acétique puis additionnée de 0,9 g d'acétate mercurique et agitée à une température voisine de 20°C pendant 16 heures. La suspension obtenue est diluée par 25 cm3 d'eau distillée et filtrée. Le filtrat jaune est concentré à sec sous pression réduite (30 mm de Hg; 4kPa) à 40°C.

Le résidu est repris par 50 cm3 d'acétate d'éthyle et 25 $cm^3$ d'eau, alcalinisé par de la soude. La phase organique est séparée et la phase aqueuse est extraite par 25 $cm^3$ d'acétate d'éthyle. Les phases organiques sont réunies, lavées successivement par 2 fois 20 $cm^3$ d'eau distillée puis par 20 cm3 d'une solution aqueuse saturée de chlorure de sodium, séchées sur sulfate de magnésium, filtrées et concentrées à sec sous pression

réduite (30 mm de Hg; 4 kPa) à 40°C. Le résidu huileux jaune (1,05 g) est dissous dans 35 cm3 d'un mélange d'acétate d'éthyle et d'oxyde d'isopropyle (15-85 en volumes) et on ajoute goutte à goutte, à une température de 5°C, 6 $cm^3$ d'une solution 0,4N d'acide chlorhydrique dans l'oxyde d'isopropyle. Un solide précipite. La suspension ainsi obtenue est agitée pendant 1 heure à 0°C puis filtrée. Le solide est lavé par 3 fois 5 cm3 d'oxyde d'isopropyle. Le solide est séché sous pression réduite (1 mm de Hg; 0,13 kPa) à 50°C pour donner 0,3 g de [N-éthyl N-méthyl amino-1 propyl-2-(2RS)]-10 N-propyl phénothiazinecarboxamide-2 sous la forme d'un solide blanc fondant à 100°C.

RMN du proton (250 MHz, DMSO D6 + quelques gouttes de $CD_3COOD$ $D_4$, δ en ppm, J en Hz).

0,9 (T, J = 7,5, 3H, $-(CH_2)_2C\underline{H_3}$) ; 1,09 (T, J = 7,5, 3H, >$NCH_2C\underline{H_3}$) ; 1,8 (D, J = 7, 3H, $-CH_3$) ; 2,75 (S, 3H, >$N-CH_3$) ; 3,13 Q, J = 7,5, 2H, $N-CH_2-CH_3$) ; 3,24 (Mt, 2H, $-CONH-C\underline{H_2}-$) ; 3,42 (DD, J = 14 et 4, 1H, 1H du >$N-CH_2-$) ; 3,73 (DD, J = 14 et 8, H, l'autre H du >$N-CH_2-$) ; 4,69 (Mt, J = 8-7 et 4, 1H, >N-CH<) ; 7 à 7,35 (Mt, 5 H, aromatiques) ; 7,53 (s, 1H, - H en 1) ; 7,54 (D, J = 8, 1H, H en 3); 8,55 (T résiduel, J = 5, -CONH-).

Spectre infra-rouge (KBr), bandes caractéristiques en $cm^{-1}$ :

3270, 2960, 2930, 2875, 2580, 2470, 1645, 1590, 1460, 1535, 875, 830, 755.

## EXEMPLE 32

On ajoute, en deux minutes, sous agitation et à une température de 20°C, 0,16 cm3 d'eau distillée dans une suspension de 1,68 g de tertiobutylate de potassium dans 20 cm3 de tertiobutanol. Dans la solution obtenue, on ajoute sous agitation et à une température de 20°C une solution de 1,68 g de (diéthylamino-1 propyl-2)-10 phénothiazinecarbonitrile-2, série L, dans 10 cm3 de tertiobutanol. La suspension jaune obtenue est alors portée au reflux pendant 1 heure. Après refroidissement à une température de 30°C, on ajoute sous agitation 1,5 cm3 d'iodo-1 propane et on porte au reflux pendant 4 heures. Après refroidissement, on concentre à sec sous pression réduite (30 mm de Hg; 4 kPa) à 40°C. Le résidu est repris avec 100 cm3 d'éther éthylique et la phase organique est lavée avec 20 cm3 d'eau distillée, séchée sur sulfate de magnésium, filtrée et concentrée à sec sous pression réduite (30 mm de Hg; 4kPa) à 40°C. Le résidu huileux jaune (2,3 g) est purifié par chromatographie sur colonne (hauteur: 20 cm; diamètre: 3 cm) de gel de silice (0,04-0,063 mm) sous légère surpression d'azote (40 kPa) en éluant successivement avec 500 cm3 de chlorure de méthylène et avec 500 $cm^3$ d'un mélange de chlorure de méthylène et de méthanol (97-3 en volumes) et en recueillant des fractions de 30 $cm^3$. Les fractions 8 à 25 sont réunies et concentrées à sec sous pression réduite (30 mm de Hg; 4 kPa) à 40°C pour donner 1,15 g d'un résidu huileux. Ce résidu est dissous dans 30 $cm^3$ d'acétate d'éthyle et on ajoute 3 $cm^3$ d'une solution 3,3 N d'une solution d'acide chlorhydrique dans l'éther éthylique. Après 2 heures d'agitation à 20°C, le précipité formé est filtré, lavé avec 4 fois 5 $cm^3$ d'acétate d'éthyle et séché sous pression réduite (5 mm de Hg; 0,67 kPa) à 40°C pour donner 1,23 g de chlorhydrate de (diéthylamino-1 propyl-2)-10 N-propyl phénothiazinecarboxamide-2, série L, sous forme d'un solide blanc fondant à 125-130°C.

RMN du proton (250 MHz, DMSO $D_6$, δ en ppm, J en Hz).

0,91 (T, J = 7, 3H, $-(CH_2)_2C\underline{H_3}$); 1 et 1,12 (2T, J = 7, respectivement 3H chacun $-N(CH_2C\underline{H_3})_2$; 1,86 (D, J = 7, 3H, $-CH_3$); 3,05 à 3,3 (Mt, 6H, $-CONH-C\underline{H_2}-$ et $-N(CH_2CH_3)_2$); 3,4 et 3,7 (2 Mt, le premier masqué en partie, l'autre H, >$N-CH_2-$); 4,84 (Mt, 1H, >N-CH<) ; 7 à 7,4 (Mt, 5H, aromatiques); 7,53 (s, 1H, H en 1); 7,55 (D, J = 8, 1H, -H en 3); 8,65 (T, J = 5,5, 1H, -CONH-); 10,28 (Mf, 1H, $-NH^+Cl^-$).

Spectre infra-rouge (KBr), bandes caractéristiques en $cm^{-1}$ :

3250, 2960, 2930, 2870, 2580, 2480, 1645, 1590, 1460, 1540, 875, 830, 750.

## EXEMPLE 33

Dans une solution de 1,7 g de tertiobutylate de potassium dans 12 cm3 de tertiobutanol, on verse 0,28 cm3 d'eau distillée, on ajoute 1,1 g de [N-éthyl N-propyl-amino-1 propyl-2-(2RS)]-10 phénothiazinecarbonitrile-2 et on porte à reflux pendant 30 minutes sous agitation. On ajoute alors 1,5 cm3 d'iodo-1 propane et on poursuit le reflux sous agitation pendant 3 heures 50 minutes. Après refroidissement, le mélange réactionnel est concentré à sec sous pression réduite (30 mm de Hg; 4 kPa) à 40°C. Le résidu est repris par 25 cm3 d'acétate d'éthyle et la solution obtenue est lavée successivement par 10 cm3 d'eau distillée puis par 10 cm3 d'une solution aqueuse saturée de chlorure de sodium, séchée sur sulfate de magnésium en présence de noir 3S, filtrée et concentrée à sec sous pression réduite (30 mm de Hg; 4 kPa) à 40°C pour donner 0,9 g d'une huile jaune. Ce produit est purifié par chromatographie sur colonne (hauteur : 25 cm; diamètre : 3 cm) de gel de silice (0,063-0,04 mm) sous une légère surpression d'azote (40 kPa) en éluant successivement par 500 cm3 de chlorure de méthylène puis par 1 litre d'un mélange de chlorure de méthylène et de méthanol (98-2 en volumes) et enfin par 500 cm3 d'un mélange de chlorure de méthylène et de méthanol (96-4 en volumes) et en recueillant des fractions de 60 cm3. Les fractions 10 à 30 sont réunies et concentrées à sec sous pression réduite (30

mm de Hg; 4 kPa) à 40°C.

On obtient ainsi 0,72 g d'une huile jaune que l'on dissout dans 45 cm3 d'un mélange d'oxyde d'isopropyle et d'acétate d'éthyle (89-11 en volumes) et on ajoute, goutte à goutte et sous agitation à une température voisine de 5°C, 5,57 cm3 d'une solution 0,34N d'acide chlorhydrique dans l'oxyde d'isopropyle. Le précipité formé est essoré, lavé par 10 cm3 d'oxyde d'isopropyle et séché sous pression réduite (5 mm de Hg; 0,7 kPa) à 40°C. On obtient ainsi 0,6 g de chlorhydrate de [N'-éthyl N'-propyl-amino-1 propyl-2-(2RS)]-10 N-propyl phénothiazinecarboxamide-2 sous forme d'un solide blanc fondant à 95-100°C (fusion pâteuse).

RMN du proton (250 MHz, DMSO, δ en ppm et J en Hz).

En solution dans le DMSO, on observe deux produits dus à la salification de l'azote.

0,73 et 0,84 (2T, J = 7, 3H, >$N(CH_2)_2C\underline{H_3}$); 0,9 (T, J = 7,5, 3H, $-NH(CH_2)_2C\underline{H_3}$); 1 et 1,19 (2T, J = 7, 3H, >$NCH_2C\underline{H_3}$); 1,56 (Mt, 4H, $-CH_2-C\underline{H_2}-CH_3$ des 2 propyles); 1,85 (D, J = 7, 3H, $-CH_3$); 3 et 3,15 (2Mf, environ 4H, >$N-CH_2-$ de l'éthyle et du propyle du N-éthyl N-propylamino); 3,23 (Mt, 2H, $-CONH-C\underline{H_2}-$); environ 3,4 et 3,75 (2Mf, de 1H chacun, >$N-CH_2-$); 4,85 (Mt, 1H, >N-CH<); 7 à 7,4 (Mt, 5H, aromatiques); 7,54 (S large, 1H, -H en 1); 7,55 (D large, J = 8, 1H, -H en 3); 8,65 (T, J = 5,5, 1H, -CONH-); 10,15 et 10,25 (2Mf, 1H en totalité, $-NH^+$).

Spectre infra-rouge (KBr), bandes caractéristiques en $cm^{-1}$ :

3270, 3060, 2960, 2930, 2875, 2580, 2490, 1645, 1590, 1555, 1535, 1460, 1415, 1395, 1380, 1310, 1230, 870, 830, 750.

Le [N-éthyl N-propyl-amino-1 propyl-2-(2RS)]-10 phénothiazinecarbonitrile-2 peut être préparé de la manière suivante :

Un mélange de 1,5 g d'[éthylamino-1 propyl-2-(2RS)]-10 phénothiazinecarbonitrile-2, de 0,5 cm3 d'iodo-1 propane et de 0,8 g de carbonate de sodium dans 20 cm3 de diméthylformamide sec est porté à une température voisine de 150°C pendant 6 heures. Après refroidissement, le mélange réactionnel est concentré à sec sous pression réduite (5 mm de Hg; 0,7 kPa) à 50°C. Le résidu est repris par 50 cm3 d'acétate d'éthyle et la solution obtenue est lavée successivement par 2 fois 50 cm3 d'eau distillée et par 25 cm3 d'une solution aqueuse saturée de chlorure de sodium. La phase organique est séparée, séchée sur sulfate de magnésium en présence de noir 3S, filtrée et concentrée à sec sous pression réduite (30 mm de Hg; 4 kPa) à 40°C. On obtient 1,5 g d'une huile orangée que l'on purifie par chromatographie sur colonne (hauteur : 25 cm; diamètre : 4 cm) de gel de silice (0,063-0,04 mm) sous légère surpression d'azote (300 mm de Hg; 40 kPa) en éluant par 1,5 litre d'un mélange de chlorure de méthylène et de méthanol (97,5-2,5 en volumes) et en recueillant des fractions de 60 cm3. Les fractions 19 à 22 sont réunies et concentrées à sec sous pression réduite (30 mm de Hg; 4 kPa) à 40°C. On obtient ainsi 1,18 g de [N-éthyl N-propyl-amino-1 propyl-2-(2RS)]-10 phénothiazinecarbonitrile-2 sous forme d'une huile jaune.

L'[éthylamino-1 propyl-2-(2RS)]-10 phénothiazinecarbonitrile-2 peut être préparé comme décrit pour la préparation des matières premières décrites aux exemples 8 et 28.

## EXEMPLE 34

A une solution de 11,2 g de tertiobutylate de potassium dans un mélange de 120 cm3 de tertiobutanol et de 1,8 cm3 d'eau distillée, on ajoute une solution de 6,18 g de [diméthylamino-1 propyl-2-(2RS)]-10 phénothiazinecarbonitrile-2 dans 40 cm3 de tertiobutanol. Le mélange est porté pendant 2 heures et 30 minutes au reflux après avoir ajouté 13,2 cm3 d'iodo-1 méthyl-3 butane. Après refroidissement, on dilue par 300 cm3 d'acétate d'éthyle et la phase organique est lavée par 3 fois 500 cm3 d'eau distillée, 100 cm3 d'une solution saturée de chlorure de sodium puis séchée sur sulfate de magnésium. Le filtrat est concentré à sec à 50°C sous pression réduite (30 mm de Hg; 4 kPa) pour donner un résidu qui est purifié par chromatographie sur colonne (hauteur : 40 cm; diamètre : 2,5 cm) de silice (0,06-0,2 mm) en éluant avec des mélanges d'acétate d'éthyle et de cyclohexane 70-30 (300 cm3), 50-50 (300 cm3), 20-80 (500 cm3) (en volumes) puis par de l'acétate pur (1 litre) tout en recueillant des fractions de 60 cm3. Les fractions 29 à 34 sont réunies et concentrées à sec à 50°C sous pression réduite (30 mm de Hg; 4 kPa) pour donner 1,5 g de [diméthylamino-1 propyl-2-(2RS)]-10 N-(méthyl-3 butyl) phénothiazinecarboxamide-2 sous la forme d'un solide jaune pâle.

On agite 1,5 g de [diméthylamino-1 propyl-2-(2RS)]-10 N-(méthyl-3 butyl) phénothiazinecarboxamide-2 dans 30 cm3 d'oxyde d'isopropyle pendant 15 minutes. Le solide est filtré sur verre fritté puis séché à 40°C sous pression réduite (30 mm de Hg ; 4 kPa) pour donner 1,2 g de [diméthylamino-1 propyl-2-(2RS)]-10 N-(méthyl-3 butyl) phénothiazinecarboxamide-2 sous la forme d'un solide amorphe jaune pâle fondant à 105°C.

RMN du proton (250 MHz, $CDCl_3$, δ en ppm, J en Hz).

0,98, (D, J = 7, 6H, $-CH(C\underline{H_3})_2$); 1,53 (Q, J = 7, 2H, $-C\underline{H_2}CH(CH_3)_2$); 1,68 (D, J = 7, 3H, $-CH_3$); 1,71 (Mt, 1H, $-C\underline{H}(CH_3)_2$); 2,38 (S, 6H, $-N(CH_3)_2$); 2,75 (DD, J = 13 et 6, 1H, 1H du >$N-CH_2-$); 3,08 (DD, J = 13 et 5, 1H, 1H du >$NCH_2-$); 3,47 (Mt, 2H, $-CONH-C\underline{H_2}-$); 4,27 (Mt, J = 7 - 6 et 5, 1H, >N-CH<); 6,2 (Mf, 1H, -CONH-); 6,90

à 7,25 (Mt, 5H, aromatiques); 7,3 (DD, J = 8 et 1,5, 1H, -H en 3); 7,7 (D, J = 1,5, 1H, -H en 1).

Spectre infra-rouge (KBr), bandes caractéristiques en $cm^{-1}$ :

3340, 3065, 3050, 2950, 2870, 2815, 2765, 1630, 1590, 1580, 1540, 1460, 1420, 1385, 1365, 1315, 1235, 845, 825, 755.

Le [diméthylamino-1 propyl-2-(2RS)]-10 phénothiazinecarbonitrile-2 peut être obtenu de la manière suivante :

Dans un erlenmeyer, on charge une solution de 342 g de chlorhydrate de chloro-2 diméthylamino-1 propane dans 700 cm3 d'eau distillée. On ajoute 270 cm3 d'une solution de soude 10N et le mélange est extrait par 1,3 litre de toluène. La phase organique est séchée sur sulfate de magnésium, filtrée et concentrée à 50°C sous pression réduite (30 mm de Hg; 4 kPa) jusqu'à obtenir un volume résiduel de 800 cm3.

A une suspension de 242 g de cyano-2 phénothiazine dans 2,2 litres de méthyléthylcétone on ajoute 96,7 g de potasse pulvérisée. La température du mélange monte spontanément à 24°C et on porte le mélange à 60°C pendant une demi-heure en agitant. On ajoute en 30 minutes la solution toluénique de chloro-2 diméthylamino-1 propane préparée précédemment. Le mélange est chauffé au reflux pendant 12 heures. Après refroidissement, le mélange est transféré dans une ampoule à décanter et est lavé par 2 fois 2 litres d'eau distillée. La phase organique est séchée sur sulfate de magnésium, filtrée puis concentrée à sec sous pression réduite (30 mm de Hg; 4 kPa) à 50°C pour donner une huile qui est diluée dans 2 litres d'éther éthylique. La solution obtenue est refroidie et amorcée par grattage. Le précipité jaune cristallisé est éliminé par filtration. Les liqueurs-mères sont concentrées à sec sous pression réduite (100 mm de Hg; 15 kPa) à 30°C pour donner une huile marron qui est purifiée par chromatographie sur colonne (hauteur : 80 cm; diamètre : 8,5 cm) de silice (0,2-0,06 mm) en éluant par un mélange de cyclohexane et d'acétate d'éthyle 80-20 (en volumes) et en recueillant des fractions de 1 litre. Les fractions 7 à 16 sont réunies et concentrées à sec à 50°C sous pression réduite (30 mm de Hg ; 4 kPa) pour donner 54,8 g de [diméthylamino-1 propyl-2-(2RS)]-10 phénothiazinecarbonitrile-2 sous la forme d'une huile marron.

EXEMPLE 35

On ajoute, en 2 minutes, sous agitation et à une température voisine de 20°C, 0,27 cm3 d'eau distillée dans une suspension de 1,71 g de tertiobutylate de potassium dans 20 cm3 de tertiobutanol. Dans la solution obtenue, on ajoute, sous agitation et à une température voisine de 20°C, une solution de 1,1 g de [(pyrrolidinyl-1)-1 propyl-2-(2RS)]-10 phénothiazinecarbonitrile-2 dans 10 cm3 de tertiobutanol. La solution brun clair est alors portée au reflux pendant 25 minutes. Après refroidissement à une température voisine de 30°C, on ajoute sous agitation 2,55 g d'iodo-1 propane et on porte au reflux pendant 3 heures 15 minutes. Après refroidissement, on concentre à sec sous pression réduite (30 mm de Hg; 4 kPa) à 40°C. Le résidu est repris par 10 cm3 d'eau distillée et on extrait par 25 cm3 d'acétate d'éthyle. La phase organique est séchée sur carbonate de potassium, filtrée et concentrée à sec sous pression réduite (30 mm de Hg; 4 kPa) à 40°C. La meringue grise résiduelle (1,5 g) est purifiée par chromatographie sur une colonne (hauteur : 25 cm; diamètre : 3,2 cm) de gel de silice (0,04-0,063 mm) sous une légère surpression d'azote (40 kPa) en éluant par 750 cm3 d'un mélange de chlorure de méthylène et de méthanol (90-10 en volumes) et en recueillant des fractions de 25 cm3. Les fractions 12 à 24 réunies sont concentrées à sec sous pression réduite (30 mm de Hg; 4 kPa) à 40°C. On obtient ainsi 0,85 g de N-propyl [(pyrrolidinyl-1)-1 propyl-2-(2RS)]-10 phénothiazinecarboxamide-2 sous forme d'une laque jaune.

EXEMPLE 36

Dans une solution de 17,6 g de tertiobutylate de potassium dans 120 cm3 de tertiobutanol, on ajoute 2,8 cm3 d'eau distillée puis, par portions, 11,7 g de chlorhydrate de [(pyrrolidinyl-1)-1 propyl-2]-10 phénothiazinecarbonitrile-2, série L, et on porte au reflux sous agitation pendant 25 minutes. Après refroidissement, on ajoute 15,3 cm3 d'iodo-1 propane et on porte à nouveau au reflux sous agitation pendant 3 heures. Après refroidissement, on concentre à sec sous pression réduite (30 mm de Hg; 4 kPa) à 40°C. Le résidu est repris par 250 cm3 d'acétate d'éthyle et la solution obtenue est lavée successivement par 100 cm3 d'eau distillée et 100 cm3 d'une solution aqueuse saturée de chlorure de sodium, séchée sur sulfate de magnésium, filtrée et concentrée à sec sous pression réduite (30 mm de Hg; 4 kPa) à 40°C. L'huile orangée résiduelle (12 g) est purifiée par chromatographie sur colonne (hauteur : 30 cm; diamètre : 6 cm) de gel de silice (0,2-0,063 mm) en éluant avec 5 litres d'un mélange de chlorure de méthylène et de méthanol (95-5 en volumes) et en recueillant des fractions de 100 cm3. Les fractions 25 à 43 sont réunies et concentrées à sec sous pression réduite (30 mm de Hg; 4 kPa) à 40°C. L'huile jaune résiduelle (8,4 g) est dissoute dans 84 cm3 d'acétate d'éthyle et additionnée, goutte à goutte et sous agitation, de 6,4 cm3 d'une solution 3,3N d'acide chlorhydrique dans

l'oxyde d'isopropyle. Le solide formé est essoré, lavé avec 15 cm3 d'acétate d'éthyle et séché sous pression réduite (5 mm de Hg; 0,7 kPa) à 40°C. On obtient ainsi 6,5 g de chlorhydrate de N-propyl [(pyrrolidinyl-1)-1 propyl-2]-10 phénothiazinecarboxamide-2, série L, sous forme d'une poudre blanche fondant à 183-188°C (fusion pâteuse) dont les caractéristiques sont identiques à celles du produit obtenu à l'exemple 5.

EXEMPLE 37

A une solution de 1,69 g de [diéthylamino-1 propyl-2-(2RS)]-10 phénothiazinecarbonitrile-2 dans 25 cm3 d'éthanol absolu, on ajoute 2,95 cm3 de méthyl-2-butylamine-(2RS). Le mélange est porté à 180°C pendant 20 heures; on y ajoute 2,95 cm3 de méthyl-2-butylamine-(2RS) puis on porte le mélange à 200°C pendant 20 heures. On fait alors barboter de l'air pendant 30 minutes et le mélange est porté à nouveau à 200°C pendant 16 heures puis dilué par 150 cm3 d'acétate d'éthyle, lavé par 4 fois 50 cm3 d'eau distillée, séché sur sulfate de magnésium, filtré et concentré à sec sous pression réduite (30 mm de Hg; 4 kPa) à 50°C. Le résidu obtenu est purifié par chromatographie sur une colonne (hauteur : 16 cm; diamètre : 2,8 cm) de gel de silice (0,04-0,06 mm) avec une légère surpression d'azote (40 kPa) en éluant avec des mélanges de cyclohexane et d'acétate d'éthyle 20-80 (1,5 litre) (en volumes) en recueillant des fractions de 60 cm3. Les fractions 8 à 20 sont réunies et concentrées à sec sous pression réduite (30 mm de Hg; 4 kPa) à 50°C pour donner 0,30 g d'un produit qui est purifié à nouveau sur colonne d'alumine basique (hauteur : 16,5 cm; diamètre : 13,8 cm) en éluant par un mélange de cyclohexane et d'acétate d'éthyle 90-10 (1 litre) (en volumes) en recueillant des fractions de 100 cm3. Les fractions 3 à 9 sont réunies et concentrées à sec sous pression réduite (30 mm de Hg; 4 kPa) pour donner 0,25 g de [diéthylamino-1 propyl-2-(2RS)]-10 N-[méthyl-2-(2RS) butyl] phénothiazinecarboxamide-2.

A une solution de 0,25 g de [diéthylamino-1 propyl-2-(2RS)]-10 N-[méthyl-2-(2RS) butyl] phénothiazinecarboxamide-2 dans 2,5 cm3 de propanol-2 bouillant, on ajoute 0,07 g d'acide fumarique dissous dans 1 cm3 de propanol-2 au reflux. La cristallisation est amorcée par grattage et le mélange est agité pendant 2 heures à 25°C puis laissé au repos pendant 12 heures à 0°C. Les cristaux sont filtrés et séchés sous pression réduite (5 mm de Hg; 0,7 kPa) à 40°C pour donner 0,19 g de fumarate de [diéthylamino-1 propyl-2 -(2RS)]-10 N-[méthyl-2-(2RS) butyl] phénothiazinecarboxamide-2 fondant à 110°C.

RMN du proton (250 MHz, DMSO, δ en ppm, J en Hz).

0,9 (Mt, 12H, 2 -$CH_3$ du méthyl-2 butyle et -$N(CH_2\underline{CH_3})_2$); 1,13 et 1,43 (2Mt, de 1H chacun, -$C\underline{H_2}$-$CH_3$); 1,63 (Mt, 1H, CH- du méthyl-2 butyle); 1,66 (D, J = 7, 1H, -$CH_3$); 2,55 (Mt masqué, 4H -$N(C\underline{H_2}$-$CH_3)_2$); 2,77 (DD, J = 14 et 6, 1H, 1H du >N-$CH_2$-); 3,08 (DD, J = 14 et 6,5, 1H du >$NCH_2$-); 3 à 3,3 (Mt, 2H, -CONH-$C\underline{H_2}$-); 4,20 (Mt, J = 7 - 6,5 et 6, 1H, >N-CH<); 6,63 (S, 2H, -CH=CH- du fumarate); 6,90 à 7,3 (Mt, 5H, aromatiques); 7,42 (DD, J = 8 et 1, 1H, -H en 3); 7,52 (D, J = 1, 1H, -H en 1); 8,43 (T, J = 5,5, 1H, -CONH-).

Spectre infra-rouge (KBr), bandes caractéristiques en $cm^{-1}$ :

3300, 3050, 2960, 2920, 2870, 2640, 2480, 1900, 1700, 1630, 1590, 1550, 1460, 1410, 1380, 1300, 1225, 980, 875, 830, 750, 635.

Le [diéthylamino-1 propyl-2-(2RS)]-10 phénothiazinecarbonitrile-2 peut être préparé comme décrit pour la préparation de la matière première utilisée à l'exemple 2.

EXEMPLE 38

Dans une solution de 5,6 g de tertiobutylate de potassium dans 40 $cm^3$ de tertiobutanol, on verse 0,45 $cm^3$ d'eau distillée et on ajoute sous agitation 3,7 g de [(pyrrolidinyl-1)-1 propyl-2-(2RS)]-10 phénothiazinecarbonitrile-2 et on porte à reflux pendant 15 minutes. On ajoute alors 7 $cm^3$ de bromo-1 éthyl-2 butane et l'agitation est poursuivie pendant 6 heures au reflux. Après refroidissement, le mélange réactionnel est concentré à sec sous pression réduite (30 mm de Hg; 4 kPa) à 40°C. Le résidu est repris dans 250 $cm^3$ d'acétate d'éthyle, lavé successivement par 2 fois 100 cm3 d'eau distillée et par 100 cm3 d'une solution aqueuse de chlorure de sodium, séché sur sulfate de magnésium, filtré et concentré à sec sous pression réduite (30 mm de Hg; 4 kPa) à 40°C. Le résidu (4,5 g) est purifié par chromatographie sur une colonne (hauteur: 25 cm; diamètre: 4 cm) de gel de silice (0,04-0,06 mm) avec une légère surpression d'azote (40,5 kPa) en éluant par 1,5 litre d'un mélange d'acétate d'éthyle et de cyclohexane (50-50 en volumes) et en recueillant des fractions de 50 cm3. Les fractions 13 à 25 sont réunies et concentrées à sec sous pression réduite (30 mm de Hg 4 kPa) à 40°C pour donner 2,5 g d'une huile jaune qui cristallise. Ce produit est repris dans 100 $cm^3$ d'oxyde d'isopropyle au reflux et la solution est refroidie. Les cristaux formés sont essorés, lavés par 10 cm3 d'oxyde d'isopropyle froid et séchés sous pression réduite (5 mm de Hg; 0,67 kPa) à 40°C pour donner 1,9 g de N-(éthyl-2 butyl) [(pyrrolidinyl-1)-1 propyl-2-(2RS)]-10 phénothiazinecarboxamide-2 sous la forme de cristaux blancs fondant à 115°C.

RMN du proton (250 MHz, $CDCl_3$, δ en ppm et J en Hz).

0,96 (T, J = 7, 6H, $-CH_3$ de l'éthyl-2 butyle); 1,42 (Mt, 4H, $-CH_2-$ de l'éthyl-2 butyle); 1,53 (Mt, 1H, >CH- de l'éthyl-2 butyle); 1,7 (D, J = 7, 3H, $-CH_3$); 1,82 (Mt, 4H, >N-$CH_2$- de la pyrrolidine); 2,66 (Mt, 4H, >N-$CH_2$- de la pyrrolidine); 2,99 (DD, J = 12,5 et 6,5, 1H, 1H du >N-$CH_2$-); 3,18 (DD, J = 12,5 et 6, 1H, 1H du N-$CH_2$-); 3,41 (Mt, 2H, -CONH-$C\underline{H}_2$-); 4,31 (Mt, J = 7 - 6,5 et 6, 1H, >N-CH<); 6,20 (T, J = 5,5, 1H, -CONH-); 6,90 à 7,20 (Mt, 5H, aromatiques); 7,27 (DD, J = 8 et 1H, H en 3); 7,65 (D, J = 1, 1H, H en 1).

Spectre infra-rouge (KBr), bandes caractéristiques en $cm^{-1}$

3245, 2960, 2930, 2870, 2780, 1630, 1595, 1580, 1465, 1545, 875, 835, 825, 750.

EXEMPLE 39

1 g de chlorhydrate d'acide [(pyrrolidinyl-1)-1 propyl-2]-10 phénothiazinecarboxylique-2, série L, dans 30 cm3 de chlorure de méthylène est refroidi à une température voisine de 5°C et on ajoute, sous agitation, 0,5 cm3 de chlorure de thionyle. Après 20 minutes, on porte la température à 20°C et on poursuit l'agitation pendant 2 heures. La solution jaune claire obtenue est concentrée à sec sous pression réduite (30 mm de Hg; 4 kPa) à 30°C. La meringue jaune résiduelle est dissoute dans 30 cm3 de chlorure de méthylène et on verse, goutte à goutte, sous agitation, 0,5 cm3 de propylamine. Après 1 heure d'agitation à 20°C, on concentre à sec sous pression réduite (30 mm de Hg; 4 kPa) à 40°C et on reprend le résidu avec 100 cm3 d'éther éthylique. La solution obtenue est lavée successivement par 15 cm3 de soude N et par 15 cm3 d'une solution aqueuse saturée de chlorure de sodium, séchée sur sulfate de magnésium, filtrée et concentrée à sec sous pression réduite (30 mm de Hg; 4 kPa) à 40°C. L'huile jaune pâle résiduelle (0,63 g) est dissoute dans 10 cm3 d'acétate d'éthyle et additionnée de 0,5 cm3 d'une solution 3,3N d'acide chlorhydrique dans l'oxyde d'isopropyle à une température voisine de 5°C et sous agitation. Le précipité formé est essoré, lavé par 2 cm3 d'oxyde d'isopropyle et séché sous pression réduite (5 mm de Hg; 0,7 kPa) à 40°C. On obtient ainsi 0,64 g de chlorhydrate de N-propyl [(pyrrolidinyl-1)-1 propyl-2]-10 phénothiazinecarboxamide-2, série L, sous forme d'un solide blanc fondant à 190°C dont les caractéristiques sont identiques à celles du produit obtenu à l'exemple 5.

Le chlorhydrate de l'acide [(pyrrolidinyl-1)-1 propyl-2]-10 phénothiazinecarboxylique-2 , série L, peut être obtenu de la manière suivante :

A une solution de 1,75 g de potasse dans 30 cm3 de glycol, on ajoute 5 g de chlorhydrate de [(pyrrolidinyl-1)-1 propyl-2]-10 phénothiazinecarbonitrile-2, série L, et on agite pendant 4 heures au reflux. Après refroidissement, la solution jaune obtenue est diluée avec 75 cm3 d'acétone et 5 cm3 d'une solution 3N d'acide chlorhydrique dans l'éther éthylique, filtrée et diluée à nouveau avec 75 cm3 d'acétone et 5 cm3 d'une solution 3N d'acide chlorhydrique dans l'éther éthylique. Après amorçage, on laisse cristalliser pendant 16 heures à une température voisine de 5°C. Le solide obtenu est essoré, lavé avec 10 cm3 d'éther éthylique et séché sous pression réduite (5 mm de Hg; 0,7 kPa) à 40°C. On obtient ainsi 2,9 g de chlorhydrate d'acide [(pyrrolidinyl-1)-1 propyl-2]-10 phénothiazinecarboxylique-2, série L, sous forme d'un solide jaune clair fondant à 200-210°C (fusion pâteuse).

EXEMPLE 40

Dans une suspension de 1,5 g de chlorhydrate d'acide [(pyrrolidinyl-1)-1 propyl-2-(2RS)]-10 phénothiazinecarboxylique-2 dans 45 $cm^3$ de chlorure de méthylène, on verse pendant 5 minutes et sous agitation, 0,75 $cm^3$ de chlorure de thionyle en maintenant la température aux environs de 5°C. L'agitation est poursuivie pendant 180 minutes en chauffant à une température voisine de 20°C et la solution jaune obtenue est concentrée à sec sous pression réduite (30 mm de Hg; 4 kPa) à 40°C. Le résidu est dissous dans 45 $cm^3$ de chlorure de méthylène et on ajoute 0,97 g de méthyl-2 pentylamine et 1,4 cm3 de triéthylamine. L'agitation est maintenue pendant 16 heures à 20°C. Le mélange réactionnel est concentré à sec sous pression réduite (30 mm de Hg; 4 kPa) à 40°C, repris par 100 $cm^3$ d'acétate d'éthyle, lavé avec 100 $cm^3$ d'une solution aqueuse N de soude, puis avec 2 fois 50 cm3 d'eau distillée et avec 50 $cm^3$ d'une solution saturée de chlorure de sodium, séché sur sulfate de magnésium, filtré et concentré à sec sous pression réduite (30 mm de Hg; 4 kPa) à 40°C. Le résidu huileux jaune (1,27 g) est dissous dans 50 cm3 d'oxyde d'isopropyle et on ajoute 6 cm3 d'une solution 0,5 N d'acide chlorhydrique dans l'oxyde d'isopropyle. Après 30 minutes d'agitation à 5°C, le précipité formé est filtré, lavé avec 3 fois 10 $cm^3$ d'oxyde d'isopropyle et séché sous pression réduite (5 mm de Hg; 0,67 kPa) à 40°C pour donner 1 g de chlorhydrate de N-(méthyl-2 pentyl) [(pyrrolidinyl-1)-1 propyl-2-(2RS)]-10 phénothiazinecarboxamide-2 sous forme d'un solide beige fondant à 138°C,

RMN du proton (250 MHz, DMSO D6, δ en ppm, J en Hz).

0,90 (Mt, 6H, $-CH_3$ du méthyl-2 pentyle); 1 à 1,5 (Mt, >CH-$CH_2$-$CH_2$- du méthyl-2 pentyle); 1,78 (D, J = 7, $-CH_3$); 1,7 à 2 (Mt, 4H, $-CH_2$-de la pyrrolidine); 2,84 - 3,08 - 3,60 et 3,74 ( 4 Mf, respectivement 1H chacun,

>N-$CH_2$- de la pyrrolidine); 3,05 et 3,18 (2 Mt, respectivement 1H chacun, -CONH-$C\underline{H}_2$-); 3,76 (AB limite, 2H, >N-$CH_2$-) ; 4,78 (Mt, 1H, >N-CH<); 7 à 7,4 (Mt, 5H, aromatiques) ; 7,53 (s, 1H, -H en 1); 7,55 (D,J = 8, 1H, H en 3); 8,65 (T, J = 5,5, 1 H, -CONH-); 10,8 (Mf, 1H, $NH^+Cl^-$).

Spectre infra-rouge (KBr), bandes caractéristiques en $cm^{-1}$ :

3260, 2960, 2925, 2870, 2580, 2470, 1640, 1590, 1460, 1535, 865, 835, 750.

Le chlorhydrate de l'acide [(pyrrolidinyl-1)-1 propyl-2-(2RS)]-10 phénothiazinecarboxilique-2 peut être préparé de la manière suivante :

En opérant comme à l'exemple 39, mais à partir de 20 g de chlorhydrate de [(pyrrolidinyl-1)-1 propyl-2-(2RS)]-10 phénothiazinecarbonitrile-2, on obtient 16,1 g de chlorhydrate d'acide [(pyrrolidinyl-1)-1 propyl-2-(2RS)]-10 phénothiazinecarboxylique-2 brut qui est recristallisé dans 330 $cm^3$ de propanol-2 bouillant. Après refroidissement, les cristaux sont filtrés et séchés sous pression réduite (5 mm de Hg; 0,67 kPa) pour donner 8,7 g de chlorhydrate de l'acide [(pyrrolidinyl-1)-1 propyl-2-(2RS)]-10 phénothiazinecarboxylique-2 sous la forme de cristaux jaunes fondant à 215-217°C.

## EXEMPLE 41

Dans une suspension de 1,5 g de chlorhydrate de l'acide [(pyrrolidinyl-1)-1 propyl-2-(2RS)]-10 phénothiazinecarboxylique-2 dans 45 $cm^3$ de chlorure de méthylène, on verse pendant 5 minutes et sous agitation, 0,75 $cm^3$ de chlorure de thionyle en maintenant la température aux environs de 5°C. L'agitation est poursuivie pendant 4 heures en chauffant à une température voisine de 20°C et la solution jaune obtenue est concentrée à sec sous pression réduite (30 mm de Hg; 4 kPa) à 50°C. Le résidu est dissous dans 45 $cm^3$ de chlorure de méthylène et on ajoute 1,3 g de chlorhydrate de diméthyl-2,3 butylamine et 1,4 $cm^3$ de triéthylamine. L'agitation est maintenue pendant seize heures à 20°C. Le mélange réactionnel est concentré à sec sous pression réduite (30 mm de Hg; 4 kPa) à 40°C, repris par 100 cm3 d'acétate d'éthyle, lavé avec 100 $cm^3$ d'une solution aqueuse N de soude, puis avec 2 fois 50 $cm^3$ d'eau distillée et avec 50 $cm^3$ d'une solution aqueuse saturée de chlorure de sodium, séché sur sulfate de magnésium, filtré et concentré à sec sous pression réduite (30 mm de Hg; 4 kPa) à 40°C. Le résidu huileux jaune est dissous dans 20 $cm^3$ d'oxyde d'isopropyle chaud. Les cristaux formés après refroidissement sont essorés, lavés par 2 $cm^3$ d'oxyde d'isopropyle froid et séchés sous pression réduite (5 mm de Hg; 0,67 kPa) à 40°C pour donner 0,8 g de N-(diméthyl-2,3 butyl) [(pyrrolidinyl-1)-1 propyl-2-(2RS)]-10 phénothiazinecarboxamide-2 sous forme d'un solide écru fondant à 138°C.

RMN du proton (250 MHz, DMSO $D_6$, $\delta$ en ppm, J en Hz).

0,75 à 0,95 (Mt, 9H, les 3 $CH_3$ du diméthyl-2,3 butyle); 1,59 (D, J = 7, 3H, -$CH_3$); 1,55 à 1,75 (Mt, 2H, CH- du diméthyl-2,3 butyle); 1,7 (Mf, 4H, -$CH_2$- de la pyrrolidine); 2,53 (Mt en partie masqué, >N-$CH_2$- de la pyrrolidine); 2,9 (DD, J = 12,5 et 7,5, 1H, 1H du >N$CH_2$-); 3,02 (DD, J = 12,5 et 6, 1H, 1H du >N-$CH_2$-); 3,06 et 3,26 (Mt, respectivement 1 H chacun, -CONH-$C\underline{H}_2$- du diméthyl-2,3 butyle); 4,21 (Mt, J = 7,5 -7 et 6, 1H, >N-CH<); 6,9 à 7,3 (Mt, 5H, aromatiques); 7,43 (DD, J = 3 et 1, 1H, -H en 3) ; 7,54 (D,J = 1, 1H, -H en 1); 8,4 (T,J = 5,5, 1H, -CO-NH-).

Spectre infra-rouge (KBr), bandes caractéristiques en $cm^{-1}$

3300, 2960, 2870, 2800, 1630, 1590, 1580, 1490, 1465, 1545, 875, 825, 750.

La présente invention concerne également les compositions pharmaceutiques constituées par un produit de formule générale (I) sous forme libre ou sous forme de sel d'addition avec un acide pharmaceutiquement acceptable, éventuellement en association avec à tout autre produit pharmaceutiquement compatible, pouvant être inerte ou physiologiquement actif. Les compositions selon l'invention peuvent être utilisées par voie parentérale, orale, rectale ou topique.

Les compositions stériles pour administration parentérale qui peuvent être notamment utilisées sous forme de perfusions sont de préférence des solutions aqueuses ou non aqueuses, des suspensions ou des émulsions. Comme solvant ou véhicule, ou peut employer l'eau, le propylèneglycol, un polyéthylèneglycol, des huiles végétales, en particulier l'huile d'olive, des esters organiques injectables, par exemple l'oléate d'éthyle ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Les compositions pour administration rectale sont les suppositoires ou les capsules rectales, qui contiennent outre le produit actif des excipients tels que le beurre de cacao, des glycérides semi-synthétiques ou des polyéthylèneglycols.

Comme compositions solides pour administration orale peuvent être utilisés des comprimés, des pilules,

des poudres ou des granulés. Dans ces compositions, le produit actif selon l'invention (éventuellement associé à un autre produit pharmaceutiquement compatible) est mélangé à un ou plusieurs diluants ou adjuvants inertes, tels que saccharose, lactose ou amidon. Ces compositions peuvent également comprendre des substances autres que les diluants, par exemple un lubrifiant tel que le stéarate de magnésium.

Comme compositions liquides pour administration orale, on peut utiliser des émulsions pharmaceutiquement acceptables, des solutions, des suspensions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions pour administration topique peuvent être par exemple des crèmes, des pommades, ou des lotions.

En thérapeutique humaine, les produits selon l'invention sont particulièrement utiles dans le traitement des douleurs d'origine traumatique, post-chirurgicale, homotopique, menstruelle, céphaliques..., ainsi que dans les traitements diurétiques.

Les doses dépendent de l'effet recherché et de la durée du traitement. Pour un adulte, elles sont généralement comprises entre 0,25 et 1500 mg par jour en prises échelonnées.

D'une façon générale, le médecin déterminera la posologie qu'il estime la plus appropriée en fonction de l'âge, du poids et de tous les autres facteurs propres au sujet à traiter.

Les exemples suivants, donnés à titre non limitatif, illustrent des compositions selon l'invention.

Exemple A

On prépare, selon la technique habituelle, des comprimés dosés à 25 mg de produit actif (base) ayant la composition suivante :

- chlorhydrate de N-propyl [(pyrrolidinyl-1)-1 propyl-2]-10 phénothiazinecarboxamide-2, série L 27 mg
- amidon 83 mg
- silice 30 mg
- stéarate de magnésium 3 mg

EXEMPLE B

On prépare selon la technique habituelle une solution pour administration intra-veineuse dosée à 25 mg/cm3 de produit actif (base) :

- chlorhydrate de N-propyl [(pyrrolidinyl-1)-1 propyl-2]-10 phénothiazinecarboxamide-2, série L 2,70 g
- acide ascorbique 0,100 g
- Sulfite neutre de sodium 0,050 g
- Soude 1N (q.s.p. pH 4) environ 0,08 cm3
- NaCl (q.s.p. isotonie) environ 0,650 g
- Eau pour préparation injectable q.s.p 100 cm3

## Revendications

### Revendications pour les Etats contractans suivants : AT, BE, CH, DE, FR, GB, IT, LI, LU, NL, SE

**1.** Application d'un dérivé de la phénothiazine de formule générale:

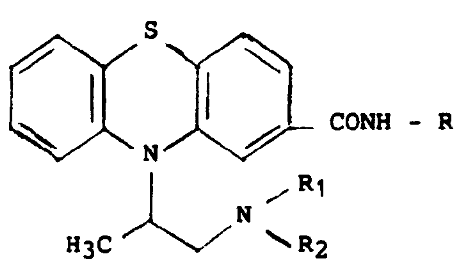

dans laquelle R est un radical alcoyle contenant 1 à 6 atomes de carbone en chaîne droite ou ramifiée et $R_1$ et $R_2$ identiques ou différents sont des radicaux alcoyle droits ou ramifiés contenant 1 à 4 atomes de carbone ou forment ensemble avec l'atome d'azote auquel ils sont rattachés, un hétérocycle contenant 4 à 7 chaînons, sous ses formes isomères ou leurs mélanges et/ou sous forme de sel d'addition avec les acides, pour obtenir un médicament destiné aux traitements analgésiques.

2. Application d'un dérivé de la phénothiazine tel que défini dans la revendication 1, pour obtenir un médicament destiné aux traitements diurétiques.

3. Application d'un dérivé de la phénothiazine selon la revendication 1, pour lequel le symbole R est un radical alcoyle contenant 2 à 6 atomes de carbone en chaîne droite ou ramifiée, et les symboles $R_1$ et $R_2$ identiques ou différents sont des radicaux alcoyle droits ou ramifiés contenant 1 à 3 atomes de carbone ou forment ensemble avec l'atome d'azote auquel ils sont rattachés un hétérocycle contenant 5 à 7 chaînons, sous ses formes isomères ou leurs mélanges et/ou sous forme de sel d'addition avec les acides, pour obtenir un médicament destiné aux traitements analgésiques.

4. Application d'un dérivé de la phénothiazine tel que défini dans la revendication 3, pour obtenir un médicament destiné aux traitements diurétiques.

5. Application d'un dérivé de la phénothiazine selon la revendication 1, pour lequel le symbole R est un radical alcoyle contenant 3 à 6 atomes de carbone en chaine droite ou ramifiée et les symboles $R_1$ et $R_2$ identiques ou différents sont des radicaux alcoyle droits ou ramifiés contenant 2 ou 3 atomes de carbone ou forment ensemble avec l'atome d'azote auquel ils sont rattachés, un hétérocycle contenant 5 à 7 chaînons, sous forme d'un mélange d'isomères ou sous forme des isomères de série L, et/ou sous forme de sel d'addition avec les acides, pour obtenir un médicament destiné aux traitements analgésiques.

6. Application d'un dérivé de la phénothiazine tel que défini dans la revendication 5, pour obtenir un médicament destiné aux traitements diurétiques.

7. Un dérivé de la phénothiazine tel que défini dans l'une des revendications 1, 3 ou 5.

8. Le N-propyl [(pyrrolidinyl-1)-1 propyl-2]-10 phénothiazinecarboxamide-2 sous ses formes isomères ou leurs mélanges.

9. Le N-(méthyl-3 butyl) [(pyrrolidinyl-1)-1 propyl-2]-10 phénothiazinecarboxamide-2 sous ses formes isomères ou leurs mélanges.

10. Le N-(méthyl-2 propyl) [(pyrrolidinyl-1)-1 propyl-2]-10 phénothiazinecarboxamide-2 sous ses formes isomères ou leurs mélanges.

11. Le N-butyl [(pyrrolidinyl-1)-1 propyl-2]-10 phénothiazinecarboxamide-2 sous ses formes isomères ou leurs mélanges.

12. Le N-(méthyl-3 butyl) [pipéridino-1 propyl-2]-10 phénothiazinecarboxamide-2 sous ses formes isomères ou leurs mélanges.

13. Procédé de préparation d'un dérivé de la phénothiazine tel que défini dans la revendication 1 caractérisé en ce que l'on fait agir une amine de formule générale :

$$R - NH_2$$

dans laquelle R est défini selon la revendication 1, sur un thioamide primaire de formule générale :

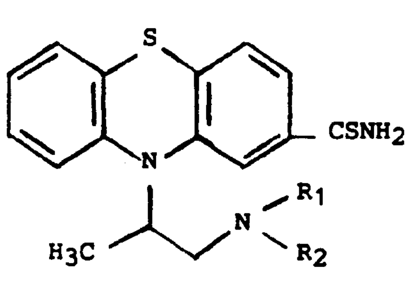

dans laquelle $R_1$ et $R_2$ sont définis comme dans la revendication 1, puis le cas échéant lorsque l'on isole le thioamide substitué intermédiaire, de formule générale :

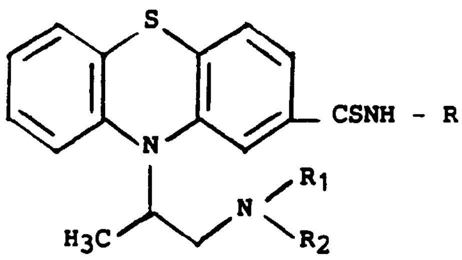

dans laquelle R, $R_1$ et $R_2$ sont définis comme dans la revendication 1, oxyde ce thioamide en l'amide correspondant et transforme éventuellement le produit obtenu en un sel d'addition avec un acide.

**14.** Procédé de préparation d'un dérivé de la phénothiazine selon la revendication 1, caractérisé en ce que l'on transforme un nitrile de formule générale :

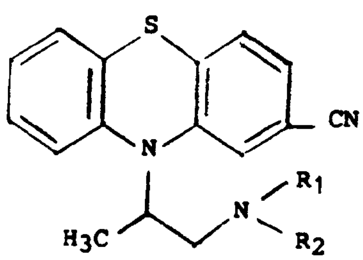

dans laquelle $R_1$ et $R_2$ sont définis comme dans la revendication 1, par toute méthode connue pour obtenir un amide substitué à partir d'un nitrile, sans toucher au reste de la molécule puis transforme éventuellement le produit obtenu en un sel d'addition avec un acide.

**15.** Procédé de préparation d'un dérivé de la phénothiazine selon la revendication 1, caractérisé en ce que l'on transforme un acide de formule générale :

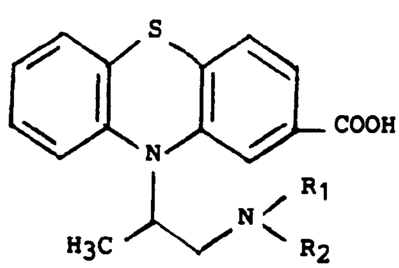

dans laquelle $R_1$ et $R_2$ sont définis comme dans la revendication 1, par toute méthode connue pour obtenir un amide substitué sans toucher au reste de la molécule, puis transforme éventuellement le produit obtenu en un sel d'addition avec un acide.

**Revendications pour les Etats contractants suivants : ES, GR**

**1.** Application d'un dérivé de la phénothiazine de formule générale:

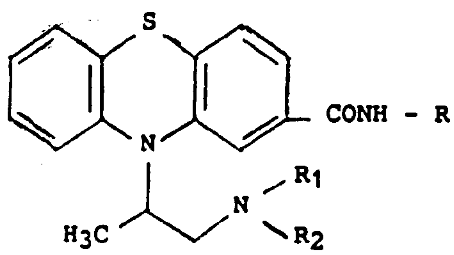

dans laquelle R est un radical alcoyle contenant 1 à 6 atomes de carbone en chaîne droite ou ramifiée et $R_1$ et $R_2$ identiques ou différents sont des radicaux alcoyle droits ou ramifiés contenant 1 à 4 atomes de carbone ou forment ensemble avec l'atome d'azote auquel ils sont rattachés, un hétérocycle contenant 4 à 7 chaînons, sous ses formes isomères ou leurs mélanges et/ou sous forme de sel d'addition avec les acides, pour obtenir un médicament destiné aux traitements analgésiques.

**2.** Application d'un dérivé de la phénothiazine tel que défini dans la revendication 1, pour obtenir un médicament destiné aux traitements diurétiques.

**3.** Application d'un dérivé de la phénothiazine selon la revendication 1, pour lequel le symbole R est un radical alcoyle contenant 2 à 6 atomes de carbone en chaîne droite ou ramifiée, et les symboles $R_1$ et $R_2$ identiques ou différents sont des radicaux alcoyle droits ou ramifiés contenant 1 à 3 atomes de carbone ou forment ensemble avec l'atome d'azote auquel ils sont rattachés un hétérocycle contenant 5 à 7 chaînons, sous ses formes isomères ou leurs mélanges et/ou sous forme de sel d'addition avec les acides, pour obtenir un médicament destiné aux traitements analgésiques.

**4.** Application d'un dérivé de la phénothiazine tel que défini dans la revendication 3, pour obtenir un médicament destiné aux traitements diurétiques.

**5.** Application d'un dérivé de la phénothiazine selon la revendication 1, pour lequel le symbole R est un radical alcoyle contenant 3 à 6 atomes de carbone en chaine droite ou ramifiée et les symboles $R_1$ et $R_2$ identiques ou différents sont des radicaux alcoyle droits ou ramifiés contenant 2 ou 3 atomes de carbone ou forment ensemble avec l'atome d'azote auquel ils sont rattachés, un hétérocycle contenant 5 à 7 chaînons, sous forme d'un mélange d'isomères ou sous forme des isomères de série L, et/ou sous forme de sel d'addition avec les acides, pour obtenir un médicament destiné aux traitements analgésiques.

**6.** Application d'un dérivé de la phénothiazine tel que défini dans la revendication 5, pour obtenir un médicament destiné aux traitements diurétiques.

**7.** Procédé de préparation d'un dérivé de la phénothiazine tel que défini dans la revendication 1 caractérisé en ce que l'on fait agir une amine de formule générale :

$$R - NH_2$$

dans laquelle R est défini selon la revendication 1, sur un thioamide primaire de formule générale :

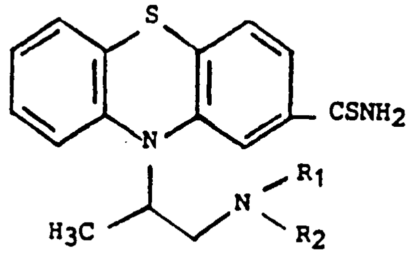

dans laquelle $R_1$ et $R_2$ sont définis comme dans la revendication 1, puis le cas échéant lorsque l'on isole le thioamide substitué intermédiaire, de formule générale :

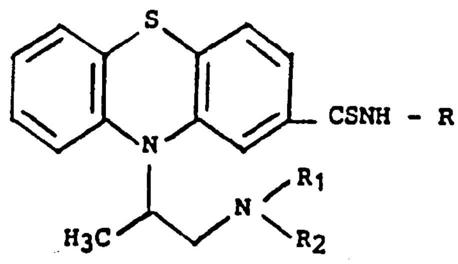

dans laquelle R, $R_1$ et $R_2$ sont définis comme dans la revendication 1, oxyde ce thioamide en l'amide correspondant et transforme éventuellement le produit obtenu en un sel d'addition avec un acide.

8. Procédé de préparation d'un dérivé de la phénothiazine selon la revendication 1, caractérisé en ce que l'on transforme un nitrile de formule générale :

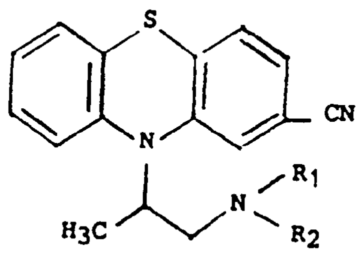

dans laquelle $R_1$ et $R_2$ sont définis comme dans la revendication 1, par toute méthode connue pour obtenir un amide substitué à partir d'un nitrile, sans toucher au reste de la molécule puis transforme éventuellement le produit obtenu en un sel d'addition avec un acide.

9. Procédé de préparation d'un dérivé de la phénothiazine selon la revendication 1, caractérisé en ce que l'on transforme un acide de formule générale :

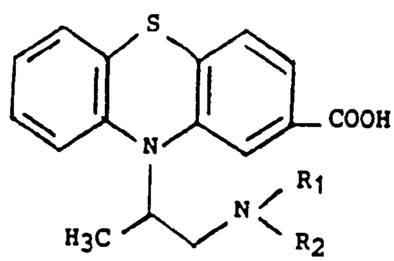

dans laquelle $R_1$ et $R_2$ sont définis comme dans la revendication 1, par toute méthode connue pour obtenir un amide substitué sans toucher au reste de la molécule, puis transforme éventuellement le produit obtenu en un sel d'addition avec un acide.

**Patentansprüche**

**Patentansprüche für folgende Vertragsstaaten : AT, BE, CH, DE, FR, GB, IT, LI, LU, NL, SE**

1. Verwendung eines Phenothiazin-Derivats der allgemeinen Formel

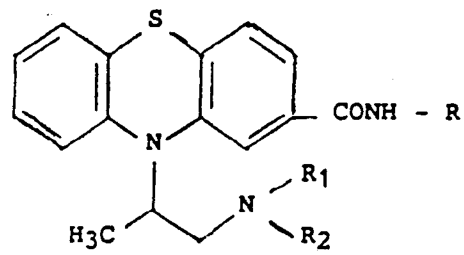

worin R für einen Alkylrest mit 1 bis 6 Kohlenstoffatomen in gerader oder verzweigter Kette steht, und $R_1$ und $R_2$, identisch oder voneinander verschieden, geradkettige oder verzweigte Alkylreste mit 1 bis 4 Kohlenstoffatomen sind oder gemeinsam mit dem Stickstoffatom, an das sie gebunden sind, einen Heterocyclus mit 4 bis 7 Kettengliedern bilden, in ihren isomeren Formen oder deren Gemischen und/oder in Form des Additionssalzes mit Säuren für die Herstellung eines Arzneimittels für analgetische Behandlungen.

**2.** Verwendung eines Phenothiazin-Derivats, wie in Anspruch 1 definiert, für die Herstellung eines Arzneimittels für diuretische Behandlungen.

**3.** Verwendung eines Phenothiazin-Derivats gemäß Anspruch 1, worin das Symbol R einen Alkylrest mit 2 bis 6 Kohlenstoffatomen in gerader oder verzweigter Kette darstellt, und die Symbole $R_1$ und $R_2$, identisch oder voneinander verschieden, geradkettige oder verzweigte Alkylreste mit 1 bis 3 Kohlenstoffatomen sind oder gemeinsam mit dem Stickstoffatom, an das sie gebunden sind, einen Heterocyclus mit 5 bis 7 Kettengliedern bilden, in ihren isomeren Formen oder deren Gemischen und/oder in Form des Additionssalzes mit Säuren für die Herstellung eines Arzneimittels für analgetische Behandlungen.

**4.** Verwendung eines Phenothiazin-Derivats, wie in Anspruch 3 definiert, für die Herstellung eines Arzneimittels für diuretische Behandlungen.

**5.** Verwendung eines Phenothiazin-Derivats gemäß Anspruch 1, worin das Symbol R einen Alkylrest mit 3 bis 6 Kohlenstoffatomen in gerader oder verzweigter Kette bedeutet, und die Symbole $R_1$ und $R_2$, identisch oder voneinander verschieden, geradkettige oder verzweigte Alkylreste mit 2 oder 3 Kohlenstoffatomen sind oder gemeinsam mit dem Stickstoffatom, an das sie gebunden sind, einen Heterocyclus mit 5 bis 7 Kettengliedern bilden, in Form eines Gemisches von Isomeren oder in Form der Isomeren der L-Reihe und/oder in Form des Additionssalzes mit Säuren für die Herstellung eines Arzneimittels für analgetische Behandlungen.

**6.** Verwendung eines Phenothiazin-Derivats, wie in Anspruch 5 definiert, für die Herstellung eines Arzneimittels für diuretische Behandlungen.

**7.** Phenothiazin-Derivat, wie in einem der Ansprüche 1, 3 oder 5 definiert.

**8.** N-Propyl-10-[1-(pyrolidin-1-yl)-prop-2-yl]-phenothiazin-2-carboxamid in seinen isomeren Formen oder deren Gemischen.

**9.** N-(3-Methylbutyl)-10-[1-(pyrolidin-1-yl)-prop-2-yl]-phenothiazin-2-carboxamid in seinen isomeren Formen oder deren Gemischen.

**10.** N-(2-Methylpropyl)-10-[1-(pyrolidin-1-yl)-prop-2-yl]-phenothiazin-2-carboxamid in seinen isomeren Formen oder deren Gemischen.

**11.** N-Butyl-10-[1-(pyrolidin-1-yl)-prop-2-yl]-phenothiazin-2-carboxamid in seinen isomeren Formen oder deren Gemischen.

**12.** N-(3-Methylbutyl)-10-[1-piperidino-prop-2-yl]-phenothiazin-2-carboxamid in seinen isomeren Formen oder deren Gemischen.

**13.** Verfahren zur Herstellung eines Phenothiazin-Derivats, wie in Anspruch 1 definiert, dadurch gekennzeichnet, daß man ein Amin der allgemeinen Formel

$R - NH_2$

worin R wie in Anspruch 1 definiert ist, mit einem primären Thioamid der allgemeinen Formel

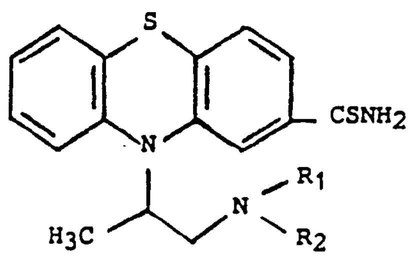

worin $R_1$ und $R_2$ wie in Anspruch 1 definiert sind, umsetzt, hiernach gegebenenfalls, wenn man das intermediäre substituierte Thioamid der allgemeinen Formel

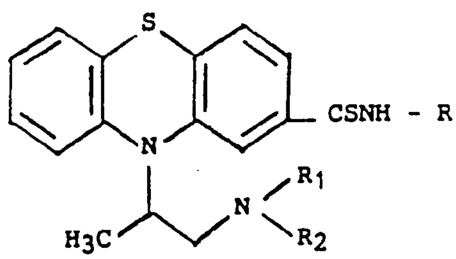

worin R, $R_1$ und $R_2$ wie in Anspruch 1 definiert sind, isoliert, dieses Thioamid zu dem entsprechenden Amid oxidiert und das erhaltene Produkt gegebenenfalls in ein Additionssalz mit einer Säure überführt.

**14.** Verfahren zur Herstellung eines Phenothiazin-Derivats gemäß Anspruch 1, dadurch gekennzeichnet, daß man ein Nitril der allgemeinen Formel

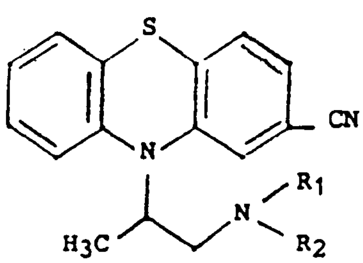

worin $R_1$ und $R_2$ wie in Anspruch 1 definiert sind, nach jeder an sich bekannten Methode zur Erzielung eines substituierten Amids aus einem Nitril ohne Beeinträchtigung des Restes des Moleküls unwandelt, und hiernach gegebenenfalls das erhaltene Produkt in ein Additionssalz mit einer Säure überführt.

**15.** Verfahren zur Herstellung eines Phenothiazin-Derivats gemäß Anspruch 1, dadurch gekennzeichnet, daß man eine Säure der allgemeinen Formel

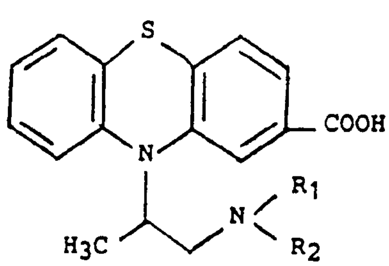

worin $R_1$ und $R_2$ wie in Anspruch 1 definiert sind, nach jeder an sich bekannten Methode für die Erzielung eines substituierten Amids ohne Beeinträchtigung des Restes des Moleküls umwandelt und danach gegebenenfalls das erhaltene Produkt in ein Additionssalz mit einer Säure überführt.

**Patentansprüche für folgende Vertragsstaaten : ES, GR**

1. Verwendung eines Phenothiazin-Derivats der allgemeinen Formel

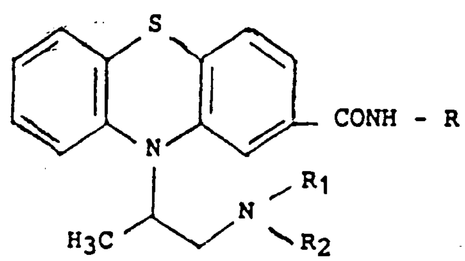

worin R für einen Alkylrest mit 1 bis 6 Kohlenstoffatomen in gerader oder verzweigter Kette steht, und $R_1$ und $R_2$, identisch oder voneinander verschieden, geradkettige oder verzweigte Alkylreste mit 1 bis 4 Kohlenstoffatomen sind oder gemeinsam mit dem Stickstoffatom, an das sie gebunden sind, einen Heterocyclus mit 4 bis 7 Kettengliedern bilden, in ihren isomeren Formen oder deren Gemischen und/oder in Form des Additionssalzes mit Säuren für die Herstellung eines Arzneimittels für analgetische Behandlungen.

2. Verwendung eines Phenothiazin-Derivats, wie in Anspruch 1 definiert, für die Herstellung eines Arzneimittels für diuretische Behandlungen.

3. Verwendung eines Phenothiazin-Derivats gemäß Anspruch 1, worin das Symbol R einen Alkylrest mit 2 bis 6 Kohlenstoffatomen in gerader oder verzweigter Kette darstellt, und die Symbole $R_1$ und $R_2$, identisch oder voneinander verschieden, geradkettige oder verzweigte Alkylreste mit 1 bis 3 Kohlenstoffatomen sind, oder gemeinsam mit dem Stickstoffatomen, an das sie gebunden sind, einen Heterocyclus mit 5 bis 7 Kettengliedern bilden, in ihren isomeren Formen oder deren Gemischen und/oder in Form des Additionssalzes mit Säuren für die Herstellung eines Arzneimittels für analgetische Behandlungen.

4. Verwendung eines Phenothiazin-Derivats, wie in Anspruch 3 definiert, für die Herstellung eines Arzneimittels für diuretische Behandlungen.

5. Verwendung eines Phenothiazin-Derivats gemäß Anspruch 1, worin das Symbol R einen Alkylrest mit 3 bis 6 Kohlenstoffatomen in gerader oder verzweigter Kette bedeutet, und die Symbole $R_1$ und $R_2$, identisch oder voneinander verschieden, geradkettige oder verzweigte Alkylreste mit 2 oder 3 Kohlenstoffatomen sind, oder gemeinsam mit dem Stickstoffatom, an das sie gebunden sind, einen Heterocyclus mit 5 bis 7 Kettengliedern bilden, in Form eines Gemisches von Isomeren oder in Form der Isomeren der L-Reihe und/oder in Form des Additionssalzes mit Säuren für die Herstellung eines Arzneimittels für analgetische Behandlungen.

6. Verwendung eines Phenothiazin-Derivats, wie in Anspruch 5 definiert, für die Herstellung eines Arzneitmittels für diuretische Behandlungen.

7. Verfahren zur Herstellung eines Phenothiazin-Derivats, wie in Anspruch 1 definiert, dadurch gekennzeichnet, daß man ein Amin der allgemeinen Formel

$$R - NH_2$$

worin R wie in Anspruch 1 definiert ist, mit einem primären Thioamid der allgemeinen Formel

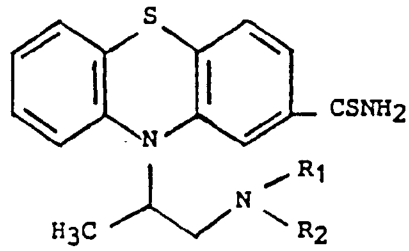

worin $R_1$ und $R_2$ wie in Anspruch 1 definiert sind, umsetzt, hiernach gegebenenfalls, wenn man das intermediäre substituierte Thioamid der allgemeinen Formel

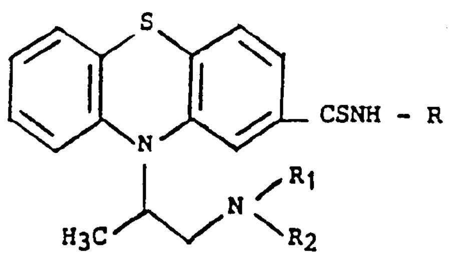

worin R, $R_1$ und $R_2$ wie in Anspruch 1 definiert sind, isoliert, dieses Thioamid zu dem entsprechenden Amid oxidiert und das erhaltene Produkt gegebenenfalls in ein Additionssalz mit einer Säure überführt.

8. Verfahren zur Herstellung eines Phenothiazin-Derivats gemäß Anspruch 1, dadurch gekennzeichnet, daß man ein Nitril der allgemeinen Formel

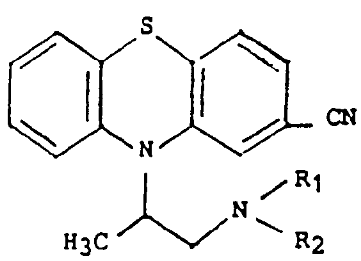

worin $R_1$ und $R_2$ wie in Anspruch 1 definiert sind, nach jeder an sich bekannten Methode zur Erzielung eines substituierten Amids aus einem Nitril ohne Beeinträchtigung des Restes des Moleküls umwandelt, und hiernach gegebenenfalls das erhaltene Produkt in ein Additionssalz mit einer Säure überführt.

9. Verfahren zur Herstellung eines Phenothiazin-Derivats gemäß Anspruch 1, dadurch gekennzeichnet, daß man eine Säure der allgemeinen Formel

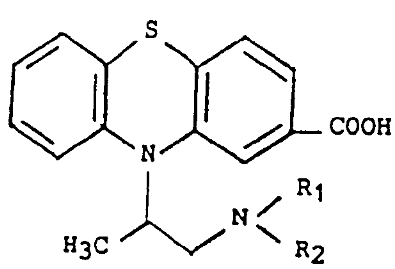

worin $R_1$ und $R_2$ wie in Anspruch 1 definiert sind, nach jeder an sich bekannten Methode für die Erzielung eines substituierten Amids ohne Beeinträchtigung des Restes des Moleküls umwandelt, und danach gegebenenfalls das erhaltene Produkt in ein Additionssalz mit einer Säure überführt.

## Claims

### Claims for the following Contracting States : AT, BE, CH, DE, FR, GB, IT, LI, LU, NL, SE

1. Application of a phenothiazine derivative of general formula:

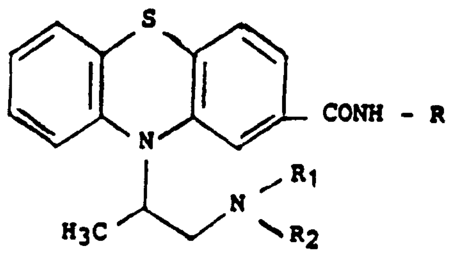

in which R is a straight- or branched-chain alkyl radical containing 1 to 6 carbon atoms and $R_1$ and $R_2$, which may be identical or different, are straight or branched alkyl radicals containing 1 to 4 carbon atoms or, together with the nitrogen atom to which they are attached form a 4- to 7-membered heterocycle, in its isomeric forms or mixtures thereof and/or in the form of an addition salt with acids, for obtaining a medicinal product intended for analgesic treatments.

2. Application of a phenothiazine derivative as defined in Claim 1, for obtaining a medicinal product intended for diuretic treatments.

3. Application of a phenothiazine derivative according to Claim 1 for which the symbol R is a straight- or branched-chain alkyl radical containing 2 to 6 carbon atoms and the symbols $R_1$ and $R_2$, which may be identical or different, are straight or branched alkyl radicals containing 1 to 3 carbon atoms or, together with the nitrogen atom to which they are attached, form a 5- to 7-membered heterocycle, in its isomeric forms or mixtures thereof and/or in the form of an addition salt with acids, for obtaining a medicinal product intended for analgesic treatments.

4. Application of a phenothiazine derivative as defined in Claim 3, for obtaining a medicinal product intended for diuretic treatments.

5. Application of a phenothiazine derivative according to Claim 1 for which the symbol R is a straight- or branched-chain alkyl radical containing 3 to 6 carbon atoms and the symbols $R_1$ and $R_2$, which may be identical or different, are straight or branched alkyl radicals containing 2 or 3 carbon atoms or, together with the nitrogen atom to which they are attached, form a 5- to 7-membered heterocycle, in the form of a mixture of isomers or in the form of the isomers of the L series, and/or in the form of an addition salt with acids, for obtaining a medicinal product intended for analgesic treatments.

6. Application of a phenothiazine derivative as defined in Claim 5, for obtaining a medicinal product intended for diuretic treatments.

7. A phenothiazine derivative as defined in one of Claims 1, 3 and 5.

8. N-Propyl-10-[1-(1-pyrrolidinyl)-2-propyl]-2-phenothiazinecarboxamide, in its isomeric forms or mixtures thereof.

9. N-(3-Methylbutyl)-10-[1-(1-pyrrolidinyl)-2-propyl]-2-phenothiazinecarboxamide, in its isomeric forms or mixtures thereof.

10. N-(2-Methylpropyl)-10-[1-(1-pyrrolidinyl)-2-propyl]-2-phenothiazinecarboxamide, in its isomeric forms or mixtures thereof.

11. N-Butyl-10-[1-(1-pyrrolidinyl)-2-propyl]-2-phenothiazinecarboxamide, in its isomeric forms or mixtures thereof.

12. N-(3-Methylbutyl)-10-(1-piperidino-2-propyl)-2-phenothiazinecarboxamide, in its isomeric forms or mixtures thereof.

13. Process for preparing a phenothiazine derivative as defined in Claim 1, characterised in that an amine of general formula:

$$R - NH_2$$

in which R is defined according to Claim 1, is reacted with a primary thioamide of general formula:

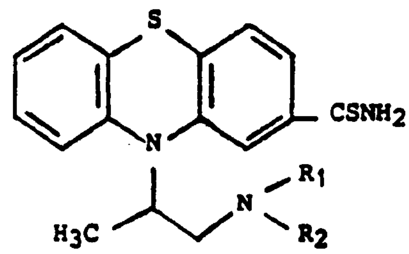

in which $R_1$ and $R_2$ are defined as in Claim 1, and then, where appropriate, when the intermediate substituted thioamide of general formula:

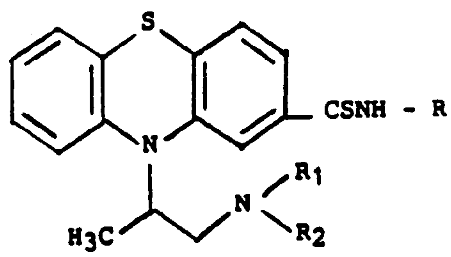

in which R, $R_1$ and $R_2$ are defined as in Claim 1, is isolated, this thioamide is oxidised to the corresponding amide and the product obtained is optionally converted to an addition salt with an acid.

14. Process for preparing a phenothiazine derivative according to Claim 1, characterised in that a nitrile of general formula:

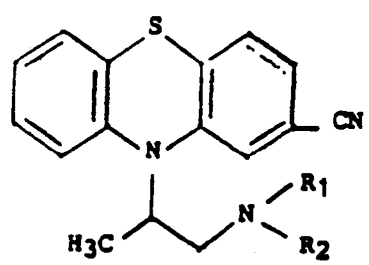

in which $R_1$ and $R_2$ are defined as in Claim 1, is converted by any known method for obtaining a substituted amide from a nitrile without affecting the remainder of the molecule, and the product obtained is then optionally converted to an addition salt with an acid.

15. Process for preparing a phenothiazine derivative according to Claim 1, characterised in that an acid of general formula:

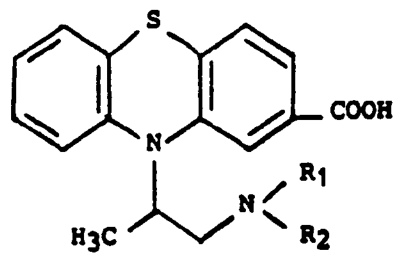

in which $R_1$ and $R_2$ are defined as in Claim 1, is converted by any known method for obtaining a substituted amide without affecting the remainder of the molecule, and the product obtained is then optionally converted to an addition salt with an acid.

**Claims for the following Contracting States : ES, GR**

1. Application of a phenothiazine derivative of general formula:

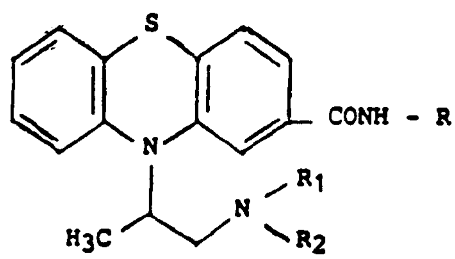

in which R is a straight- or branched-chain alkyl radical containing 1 to 6 carbon atoms and $R_1$ and $R_2$, which may be identical or different, are straight or branched alkyl radicals containing 1 to 4 carbon atoms or, together with the nitrogen atom to which they are attached form a 4- to 7-membered heterocycle, in its isomeric forms or mixtures thereof and/or in the form of an addition salt with acids, for obtaining a medicinal product intended for analgesic treatments.

2. Application of a phenothiazine derivative as defined in Claim 1, for obtaining a medicinal product intended for diuretic treatments.

3. Application of a phenothiazine derivative according to Claim 1 for which the symbol R is a straight- or branched-chain alkyl radical containing 2 to 6 carbon atoms and the symbols $R_1$ and $R_2$, which may be identical or different, are straight or branched alkyl radicals containing 1 to 3 carbon atoms or, together with the nitrogen atom to which they are attached, form a 5- to 7-membered heterocycle, in its isomeric forms or mixtures thereof and/or in the form of an addition salt with acids, for obtaining a medicinal product intended for analgesic treatments.

4. Application of a phenothiazine derivative as defined in Claim 3, for obtaining a medicinal product intended for diuretic treatments.

5. Application of a phenothiazine derivative according to Claim 1 for which the symbol R is a straight- or branched-chain alkyl radical containing 3 to 6 carbon atoms and the symbols $R_1$ and $R_2$, which may be identical or different, are straight or branched alkyl radicals containing 2 or 3 carbon atoms or, together with the nitrogen atom to which they are attached, form a 5- to 7-membered heterocycle, in the form of a mixture of isomers or in the form of the isomers of the L series, and/or in the form of an addition salt with acids, for obtaining a medicinal product intended for analgesic treatments.

6. Application of a phenothiazine derivative as defined in Claim 5, for obtaining a medicinal product intended for diuretic treatments.

7. Process for preparing a phenothiazine derivative as defined in Claim 1, characterised in that an amine of general formula:

$$R - NH_2$$

in which R is defined according to Claim 1, is reacted with a primary thioamide of general formula:

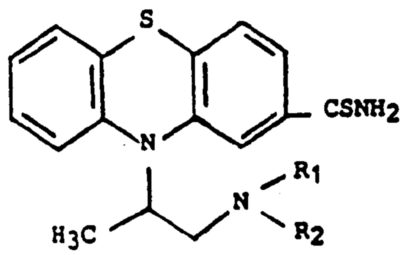

in which $R_1$ and $R_2$ are defined as in Claim 1, and then, where appropriate, when the intermediate substituted thioamide of general formula:

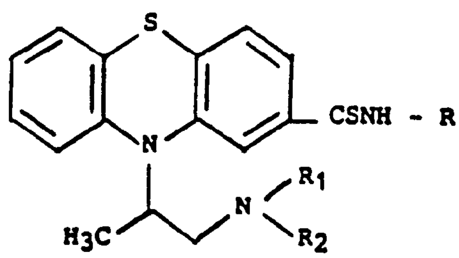

in which R, $R_1$ and $R_2$ are defined as in Claim 1, is isolated, this thioamide is oxidised to the corresponding amide and the product obtained is optionally converted to an addition salt with an acid.

8. Process for preparing a phenothiazine derivative according to Claim 1, characterised in that a nitrile of general formula:

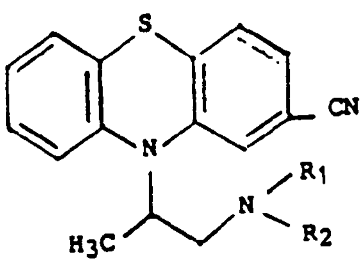

in which $R_1$ and $R_2$ are defined as in Claim 1, is converted by any known method for obtaining a substituted amide from a nitrile without affecting the remainder of the molecule, and the product obtained is then optionally converted to an addition salt with an acid.

9. Process for preparing a phenothiazine derivative according to Claim 1, characterised in that an acid of general formula:

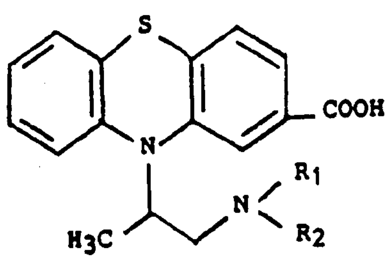

in which $R_1$ and $R_2$ are defined as in Claim 1, is converted by any known method for obtaining a substituted

amide without affecting the remainder of the molecule, and the product obtained is then optionally converted to an addition salt with an acid.